(19) Europäisches Patentamt
European Patent Office
Office européen des brevets

(11) Publication number: 0 071 281 B1

(12) **EUROPEAN PATENT SPECIFICATION**

(45) Date of publication of patent specification: **18.06.86**

(21) Application number: **82107978.7**

(22) Date of filing: **12.02.80**

(60) Publication number of the earlier application in accordance with Art. 76 EPC: **0 024 091**

(51) Int. Cl.[4]: **C 07 C 45/50,** C 07 C 45/49, C 07 C 45/74, C 07 C 45/62, C 07 C 47/02, C 07 C 47/21, B 01 J 31/24, C 07 F 15/00

# (54) Hydroformylation process.

(30) Priority: **12.02.79 US 11238**
**29.05.79 US 43548**
**23.01.80 US 114627**

(43) Date of publication of application:
**09.02.83 Bulletin 83/06**

(45) Publication of the grant of the patent:
**18.06.86 Bulletin 86/25**

(84) Designated Contracting States:
**FR**

(56) References cited:
**DE-A-2 026 163**
**FR-A-2 041 776**
**FR-A-2 071 942**
**GB-A-2 002 753**
**US-A-3 939 188**

(73) Proprietor: **Exxon Research and Engineering Company**
**P.O.Box 390 180 Park Avenue**
**Florham Park New Jersey 07932 (US)**

(72) Inventor: **Oswald, Alexis Alexander**
**1098 Sunnyslope Drive**
**Mountainside New Jersey (US)**
Inventor: **Jermansen, Torris Grahn**
**245 Getz Avenue**
**Staten Island New York (US)**
Inventor: **Westner, Andrew Arkadij**
**1 Short Way**
**Paramus New Jersey (US)**
Inventor: **Huang, I-Der**
**29, Danebury Downs**
**Upper Saddle River New Jersey 07458 (US)**

(74) Representative: **Field, Roger Norton et al**
**ESSO Engineering (Europe) Ltd. Patents & Licences Apex Tower High Street**
**New Malden Surrey KT3 4DJ (GB)**

**EP 0 071 281 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to hydroformylation processes.

One of the key unexpected factors in process of the present invention is that the present catalysts can be employed in a large excess without a drastic loss of catalyst activity. The other factor, also important for high selectivity, is the high ratio $H_2$ to CO. Unexpectedly, the excess of hydrogen does not result in the reduction of the aldehyde hydroformylation products to the corresponding alcohols. Coupled with the high $H_2$/CO ratios, it is essential in the present process to employ relatively low pressures, effectively limiting the CO partial pressure. Finally, the continuous process of the present invention is distinguished by relatively low olefin conversions. These are important for both catalyst stability and selectivity.

Due to the above characteristics, the present alkyl diaryl phosphine complex catalysts are uniquely suited for an operation wherein the aldehyde product is separated from the catalyst by distillation. Such a specifically advantageous operation is carried out in a continuous fashion wherein the olefin and synthesis gas feed are continuously introduced into the reactor comprising the catalyst solution and a mixture of the aldehyde product and the feed is continuously withdrawn in the gas phase.

The preferred selective process of the present invention, particularly the combination of the above features, is unique. It is not only unexpected in view of the prior art but was described as a process which should be inoperative due to the type of phosphine ligands employed.

When compared with the tris-(triphenylphosphine) rhodium carbonyl hydride (TPP-rhodium) plus triphenyl phosphine based commercial, continuous process, the present process exhibits surprising advantages. The alkyl diaryl phosphines of the present process do not undergo P-C bond scission. The only catalyst by-products are the corresponding phosphine oxides. The latter are not inhibitors. The secondary by-products derived from the aldehyde products such as aldehyde trimers do not seriously inhibit the present catalytic system either. The catalysts used in the process of this invention stand out with regard to long term activity maintenance in a continuous process. In contrast to the known process, no introduction of oxygen and/or chelating compounds or use of hydroxylic solvent is required for activity maintenance. As a consequence of higher catalyst stability, the present process can be operated at higher temperatures. This, in turn, can lead to an improved product to feed ratio in the distillate of the continuous product flash-off process. Also, it extends applicability to higher olefins and olefin derivatives. In addition, it provides unexpected advantages when employed for combined hydroformylation-aldolization-hydrogenation processes.

The process of the present invention comprises reacting an olefinic compound with hydrogen and carbon monoxide in the presence of a reactive mixture comprising (1) a rhodium-containing catalyst having no reactive halogen and having at least one ligand complexed with said rhodium, wherein the complexed ligand comprises a complexed compound containing at least one diaryl phosphino alkyl group and wherein the number of such diaryl phosphino alkyl groups in complex association with said rhodium is at least two, and (2) a non-complexed ligand substantially all of which is a non-complexed compound containing at least one diaryl phosphino alkyl group, wherein the molar ratio of the non-complexed ligand to rhodium is greater than 75:1, preferably greater than 100:1, and wherein the ratio of the partial pressures of hydrogen to carbon monoxide is at least 3:1.

The most preferred selective hydroformylation comprises reacting an olefin with a mixture of carbon monoxide and hydrogen in the presence of an alkyl diaryl phosphine halogen free rhodium complex as a catalyst to produce mainly an aldehyde, preferably via carbonylation at the less substituted vinylic carbon. Halogen free means that there is no reactive halogen, particularly chlorine, bonded to rhodium.

An improved method for hydroformylation was discovered comprising reacting an olefin with CO and $H_2$ in the presence of a tris- and bis-(alkyl diaryl phosphine) rhodium carbonyl complex catalyst free from halogen on the rhodium and excess free tertiary phosphine ligand wherein said improvement is effected by an appropriately high ratio of both $H_2$/CO and ligand/Rh to produce an improved stability which leads to a ratio above four of n- and i-aldehyde primary products said products being the major primary products when the method employs a 1-n-olefin as starting reactant.

As stated, such selective reactions were unexpectedly found to depend critically on the alkyl diaryl phosphine complex catalysts, the excess of phosphine ligand and the ratio of $H_2$/CO synthesis gas reactant, i.e., the CO partial pressure. The selectivity also depends on the type of olefin employed. In an important embodiment of the new process, the process is run on a continuous basis with the reaction being conducted at a temperature, olefin, $H_2$ and CO feed rates, a rhodium concentration and a rhodium to non-complexed ligand molar ratio effective to provide a rate of production of said aldehydes of at least about 0.1 g mole/l-hr, and wherein the ratio of the partial pressures of hydrogen to carbon monoxide is at least 3:1; and the aldehyde product is continuously removed as a vapor from the reaction mixture. Each of the above-described processes is hereinafter described as the process of the invention. In these processes, the CO partial pressure is preferably kept low, e.g., below 100 psi.

The process of the present invention has been found to provide a catalyst system having good thermal stability. Moreover, in the presence of a large excess of the diaryl alkyl phosphine ligand, the catalyst activity was surprisingly maintained while stability was increased. The process of the present invention was also found to provide unexpectedly good selectivity for producing n-aldehyde products from alpha-olefins.

In a preferred embodiment of the process of the present invention, the olefinic carbon compound is one containing an alpha-olefinic double bond. In this preferred process, the $H_2$/CO ratio and the amount of the non-complexed compound containing at least one diaryl phosphino alkyl group are effective to provide an aldehyde product having a normal to iso isomer ratio of at least about 4:1. Again, it is preferable to maintain a low CO partial pressure.

In another embodiment of the process of the present invention, the reaction is preferably conducted at a temperature of at least about 90°C, a rhodium concentration of at least about .0001 molar and a non-complexed ligand to rhodium molar ratio of over 100. Also, the non-complexed ligand present in the reaction mixture preferably consists essentially of the non-complexed compound containing at least one diaryl phosphino alkyl group.

In yet another preferred embodiment of the process of the present invention, the L/Rh ratio, i.e., of the non-complexed compound containing at least one diaryl phosphino alkyl group to rhodium, is preferably about 120, more preferably above 240, and most preferably above 400. By raising the ligand to rhodium ratio when alpha-olefins are used in the process of the invention, higher normal to iso isomer ratios of the aldehyde product are obtained and accordingly, higher ligand to rhodium ratios are preferred in such cases. In a particularly preferred embodiment of the invention, the non-complexed ligand present during the reaction consists essentially of the non-complexed compound containing at least one diaryl phosphino alkyl group, and this ligand is present in a molar ratio, i.e., L/Rh of greater than 100.

Suitable complexed and non-complexed compounds containing at least one diaryl phosphino alkyl group for use in the process of the invention include compounds of the following formula (which includes known diaryl alkyl phosphines as well as the novel substituted diaryl phosphino alkyl compounds which are exemplified by Formula I):

$$(Ar_2PQ)_bE^yR_{y-b}$$

wherein Ar is an aryl group containing from 6 to 10 carbon atoms;

Q is an alkylene radical or an alkylene radical, the carbon chain of which is interrupted with either oxygen or phenylene groups, wherein the alkylene radical contains from 2 to 30 carbon atoms;

E represents

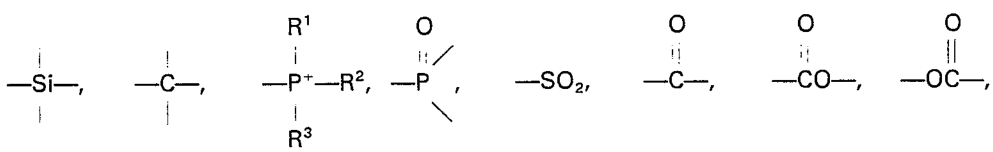

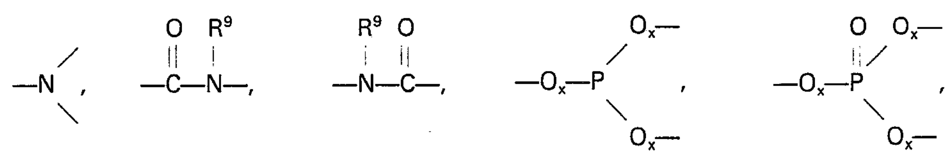

—O— or —S—, wherein $R^1$, $R^2$ and $R^3$ are each alkyl groups containing from 1 to 30 carbon atoms, wherein $R^9$ is H, an alkyl group containing from 1 to 30 carbon atoms or an aryl group containing from 6 to 10 carbon atoms, and wherein x is 0 or 1 with the proviso that at least one x is 1;

y represents the valence bonds of the group E available for bonding to the groups Q and R (Thus, for a covalent bond, y is 2; for —Si—, y is 4; and for

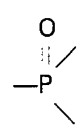

y is 3.); and b is an integer of from 1 to 4, provided that y−b is not less than zero; and

each R group independently represents an alkyl group containing from 1 to 30 carbon atoms or an aryl group containing from 6 to 10 carbon atoms, and when E is

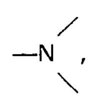

y=3 and b=1, R is $R_2$ the divalent radical

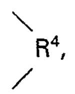 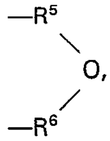 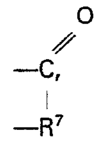 or 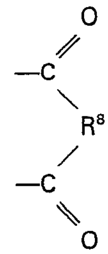

which member together with the N atom forms a heterocyclic ring, wherein $R^4$, $R^5$, $R^6$, $R^7$ and $R^8$ are hydrocarbyl radicals such that the heterocyclic ring contains from 5 to 6 atoms;

Preferably, the complexed and non-complexed compounds containing diaryl phosphino groups are of the formula

$$Ar_2PQE^yR_{y-1}$$

wherein Ar, Q, E, y and R are as defined above.

Suitable complexed and non-complexed compounds containing diaryl phosphino alkyl groups which can be used in the process of the present invention include the known diaryl phosphino alkyl compounds, tetraalkyl phosphonium substituted phosphine ligands and the ligands of our novel catalysts claimed and described in our European patent application No. 80900540.8 (Pub. No. WO 80/01690).

Non-chelating ligands of the formula

$$Ar_2P(CH_2)_mPAr_2$$

wherein Ar is aryl as defined above and m is an integer of from 4 to 14, represent yet another class of suitable complexed and non-complexed ligands.

The tetraalkyl phosphonium compounds as mentioned above represent still another class of ligands suitable for use in the process of the present invention. These phosphonium derivatives can be exemplified by the general formula

$$(Ar_2PQ)P^{+}R^{1}R_{2}R^{3}Z^{-}$$

wherein Ar, Q, $R^1$, $R^2$ and $R^3$ are as defined above and $Z^-$ represents an anion. The $Z^-$ anion electrically neutralises the ligand moiety; can be monovalent or polyvalent and is preferably a non-coordinating anion. Examples of suitable Z anions include halide, hydroxide, sulfate, sulfonate, phosphate, phosphonate, phosphite, tetraphenyl boride, fluorophosphate, carboxylate such as acetate, phenoxide and alkoxide. A particularly preferred subclass of such phosphonium ligands is of the formula

$$Ph_2P\text{-}(CH_2)_m\text{-}P^{+}R^{1}R^{2}R^{3}Z$$

wherein m is an integer of from 1 to 30 and the other symbols are as defined above. These phosphonium ligands are also employed in complex association with rhodium as catalyst suitable for use in the process of the present invention.

Specific examples of such complexed and non-complexed compounds containing diaryl phosphino alkyl groups include ethyl diphenyl phosphine, propyl diphenyl phosphine, butyl diphenyl phosphine,

O

$Ph_2PCH_2CH_2Ph$, $Ph_2PCH_2CH_2PPh_2$, 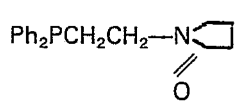, $Ph_2PCH_2CH_2COCH_3$,

O

$Ph_2PCH_2CH_2CCH_3$, $Ph_2PCH_2CH_2Si(CH_3)_3$, $Ph_2PCH_2CH_2C(CH_3)_3$, and $Ph_2PCH_2C(CH_3)_3$,

wherein Ph here and through this specification and attached claims represents phenyl.

In yet another embodiment of the process of the present invention the rhodium-containing catalyst employed is of the general formula

$$[(Ar_2PQ)_bE^yR_{b-y}]_g \cdot (RhX_n)_s$$

wherein Ar, Q, y, E, R and b are as defined above, X is an anion or organic ligand, excluding halogen, satisfying the coordination sites of the rhodium metal; g is 1 to 6 provided g times b is 1 to 6; n is 2 to 6; and

s is 1 to 3. Other preferred catalysts for use in the process of the present invention include rhodium-containing catalysts of the formulae:

$$[(Ar_2PQ)_bE^yR_{y-b}]_g \cdot [RhH(CO)]_s$$

and

$$(Ar_2PQE^yR_{y-1})_3RhH(CO)$$

wherein Ar, Q, E, y, R, b, g and s are as defined above.

A preferred class of complexes suitable for use in the process of the present invention include complexes of the formula

$$(Ar_2PQE^yR_{y-1})_3Rh(CO)H$$

and

$$[(Ar_2PQ)_bE^yR_{y-b}]_g[Rh(CO)H]_s$$

wherein E represents a covalent bond or

$$-C-;$$

R is an alkyl group, preferably a substituted or unsubstituted alkyl group containing 1 to 30 carbon atoms and more preferably a saturated open chain alkyl group; and Ar, Q, y and b are as defined above. Particularly suitable compounds within this class include compounds of the formula

$$[Ar_2P{-}(CH_2)_m{-}R]_3Rh(CO)H$$

wherein m is an integer of from 1 to 30, preferably from 2 to 22 and more preferably from 2 to 4; R is a straight-chain, branched or cyclic alkyl group or an aryl group such as isopropyl, tertiary butyl, cyclohexyl, or phenyl. A particularly suitable catalyst for use in the process of the present invention is

$$(Ph_2PCH_2CH_2CH_3)_3Rh(CO)H.$$

Other examples of suitable catalysts for use in the present invention include

$$Rh(CO)H(Ph_2PCH_2CH_2Ph)_3,\ Rh(CO)H(Ph_2PCH_2CH_2\overset{\overset{O}{\|}}{P}Ph_2)_3,$$

$$Rh(CO)H(Ph_2PCH_2CH_2{-}\overset{\overset{O}{\|}}{N}\!<\!\square)_3,\ Rh(CO)H(Ph_2PCH_2CH_2\overset{\overset{O}{\|}}{C}CH_3)_3,$$

$$Rh(CO)H(Ph_2PCH_2CH_2COCH_3)_3,\ Rh(CO)H(Ph_2PCH_2CH_2Si(CH_3)_3)_3,$$

$$Rh(CO)H(Ph_2PCH_2CH_2C(CH_3)_3)_3, \text{ and } Rh(CO)H(Ph_2PCH_2C(CH_3)_3)_3.$$

The Ar, Q and R groups in the above-discussed ligands and complexes thereof can also be substituted with various substituent groups. In general, the substituents on the Ar, Q and R groups, and for that matter any substituent in the ligands and complexes used in the process of the present invention or in the novel complexes of the invention as claimed in European patent application No. 80900540.8 are those which are chemically unreactive with the reactants used in and the products of the desired catalyzed reaction, e.g., a hydroformylation reaction. The same exemplary substituents can be used on any of the Ar, Q and/or R groups. Suitable substituents include halogen, carboxy, phenoxy, and hydroxy groups and also alkyl, alkoxy, acyl, acyloxy, acylamide, carbamido and carbohydrocarbyloxy groups containing from 1 to 30 carbon atoms, and preferably from 1 to 12 carbon atoms.

Suitable Ar groups for use in the complexed and non-complexed compounds or rhodium complexes thereof include aryl groups containing from 6 to 10 carbon atoms. The terminology "aryl group containing from 6 to 10 carbon atoms", as used in this specification and in the attached claims, is meant to include aromatic groups containing from 6 to 10 carbon atoms in the basic aromatic structure which structure can be substituted with any chemically unreactive substituent as discussed above. The aryl groups are also intended to include heterocyclic aromatic groups such as pyrryl, thienyl and furyl. The substituents on the aryl group, if any, are preferably bound to a phenyl group. Mono- and di-substituted phenyl groups are

preferred. Thus, examples of suitable aromatic groups include phenyl, fluorophenyl, difluorophenyl, tolyl, xylyl, benzoyloxyphenyl, carboethoxyphenyl, acetylphenyl, ethoxyphenyl, phenoxyphenyl, biphenyl, naphthyl hydroxyphenyl, carboxyphenyl, trifluoromethylphenyl, tetrahydronaphthyl, furyl, pyrryl, thienyl, methoxyethylphenyl, acetamidophenyl, and dimethylcarbamylphenyl.

Q in the above formulae represents a divalent organic radical selected from an alkylene group and an alkylene group the carbon chain of which is interrupted with ether oxygen or phenylene groups, wherein the alkylene group contains from 1 to 30 carbon atoms, preferably from 2 to 22 carbon atoms, and more preferably from 2 to 4 carbon atoms. The terminology "alkylene group", as used in this specification and in the attached claims, is meant to include an alkylene group containing 1 to 30 carbon atoms in the basic alkylene structure, which structure may again be substituted with any chemically unreactive substituent as discussed above. Examples of suitable Q organic radicals include ethylene, trimethylene, butylene, decamethylene, docosamethylene, tricontamethylene, phenyl bis(ethyl), ethylene bis-(oxyethyl), ethylene-bis oligo-(oxyethyl), oxy ethyl propyl, oxy ethyl perfluoroethyl, oxy ethyl hydroxypropyl, xylylene and octadecamethylene. Preferably Q represents $-(CH_2)_m-$ wherein m ranges from 1 to 30, preferably from 2 to 14, and most preferably from 2 to 4.

Suitable R groups for use in the above compounds include aryl groups containing from 6 to 10 carbon atoms and alkyl groups containing from 1 to 30 carbon atoms and when E is

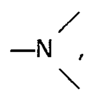 ,

R can also represent a member selected from

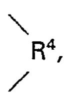, 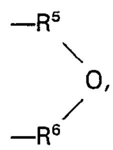, 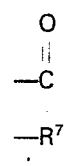 and 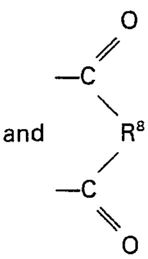

which member together with the N atom forms a heterocyclic ring, wherein $R^4$, $R^5$, $R^6$, $R^7$ and $R^8$ are hydrocarbyl radicals such that the heterocyclic ring contains from 5 to 6 atoms. These R groups may again be substituted with substituents that are chemically unreactive as discussed above. Suitable R aryl groups include any of those mentioned above in the definition of suitable Ar groups. By the terminology "alkyl group containing from 1 to 30 carbon atoms", we mean to include alkyl groups containing from 1 to 30 carbon atoms in the basic alkyl structure, which can be straight-chain, branched or cyclic and which can be substituted with any chemically unreactive substituent as discussed above. The alkyl groups are preferably primary or secondary alkyl groups, more preferably primary alkyl groups containing from 2 to 22 carbon atoms, and even more preferably from 6 to 14 carbon atoms. Exemplary alkyl groups include methyl, ethyl, propyl, n-butyl, iso-butyl, t-butyl, n-hexyl, cyclohexyl, methylcyclopentyl, isopropyl, decyl, fluoropropyl, docosyl, triacontyl, cyclopentyl, phenyl, methoxyethoxyethyl, acetylethyl, tris-hydroxy substituted t-butylethyl, triphenylmethylethyl, hydroxypropyl, carbomethoxyethyl, phenoxyethyl, benzamidoethyl, benzoyloxyethyl, pyrrylethyl, furylethyl and thienylethyl.

X in the above formulae represents an anion or organic ligand which satisfies the coordination sites of the rhodium metal, preferably a non-coordinating anion. Suitable X groups include $H^-$, alkyl$^-$, aryl$^-$, substituted aryl$^-$, $CR_3^-$, $C_2F_5^-$, $CN^-$, $N_3^-$, $COR^-$, $PR_4^-$, (where R is alkyl or aryl), carboxylate such as acetate, acetylacetonate, $SO_4^{2-}$, sulfonate, $NO_2^-$, $NO_3^-$, $O_2^-$, $CH_3O^-$, $CH_2{=}CHCH_2^-$, CO, $C_6H_5CN$, $CH_3CN$, NO, $NH_3$, pyridine, $(C_4H_9)_3P$, $(C_2H_5)_3N$, chelating olefins, diolefins and triolefins, tetrahydrofuran, $CH_3CN$ and triphenyl phosphine. Preferred organic ligands are readily displaceable such as carbonyl, olefins, tetrahydrofuran and acetonitrile. The most preferred X ligands are CO and H.

Suitable complex catalysts include higher valent phosphorus derivatives of the formula

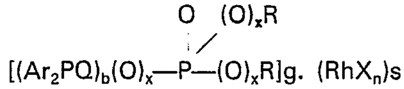

Preferred complexes within this class are

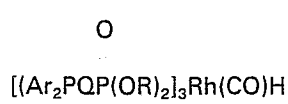

$$[(Ar_2P(CH_2)_m\overset{O}{\overset{\|}{P}}R_2]_3Rh(CO)H$$

$$[(Ar_2P(CH_2)_m)_3\overset{O}{\overset{\|}{P}}]Rh(CO)H$$

$$[Ar_2P(CH_2)_m\overset{O}{\overset{\|}{P}}Ar_2]_3Rh(CO)H$$

$$(Ar_2PQ\overset{O}{\overset{\|}{P}}R_2)_3RhH(CO),$$

$$[Ph_2P(CH_2)_m\overset{O}{\overset{\|}{P}}Ph_2]_3Rh(CO)H$$

and

$$[(Ar_2P(CH_2)_mO)_3\overset{O}{\overset{\|}{P}}]_3Rh(CO)H$$

wherein Ar, Q, R, X, b, x, g, n, Ph and s are as defined above. A preferred compound is of the formula

$$[Ph_2P\text{-}(CH_2)_m\text{-}\overset{O}{\overset{\|}{P}}R_2]_3Rh(CO)H$$

wherein m is an integer of from 1 to 30, especially from 2 to 14, and R is defined as above. One example of this type catalyst is

$$(Ph_2PCH_2CH_2\overset{O}{\overset{\|}{P}}Ph_2)_3Rh(CO)H$$

More suitable complexes contain 2 or 3, preferably 3, coordinated alkyl diaryl phosphine moieties per rhodium, 1 to 3, preferably 1, carbon monoxide ligands per rhodium, and 1 or 0, preferably 1, hydride ligand per rhodium.

These complexes are non-charged non-chelated bis- and tris-(alkyl diaryl phosphine) rhodium carbonyl hydrides of the formula

$$[Ar_2P)_yR^1_y]_g[Rh(CO)H]_s$$

wherein Ar is aryl, preferably an independently selected $C_6$ to $C_{10}$ aromatic nonsubstituted or substituted hydrocarbyl radical, more preferably phenyl, mono-, di- and tri-substituted phenyl, most preferably phenyl; $R^1$ is a $C_1$ to $C_{30}$, preferably $C_2$ to $C_{20}$, mono-, di-, tri- or tetravalent nonsubstituted or substituted saturated alkyl, including alkyl groups interrupted by noncharged heteroorganic groups such as those containing O, N, P, S, Si, with the proviso that if $R^1$ is a nonsubstituted monovalent alkyl, the minimum number of alkyl carbons is six; y is the valency of the alkyl group, g times y is 1 to 6, preferably 2 to 6, s is 1 to 3, preferably 2 or 3; said y and s being selected to satisfy the coordinative valencies of rhodium in such a manner that there are 2 or 3, preferably 3, coordinated phosphine moieties per rhodium, all the aromatic and aliphatic groups and their substituents including non-hydrocarbon groups being chemically stable in hydroformylation systems.

While the values of g, y and s are dependent on the coordinative bonding of the rhodium, the tris-phosphine rhodium complex compositions are unexpectedly stable and as such preferred. In the case of monovalent alkyl, preferably substituted alkyl, diaryl phosphine rhodium complexes, the preferred compositions are accordingly of the formula

$$(Ar_2PR^1)_gRh(CO)H$$

wherein $R^1$ is a monovalent alkyl as previously defined, preferably a substituted alkyl; g is 1 to 3, preferably 2 or 3, more preferably 3, and, of the formula

$(Ar_2PR^1)_3Rh(CO)H$

The preferred substituents of the aromatic groups are $C_1$ to $C_{30}$, preferably $C_1$ to $C_{12}$ alkyl, alkoxy, acyl, acyloxy, acrylamido, carbamido, carbohydrocarbyloxy, halogen, phenoxy, hydroxy, carboxy. These substituents are preferably bound to a phenyl group. Mono- and di-substituted phenyl groups are preferred.

Examples of the aromatic groups are phenyl, fluorophenyl, difluorophenyl, tolyl, xylyl, benzoyloxyphenyl, carboethoxyphenyl, acetylphenyl, ethoxyphenyl, phenoxyphenyl, biphenyl, naphthyl, hydroxyphenyl, carboxyphenyl, trifluoromethylphenyl, tetrahydronaphthyl, furyl, pyrryl, methoxyethylphenyl, acetamidophenyl, dimethylcarbamylphenyl.

The alkyl groups are primary and secondary alkyl groups, preferably primary alkyl groups. Next to the preferred primary α-carbons of the alkyl groups, the β-carbons are primary and secondary, preferably also primary. Accordingly, a preferred class of complexes is of the general formula

$$[Ar_2PCH_2CH_2R']_3Rh(CO)H$$

wherein R' is a $C_4$ to $C_{28}$, nonsubstituted or substituted alkyl of a preferably branched or cyclic character; a non-substituted or substituted $C_6$ to $C_{10}$ aryl, preferably phenyl; a nonhydrocarbyl group, preferably selected from organic radicals containing silicon, oxygen, nitrogen and phosphorus. The heteroatoms of the organic radicals are preferably of the trihydrocarbyl silane, hydroxy, ether, acyl, amine, amide, and phosphine oxide groups. The heteroatom is preferably directly bound to the β-methylene group.

The preferred substituents of the primary alkyl groups are the same. Some more preferred substituted alkyl diaryl phosphine complexes will be defined later.

Exemplary alkyl groups are methyl, n-hexyl, docosyl, triacontyl, fluoropropyl, perfluoroethyl-ethyl, isopropyl, primary isobutyl, cyclopentyl, t-butylethyl, cyclohexylethyl, phenylethyl, trimethylsilylethyl, hydroxy, methoxyethoxyethyl, acetylethyl, pyrrolidinonylethyl, tributylphosphonium substituted ethyl, tris-hydroxy substituted t-butylethyl, triphenylmethylethyl, hydroxypropyl, carbomethoxyethyl, phenoxyethyl, benzamidoethyl, benzoyloxyethyl, pyrrylethyl, furylethyl, thienylethyl. The non-hydrocarbyl, i.e., heteroorganic R' groups will be further defined.

The alkyl groups as defined by R' are mono- or polyvalent alkyl groups, their valence ranging from 1 to 4.

The polyvalent groups may have a carbon skeleton or can be interrupted by appropriate heteroatoms such as oxygen, sulfur, nitrogen, silicon.

Exemplary polyvalent alkyl groups are tetramethylene, xylylene, oxy-bis-propyl, sulfone-bis-propyl, nitrilo-tripropyl, silicone-tetraethyl, cyclohexylene diethyl, keto-bis-ethyl.

A class of the alkyl groups is represented by aliphatic hydrocarbyl groups. Preferred subgroups of the latter are n-alkyl groups and hydrocarbyl substituted n-alkyl groups. When $R^1$ is one of these two subgroups, the preferred complexes are of the formula

$$[Ar_2P(CH_2)_nCH_3]_gRh(CO)H$$

and

$$[Ar_2P(CH_2)_mR'']_gRh(CO)H$$

wherein n is 6 to 30 and m is 1—22, preferably 2 to 22, more preferably 2 or 3, R'' is a $C_3$ to $C_{27}$ branched alkyl, cycloalkyl, aryl, such as isopropyl, t-butyl, cyclohexyl, phenyl.

The choice of aryl and alkyl groups and their substituents is limited only by stereochemical and reactivity considerations. Sterically demanding groups inhibit the formation of the present tris-phosphine complexes. Groups which are reactive under the use conditions of the present complexes are apparently undesirable as catalysts.

Also suitable are alkyl diphenyl phosphine rhodium complexes of the following formula

$$(Ar_2PQY)_gRh(CO)H$$

and

$$[Ar_2P(CH_2)_mY]_gRh(CO)H$$

wherein Ar and g have the previously defined meaning, m is 1 to 30, preferably 2 to 22, more preferably 2 or 3, most preferably 2; Q is a $C_1$ to $C_{30}$, preferably $C_2$ to $C_{22}$, more preferably a $C_2$ to $C_3$, most preferably a $C_2$ unsubstituted or substituted, preferably unsubstituted, saturated straight chain divalent organic radical, more preferably a polymethylene radical which can be interrupted by either oxygen or phenylene; Y is a noncharged organic, preferably hetero-organic substituent, preferably with 3 to 30 carbon atoms, having a higher steric requirement than methylene and polymethylene such as trihydrocarbylsilyl, quaternary tetraalkyl phosphonium, heterocyclic tertiary nitrogen, phosphine oxide, sulfone, carbonyl, carboxylate and sterically demanding hydrocarbon groups, the latter being exemplified by phenyl, triphenylmethyl, t-butyl, tris-hydroxy substituted butyl and the like.

As far as compounds having phosphorus based heteroorganic substituents for Y are concerned, chelate forming amines, phosphines and phosphonium salts are excluded from this application. Y is preferably a $C_1$ to $C_{30}$, preferably $C_1$ to $C_{10}$, organic radical selected from the group consisting of substituted and unsubstituted secondary and tertiary alkyl, substituted and unsubstituted aryl, preferably phenyl and heteroorganic radicals. The heteroorganic radicals are defined as radicals having an atom, with an unsatisfied valency to be bound to Q, which is either a carbon having a hetero-atom substituent or is a heteroatom itself. The heteroatoms are preferably oxygen, sulfur, phosphorus, silicon and nitrogen, more preferably carbonyl, O, sulfone, S, phosphine or phosphine oxide, P, silane, Si, and amide, N. Heteroorganic radicals, especially silyl radicals, are most preferred.

If the Q is substituted, the substituents are the same as previously defined for Ar and R. Exemplary Q radicals are ethylene, butylene, docosamethylene, tricontamethylene, phenyl bis (ethyl), ethylene bis (oxyethyl), ethylene-bis oligo (oxyethyl), oxy ethyl propyl, oxy ethyl perfluoroethyl, oxy ethyl hydroxypropyl.

When Y is an alkyl radical, it is preferably saturated open chain and/or cyclic. The preferred substituent is hydroxy. Unsubstituted secondary and tertiary alkyl radicals are another preferred type.

In case Y is an aryl radical, it is preferably substituted or unsubstituted phenyl, most preferably phenyl.

Oxygen based heteroorganic radicals for Y are hydroxy, carbonyl, carboxylate, acyloxy, ether, more preferably hydroxy, ether, carbonyl, acyloxy. Sulfur based heteroorganic radicals are thiyl and sulfonyl. Phosphorus based heteroorganic radicals are diarylphosphino, dihydrocarbylphosphate, dihydrocarbylphosphonate, dihydrocarbylphosphite. Nitrogen based heteroorganic radicals are amino and those of reduced basicity, i.e., amido, ureido, imido, amine oxide, bis-(hydroxyethyl) amine. Cyclic amido, such as N-2 pyrrolidinonyl, is preferred.

Exemplary Y radicals are the following: tri-methylsilyl, tripropylsilyl, triphenylsilyl,—diphenyl phosphine oxide, diisobutyl phosphine oxide, diphenyl phosphine, dihydroxypropyl phosphine, dipropyl phosphite, diphenyl phosphate, tributyl phosphonium benzene sulfonate, didecyldibutyl phosphonium tetraphenyl borate, benzyl dicyclohexyl phosphonium methane sulfonate—pyrryl, dimethylpyrryl, pyrrolidinonyl, morpholinyl, acetamido, benzamido, amido, carbamido, ureido, bis-hydroxyethylamino—phenyl sulfone, fluorophenyl sulfone, ethyl sulfone, ethylthio, phenylthio—acetyl, benzoyl, carbomethoxy, benzoyloxy, carbobenzoxy, acetate, benzoate phenylacetate, hydroxy, carbamate, phenoxy; i-propyl, phenoxyphenyl, diisobutyl, cyclopentyl, diisopropylamino, anilino, diphenylamino, furyl, mesityl, pentafluorophenyl, tetrahydronaphthyl, tris(hydroxymethyl) methine.

In case Q is bound to a y+1 valent heteroorganic radical, E, the hydrocarbyl substituents of E are indicated by the symbol R''':

$$(Ar_2PQER_y''')_gRh(CO)H$$

and

$$[Ar_2P(CH_2)_mER_y''']_gRh(CO)H$$

wherein the meaning of Ar, Q, g and m is the same as defined previously; E is the inorganic part of the heteroorganic radical, $ER_y'''$ is a noncharged, nonchelating heteroorganic radical selected from the group consisting of silane silicone, ether, ester, keto and hydroxy oxygen, phosphine and phosphorus ester phosphorus, amine, amide, amine oxide and heterocyclic nitrogen, sulfide and sulfone sulfur; and R''' is an independently selected $C_1$ to $C_{30}$, preferably $C_1$ to $C_{10}$, substituted or unsubstituted, preferably unsubstituted or monosubstituted, more preferably unsubstituted hydrocarbyl radical. R''' is preferably selected from the group of hydrocarbyl radicals consisting of $C_1$ to $C_6$ alkyl, $C_5$ and $C_6$ cycloalkyl, phenyl, $C_1$ to $C_6$ monosubstituted alkyl, monosubstituted phenyl. More preferably, R''' is $C_1$ to $C_6$ alkyl or phenyl. As such, the R''' groups include methyl, propyl, ω-trifluoropropyl, pentafluoropropyl, pentafluorophenylethyl, phenyl, cyclotetramethylene, tolyl, methylcyclopentyl, decyl, fluoropropyl, benzyl, cyclohexyl, fluoropentyl, methoxyethyl, tricosyl, hydroxyethyl, methoxyethoxyethyl. Further examples of R''' were given when listing examples of the Ar and R' hydrocarbyl groups.

Examples of diaryl phosphine substituted derivative catalysts are non-chelating compounds of the formula

$$[Ar_2PQPAr_2]_3Rh(CO)H$$

$$[Ar_2P(CH_2)_mPAr_2]_3Rh(CO)H$$

In the case of these compounds, m is 4 to 22, more preferably 6 to 14. Such compounds are

$$[Ph_2P(CH_2)_4PPH_2]_3Rh(CO)H$$

$$[Ph_2P(CH_2)_{14}PPh_2]_3Rh(CO)H$$

$$[Ph_2P(CH_2)_6PPh_2]_3Rh(CO)H$$

Some alkylene bis-(diphenyl phosphine) ligands were studied by $^{31}P$ nmr. The parameters obtained for the free and complexed ligands of this type are summarised in the following Table. The first two bis-phosphines of the table are chelate forming compounds (Examples A and B). These were studied for comparison only. The next four compounds, i.e. polymethylene bis-phosphines did form the open chain tris-phosphine catalyst complexes as disclosed above (Examples C, D, E and F). Overall, the stability of the complex solutions increased in the order of their listing as indicated by the colour stability. Comments on some of the details are made in the following.

The reaction mixture of methylene bis-diphenyl phosphine and the TPP complex was highly unstable (Example A). Some of the TPP was displaced but no single new complex predominated. The mixture rapidly turned dark.

The complex derived from the trimethylene bis-phosphine was of further increased stability and reduced ligand exchange (Example B). Based on the complicated set of doublet signals, the presence of chelating bis-phosphine complexes such as the compound shown in the table is suggested.

The complexing behaviour and the nmr parameters of the non-chelating polymethylene bis-diphenyl phosphines (Examples C to F) were, in general, very similar to that of the simple n-alkyl diphenyl phosphines. More particularly, the ligand exchange rates observed were very similar to those previously found for the SEP ligand. In the presence of excess ligand, mostly one phosphine moiety of the bis-phosphine was coordinated. The phosphine group at the other end was mostly free as indicated by the formulae of the Table.

TABLE

$^{31}P$ Nuclear magnetic resonance parameter of free and rhodium complexed alkylene bis-(diphenyl phosphines)

| Example No. of Complex | Example No. of Ligand | Chemical structure of complex (Rh(CO)H) | Chemical shift; ppm Free ligand | Chemical shift; ppm Complexed ligand | Coupling constant P-Rh complexed ligand | Chemical shift difference, ppm complex ligand |
|---|---|---|---|---|---|---|
| A | 16 | $Ph_2PCH_2PPh_2$ | −24.0 | | | |
| B | 18 | 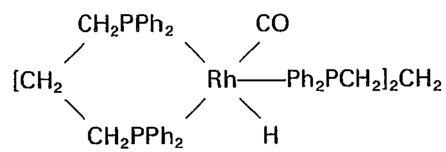 | −19.7 | | | |
| C | 19 | $[Ph_2P(CH_2)_4PPh_2]_3Rh(CO)H$ | −18.4 | +29.8 | 148 | 48.2 |
| D | 20 | $[Ph_2P(CH_2)_5PPh_2]_3Rh(CO)H$ | −18.5 | +27.7 | 153 | 46.2 |
| E | 21 | $[Ph_2P(CH_2)_6PPh_2]_3Rh(CO)H$ | −18.3 | +26.9 | 153 | 45.2 |
| F | 22 | $[Ph_2P(CH_2)_{14}PPh_2]_3Rh(CO)H$ | −18.6 | +27.5 | 152 | 46.1 |

The preferred olefin reactants for use in the hydroformylation process of the present invention are ethylene and its mono- and disubstituted derivatives. The formula of the preferred compounds is shown in the following representation of the hydroformylation reaction:

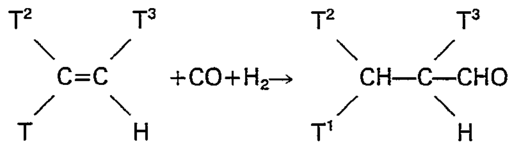

wherein $T^1$, $T^2$ and $T^3$ are independently selected from hydrogen and organic radicals containing from 1 to 1000 carbon atoms, preferably from 1 to 40 carbon atoms, more preferably from 1 to 12 carbon atoms, and most preferably from 4 to 6 carbon atoms, with the proviso that at least one of $T^1$, $T^2$ or $T^3$ be hydrogen. These radicals can be unsubstituted or substituted, but preferably they are unsubstituted.

As such, the preferred olefins include symmetrically disubstituted, i.e., internal, olefins of the formula

$$T^3—CH=CH—T^2$$

wherein the meanings of $T^3$ and $T^2$ in this case is the same as above except that they exclude H. Other, particularly preferred olefins are mono- and disubstituted unsymmetrical olefins of the formula

$T^1CH=CH_2$ and 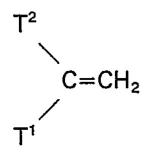

wherein the meaning of $T^1$ and $T^2$ in this case also excludes hydrogen. The monosubstituted olefins are particularly preferred. Such specifically preferred olefins are propylene, 1-butene, 1-pentene, 1-hexene, 1-heptene, 1-octene. The unsubstituted parent compound, ethylene, is also a specifically preferred reactant.

As far as the terminal versus internal attack of unsymmetrically substituted olefines is concerned, the disubstituted compound is a highly specific reagent in hydroformylation. It leads to mostly terminal or so-called n-aldehydes:

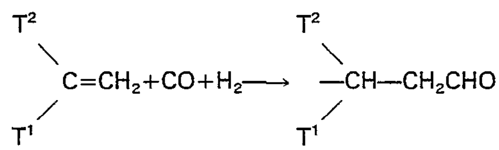

Specifically, preferred reagents of this type are isobutene, 2-methylbutene, 2-methylpentene, 2-ethylhexene.

In contrast to these disubstituted alpha-olefins, the selectivity of the hydroformylation of unsymmetrically monosubstituted olefins in the process of the present invention and fully explained further below depends on the excess phosphine concentration and CO partial pressure. The preferred course of the reaction is via terminal attack on the olefin to produce the corresponding n- rather than i-aldehydes:

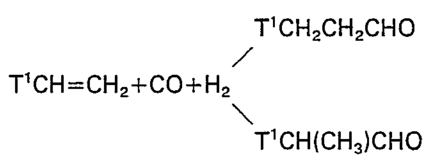

The size, i.e., steric demand of the $T^1$ substituent, also affects the selectivity. In the case of propylene, having the small methyl group for $T^1$, the selectivity to the n-product is relatively small. 1-Butene, with ethyl for $T^1$, is hydroformylated with surprisingly higher selectivity. 3-Methyl-1-butene, where $T^1$ equals i-propyl, reacts even much more selectively. Apparently, the bulkier and more branched $T^1$ groups hinder the attack on the internal, beta-vinylic carbon. The preferred monosubstituted olefins are n-1-olefins, wherein $T^1$ is n-alkyl. Particularly preferred reactants are 1-butene and propylene.

Exemplary olefinic reactants for use in the process of the present invention can be of open chain or cyclic structure. There can be a multiplicity of double bonds present in the higher molecular weight reactants. However, diolefin and polyolefin reactants of nonconjugated character are preferred. The saturated carbon atoms of these olefins can have non-hydrocarbon substituents such as hydroxy, carbonyl,

carboxylate, ester, alkoxide, acetal and fluorine groups. Of course, these substituents must not react with the components or the products of the hydroformylation reaction systems. Suitable cyclic olefins or olefins having a multiplicity of double bonds include 1-hexadecene, 3-hexene, cyclohexene, 1,7-octadiene, 1,5-cyclododecadiene, methyl cyclopentene, 1-tricosene, 1,4-polybutadiene, methyl oleate, ethyl 10-undecanoate, 3-butenyl acetate, diallyl ether, allyl fluorobenzene, 6-hydroxyhexene, 1-hexenyl acetate, 7-heptenyl diethyl acetal, norbornene, dicyclopentadiene, methylene norbornene, trivinyl cyclohexane, allyl alcohol.

In the drawings

Figure 1 shows the key steps in the mechanism of phosphine-rhodium complex catalyzed hydroformylation of olefins.

Figure 2 illustrates a comparative $^{31}$PNMR spectra of certain rhodium complexes.

Figure 3 illustrates a $^{31}$P NMR study of certain ligand exchange rates as a function of temperature.

Figure 4 is a schematic representation of an autoclave for hydroformylation.

Figure 5A is a graphical comparison of certain catalyst stabilities at high temperature hydroformylation of butene-1.

Figure 5B is a graphical comparison of certain catalyst stabilities at low ligand/Rh ratio in Hydroformylation of butene-1.

Figure 6 is a graphical representation of butene hydroformylation rate versus temperature correlations in the presence of SEP and TPP based rhodium-phosphine complex catalysts.

Figure 7 is a graphical representation of 1-butene hydroformylation temperature versus n-valeraldehyde (n) to total valeraldehyde (n+i) ratio correlations in the presence of SEP and TPP based rhodium-phosphine complex catalysts.

Figure 8 is a graphical representation of 1-butene hydroformylation temperature versus butane by-product formation correlations in the presence of SEP and TPP based complex catalysts.

Figure 9 is a graphical representation of 1-butene hydroformylation temperature versus 2-butenes by-product formation correlations in the presence of SEP and TPP complex catalysts.

Figure 10 is a graphical representation of the effect of SEP ligand to rhodium ratio on selectivity of butene hydroformulation at 145 and 170°C.

Figure 11 is a graphical representation of the effect of carbon monoxide pressure on ratio of n-valeraldehyde (n) to total valeraldehyde (n+i) product of 1-butene hydroformylation.

Figure 12 is a graphical representation of aldehyde production rate during continuous butene hydroformylation.

While we do not wish to be limited by any theory by which the process of the invention and our novel complexes work, it is believed that in solution and particularly under reaction conditions, both the tris- and bis-phosphine rhodium complexes are present. It was found via $^{31}$P nmr studies that the widely accepted equilibration mechanism of tris- and bis- phosphine rhodium carbonyl hydride complexes occurs according to the reaction formula:

$$(Ar_2PR)_3Rh(CO)H \underset{+Ar_2PR}{\overset{-Ar_2PR}{\rightleftharpoons}} (Ar_2PR)_2Rh(CO)H$$

tris-phosphine complex — trans-bis-phosphine complex

The overall mechanism of hydroformylation catalysis of the present complexes is shown by Figure 1. In Figure 3, there is shown a side-by-side comparison of the $^{31}$P nmr spectra at 30°, 60° and 90°C. for solutions containing, on the one hand, $(Ph_3P)_3Rh(CO)H$ and $Ph_3P$(TPP) as excess ligand, and on the other hand, containing $(Ph_3P)_3Rh(CO)H$ and excess $Ph_2PCH_2CH_2Si(CH_3)_3$ ligand (SEP ligand) as starting materials.

Equilibration of the stable tris-phosphine complex to provide some of the highly reactive, coordinatively unsaturated trans-bis-phosphine is to occur in an active catalytic system. However, in the case of stable catalysts, most of the rhodium is present in the stable tris- phosphine complex form.

Figure 3 also shows that the line shapes of the signals of the 30°C. spectra of both systems showed little signal broadening in both cases. This indicated comparably slow exchange rates of about 25 per second. In alternative terms, relatively long average exchange lifetimes, in the order of $2\times10^{-2}$ sec, were indicated for both complex systems tested. At 60°C., considerable line broadening occurred, indicating a much faster exchange. The exchange acceleration was greater in the case of the excess triphenyl phosphine ligand system (k=600 vs. 80), indicating an average lifetime of about $3\times10^{-3}$ sec for the excess triphenyl phosphine ligand system and of about $6\times10^{-3}$ sec for the excess trimethylsilylethyl diphenyl phosphine ligand system. At 90°C., only a single, broad signal could be observed for the excess triphenyl phosphine ligand system, while the excess trimethylsilylethyl diphenyl phosphine ligand system still exhibited separate, although extremely broad, chemical shift ranges for the complexed and free phosphorus species. Apparently, the exchange acceleration in the case of the excess triphenyl phosphine ligand system was tremendous at 90°C. with the average lifetime between exchanges being reduced by about two orders of magnitude to $5\times10^{-5}$ sec (k=10,000). In the case of the excess trimethylsilylethyl diphenyl phosphine

ligand system, the average lifetime dropped by about one order to $5 \times 10^{-4}$ sec (k=1,500). It must be emphasized that the exchange rates and lifetimes may change somewhat when the lineshape is subjected to a rigorous computer analysis. The relative order of their values will remain unaltered, however.

It is interesting to note that there was no great change of equilibria with the increasing exchange rates. Apparently, both ligand elimination and addition increase similarly in this temperature range. The tris-phosphine rhodium species remained the dominant form of complexes. However, in the triphenyl phosphine complex system including free excess trimethylsilylethyl diphenyl phosphine ligand, the rhodium predominantly complexed with the SEP ligand.

The role of excess phosphine ligand is apparently to maintain the equilibria in favor of the tris-phosphine complex, i.e., to reduce both the concentration and average lifetime of the unstable and highly reactive bis-phosphine complex. The increased ligand exchange rate provides enough active bis-phosphine complex catalytic species for fast hydroformylation, without leading to non-catalytic side reactions, i.e., catalyst decomposition.

Our comparative $^{31}P$ nmr studies of the known TPP catalyst plus excess TPP ligand system showed that it has a mechanism similar to that of the SEP system of the invention; however, the thermal activation and catalyst destabilization of the TPP system occurs at lower temperatures than for the SEP system. In other words, the tris(alkyl diaryl phosphine) rhodium carbonyl hydride plus excess alkyl diaryl phosphine-based systems of the present invention are surprisingly improved high temperature hydroformylation catalysts systems.

The results of $^{31}P$ nmr studies of tris-phosphine rhodium complex formation were also correlated with catalyst activity. It was found that those alkyl diphenyl phosphines which do not form tris-phosphines complexes for steric reasons are not suitable ligands for the present selective catalysts. Also, it was found that substitution of the alpha-carbon and multiple substitution of the beta-carbon of the Q alkylene group and o,o'-substitutions of the Ar aryl groups of the ligands used in the process of the present invention generally interfere with the complexation, i.e., the desired catalyst formation.

Common five and six membered chelate type complexes of alkylene bis-phosphines, e.g.,

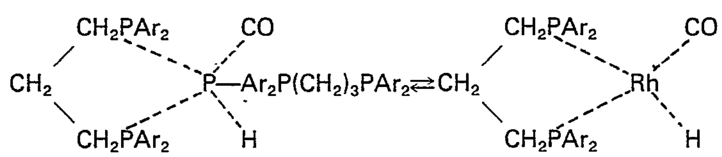

are not acceptable selective catalyst ligands either according to the concept of the process of the present invention, because they form bis-phosphine complexes of cis- rather than trans-configuration.

In the process of the present invention, substantially all of the excess phosphine ligand is an alkyl diaryl phosphine, preferably a phosphine ligand identical with that of the complex. Preferably, from about 1 to 90% by weight of the excess phosphine ligand is a diaryl alkyl phosphine and more preferably from about 5 to 50% by weight is a diaryl alkyl phosphine. In another preferred embodiment, the excess phosphine ligand consists essentially of an alkyl diaryl phosphine. By the terminology "consists essentially of" as used in this specification and in the claims attached hereto, we mean that only small amounts of non-diaryl alkyl phosphine ligand are present which will not affect the stability and selectivity of the catalyst system, e.g., such amount as might be present by forming the rhodium complex *in situ* starting with tris-(triphenyl phosphine) carbonyl hydrido rhodium complex and, as the sole excess ligand, a diaryl alkyl phosphine such as SEP or diphenyl propyl phosphine.

The rhodium complex catalysts are obviously very expensive due to the high cost of rhodium. As such, in the process of the present invention and in other processes employing the novel rhodium complexes described in European patent application No. 80900540.8, the rhodium complex concentration is to be carefully selected to be most effective on a rhodium basis. Of course, a catalytically effective amount of the rhodium will be present.

The preferred concentration of the rhodium complex as used in the process of the present invention and in other processes employing the novel rhodium complexes described in the European patent application No. 80900540.8 is in the range of $1 \times 10^{-6}$ to $1 \times 10^{-1}$ mole of rhodium per mole of olefin reactant. More preferred concentrations are in the range of $1 \times 10^{-5}$ to $1 \times 10^{-1}$ mole of rhodium per mole of olefin and the most preferred range is $1 \times 10^{-4}$ to $1 \times 10^{-2}$ mole of rhodium per mole of olefin. Thus, the preferred rhodium concentration is normally in the range of from 10 to 1000 ppm. However, the preferred catalyst concentrations are directly affected by the concentration of free ligand present, especially the excess diaryl alkyl phosphine ligand. Since the excess phosphine reduces the concentration of the active bis-phosphine complexes, a larger excess reduces the effectiveness of the total rhodium complex present. The higher the ligand concentration, the higher the rhodium level required for a certain reaction rate. Nevertheless, a high phosphine concentration is employed in the process of the invention to achieve the desired catalyst stability and selectivity.

In the process of the invention, the minimum weight percent amount of excess ligand in the reaction medium is preferably about 1 wt.%, more preferably 5 wt.%. However, in general, the phosphine concentration is limited to 50 wt.% of the reaction mixture. At an appropriate rhodium concentration, the

reaction can be carried out using the excess diaryl alkyl phosphine as the solvent. Sufficient excess diaryl alkyl phosphine concentration is used in the preferred process to carry out the reaction at the desired temperature under the desired conditions with the desired selectivity and activity maintenance. The rhodium complex concentration can then be adjusted to achieve the desired reaction rate.

Due to the interdependence of the alkyl diaryl phosphine rhodium complex and the excess phosphine ligand in the process of the invention, the mole ratio of diaryl alkyl phosphine ligand to mole equivalent rhodium complex, L/Rh, is preferably in the range of from about 40 to about 3000. The L/Rh ratio is preferably above 120, more preferably above 240, most preferably above 400. In general, higher ratios are selected when the desired operation is a continuous rather than a batchwise operation.

The selectivity of the process of the present invention is also dependent on the molar ratio of gaseous CO and $H_2$ reactants. This $H_2/CO$ ratio should generously be greater than 3:1 preferably in the range of 200:1 to 3:1 and more preferably, from 100:1 to 5.5:1 and most preferably from 20:1 to 10:1.

The present process of the invention is also operated at surprisingly low pressures, but can be operated at pressures of from 1 to 10,000 psi. The preferred pressures are between about 1 to 1000 psi, i.e., about 1 and 68 atomsphere. It is more preferred to operate between about 25 and 500 psi, i.e., about 2 and 34 atm.

Some of the above pressure limitations are due to the sensitivity of the present rhodium complex catalyst to the partial pressure of CO. The total partial pressure of CO is preferably less than about 200 psi (approximately 8 atm.), more preferably less than about 100 psi, and most preferably less than about 50 psi. If the CO partial pressure is too high, the catalyst complex is deactivated due to the formation of carbonyl derivatives.

In the process of the present invention, the partial pressure of hydrogen has no critical upper limit from the viewpoint of hydroformylation. Nevertheless, the preferred partial pressure of hydrogen is between about 50 and 500 psi, i.e., 4 and 34 atm. Above a certain partial pressure of hydrogen, the relative rates of competing hydrogenation and isomerization reactions suddenly increase, which is to be avoided in the process of the present invention. However, if such hydrogenation and isomerization reactions are desired, the novel complexes of the invention are surprisingly active hydrogenation and isomerization catalysts. In the latter case, the rhodium complex of the invention becomes a multifunctional catalyst when the $H_2/CO$ ratio is too high and/or the CO concentration is insufficient.

When working with a terminal olefin, the selectivity to paraffin and internal olefin was sometimes significantly increased for the above reasons. For example, the reaction of 1-butene led not only to n- and i-valeraldehydes, but also to significant amounts of n-butane and cis- and trans-2-butenes. This effect of high hydrogen partial pressures becomes particularly critical under non-equilibrium conditions where the system is starved of CO. In such a case, practically only the n-aldehyde plus by-products are formed. In a preferred mode of operation, the optimum combination of reaction parameters is maintained by ensuring equilibrium conditions by appropriate reactant introduction and mixing.

In the upper temperature range of the process of the invention, a significant part of the total pressure can be maintained by either a volatile reactive or unreactive olefin or a saturated, aliphatic or aromatic hydrocarbon or by an inert gas. This preferred mode of operation allows a limitation of synthesis gas pressure, while assuring a higher solubility of the gaseous reactants in the liquid reaction mixture.

The operation of the process of the invention can be optimized in a surprisingly broad temperature range. The range of temperature is preferably between 50 and 200°C., more preferably, between 90 and 175°C., and most preferably, between 120 and 150°C. Compared to prior art catalyst systems employing triphenyl phosphine, the maintenance of the catalyst activity and selectivity at the higher temperatures in the process of the present invention is particularly unique. High rates of selective hydroformylation of alpha-n-olefins can be realized and maintained to high conversion at 145°C. when using the present process conditions.

The process of the invention can be carried out either in the liquid or in the gaseous state. The catalyst can be employed as such either dissolved in the liquid reaction medium or deposited on a suitable solid such as silica or alumina. The preferred process employs a liquid, more preferably homogeneous liquid, reaction phase with the catalyst system dissolved, i.e., homogeneous catalyst.

The preferred homogeneous catalysis process of the invention is affected by the solvents used although a large variety of organic solvents is employable. In general, the more polar solvents of higher dielectric constant are increasingly preferred as long as they possess sufficient solvent power for the olefin reactant and do not interfere with the stability of the desired catalyst complex species. As such, aromatic hydrocarbons are suitable solvents, although organic nonhydrocarbon solvents are preferably used. More preferably, the latter are of a weak non-substituting ligand character. As such, oxygenated solvents are most preferred.

Preferred solvents include those of ligand character, e.g., diaryl alkyl phosphine, or organic solvents, e.g., ketones such as acetophenone, diphenyl ketone; polyethylene glycol; organic silicone compounds such as diphenyl dipropyl silane; esters such as 2-ethylhexyl acetate, dipropyl adipate, ethylene glycol diacetate; 1,4-butane diol; dimethyl formamide; N-methyl pyrrolid 4-hydroxybutyl 2-ethylhexanoate. One of the most preferred solvents is an excess of the alkyl diaryl phosphine ligand. Other organic non-hydrocarbon solvents preferably of weak nonsubstituted ligand character which can advantageously be used are triaryl phosphines, for example, triphenyl phosphine, triaryl stibines and triaryl arsines.

In general, the preferred solvents for the process of the invention, particularly ligands, stabilize the catalyst system and increase its selectivity, particularly as to the ratio of linear versus branched products. The aldehyde product of the invention is generally an excellent solvent. Accordingly, the addition of a separate solvent is not required.

In contrast to the disclosure on the triphenyl phosphine type rhodium complex catalyst system of the previously discussed U.S. Patent No. 4,148,820 by Pruett and Smith, the alkyl diaryl phosphine rhodium complex catalyst systems used in the process of the present invention and the novel rhodium complexes described in European patent application No. 80900540.8 (Pub No. WO 80/01690) are compatible with, i.e. soluble in, a large variety of organic solvents. These solvents include the aldehyde trimer condensation products which, according to Pruett and Smith, are the only suitable solvents for the triphenyl phosphine type based rhodium catalyst system.

Due to the improved stability provided by the process of the present invention employing alkyl diaryl phosphine rhodium complex catalysts, a continuous mode of operation is often advantageous. When using a homogeneous liquid catalyst system, such an operation can be of a continuous plug flow type, including a step for catalyst recovery and then recirculation. The process of the present invention may also involve a quasi-continuous use of the catalyst employing the cyclic operation of a unit for hydroformylation and then for product flash-off. Catalyst concentration or other methods of catalyst recovery may involve complete or partial recycle. However, a preferred method of operation for the process of the present invention involves continuous product flash-off.

In the continuous product flash-off process of the present invention, the aldehyde product of the hydroformylation is continuously removed as a component of a vapor mixture, while the CO, $H_2$ and olefin reactants are continuously introduced. This process preferably includes the recirculation of most of the unreacted reactants in the gaseous state and the condensation and thereby removal of most of the aldehyde and aldehyde derivative products. Additional olefin, CO and $H_2$ are added as required to maintain aldehyde production and optimum process parameters. The space velocity of the gas stream is appropriately adjusted and additional gas purge is used as required to maintain production and catalyst activity. Since the rhodium complex is not volatile, no catalyst losses occur. If the diaryl alkyl phosphine ligand is volatile, additional phosphine is added occasionally to maintain its concentration in the reaction mixture.

During the continuous product flash-off operation, relatively non-volatile aldehyde, oligomers are formed and concentrated in the liquid reaction mixture. The oligomeric hydroxy substituted carboxylic ester condensation and redox disproportionation products formed during propylene hydroformylation were disclosed in the previously discussed U.S. Patent No. 4,148,820 of Pruett and Smith. In the present work, it was found that analogous derivatives, mainly trimers, are formed during 1-butene hydroformylation. The general structure of the isomeric trimers is the following:

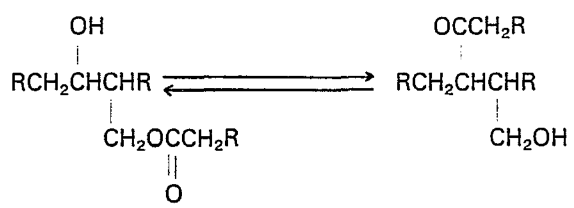

wherein R is $C_2$ to $C_5$, preferably $C_3$, alkyl.

The above aldehyde trimer is generally the main derivative and at equilibrium conditions of a preferred continuous flashoff process of the present invention, it can automatically become the main solvent component. When this occurs during 1-butene hydroformylation in accordance with the process of the present invention, selectivity and production rate can be maintained and the concentration of the trimer can be limited to an equilibrium value.

In the continuous product flash-off operation of the process of the present invention, carbonylations, especially the hydroformylation of olefins, are advantageously carried out at a low olefin conversion, preferably at a 20 to 80% olefin conversion. Aldehyde production rates are preferably between 0.1 and 5 g mole/liter/hour, more preferably between 0.5 and 2 g mole/liter/hour. Operating in this manner with optimized reactant ratios, particularly high linear to branched aldehyde product ratios are obtained from alpha-n-olefins.

The continuous process of the present invention can be also employed for the selective or complete conversion of different types of olefins. For example, a mixture of 1- and 2-butenes can be hydroformylated to produce mainly n-valeraldehyde and 2-butene. Similarly, a mixture of 1-butene, 2-butene and i-butene can be converted selectively to varying degrees.

Using the process of the present invention, the catalysts have improved thermal stability and thus the application of continuous or batch flashoff processes can be extended to higher olefins leading to aldehyde products which are not sufficiently volatile at the normal lower temperatures used in previous continuous operations. The preferred olefins for continuous product flashoff are of the $C_2$ and $C_6$ range and alpha-n-olefin type. 1-Butene and propylene are particularly preferred.

Using the present catalysts of improved thermal stability, the application of continuous or batch flashoff processes can be extended to higher olefins leading to nonvolatile products. The preferred olefins for continuous product flashoff are of the $C_2$ to $C_6$ range and n-1-olefin type. 1-Butene is a particularly preferred reactant.

In an improved method for continuous hydroformylation, 1-butene is reacted with CO and $H_2$ in the presence of a tris-(2-trimethylsilylethyl diphenyl phosphine) rhodium carbonyl hydride complex and excess 2-trimethylsilylethyl diphenyl phosphine ligand based catalyst system wherein the reactants are continuously introduced into a liquid reaction mixture comprising dissolved catalyst and ligand and preferably major amounts of n-valeraldehyde trimer, and wherein the aldehyde products are continuously removed in the vapor phase, and wherein some of the reactants are recirculated. The improvement is effected by having the carbon monoxide partial pressure between about 4 and 100 psi, preferably between about 80 and 70 psi; the hydrogen partial pressure, preferably between about 5 and 500 psi; and the total gas pressure between 25 and 500 psi, preferably between 55 and 500 psi; a rhodium complex concentration between $1\times10^{-2}$ and $1\times10^{-4}$ mole/liter and phosphine ligand concentration between 5 and 50 wt. percent, in a temperature range between 80° and 175°C, preferably 110 and 145°C. The above concentrations and temperatures are selected to provide appropriately high $H_2$/CO and Rh/L ratios to constitute an effective catalyst system in the present continuous operation. Such a system produces 0.5 to 2 g mole/liter/hour aldehyde and loses less than 1%, preferably less than 0.3%, of its activity per day while a n/i-aldehyde product ratio in excess of 9, preferably in excess of 15, is maintained.

The process of the present invention employing alkyl diaryl phosphine rhodium complexes can also be advantageously combined with other processes because of the thermal stability and selectivity of the catalysis obtained by such processes. The hydroformylation could be advantageously carried out either when coupled with aldol condensation alone or when coupled with aldol condensation and hydrogenation. Such combined processes are highly selective to the corresponding aldehydes. For example, in the case of terminal olefins, such as alpha-olefin reactants, the following main aldehyde forming reactions take place when the present alkyl diaryl phosphine rhodium complex hydroformylation and hydrogenation catalyst is combined with a base catalyst for aldolization such as KOH:

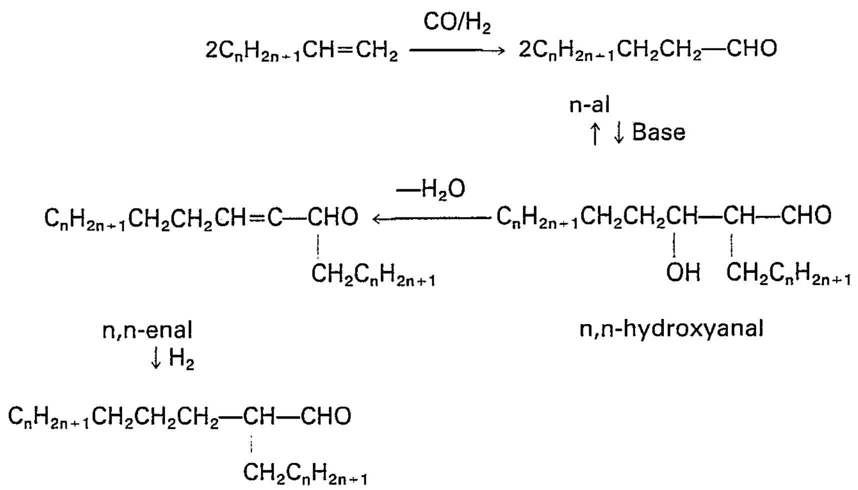


n-al

n,n-hydroxyanal

n,n-enal

n,n-anal

wherein the simple n-aldehyde product of hydroformylation is n-al, the thermally unstable primary product of aldolization is n,n-hydroxyanal, the unsaturated aldehyde resulting from dehydration is n,n-enal, and the selectively hydrogenated final saturated aldehyde is n,n-anal, wherein the n,n-prefixes indicate that both segments of the aldol compounds are derived from the terminal, i.e., normal, product of the hydroformylation. For known, applicable aldolization catalysts, reference is made to Volume 16, Chapter 1 of the monograph Organic Reactions, edited by A.C. Cope et al., published by J. Wiley and Sons, Inc., New York, N.Y., 1968.

It has been found in accordance with the invention that a combined hydroformylation/aldolization using alkyl diaryl phosphine ligands, in a large excess over the rhodium complex catalyst and a high ratio of the $H_2$/CO reactant gas resulted in a catalyst system of higher thermal stability and provided a high normal to iso isomer ratio in the production of dimer aldehyde product from alpha-olefins. It has been found that the presence of a diaryl alkyl phosphine rhodium catalyst results in greater effectiveness for the aldolization step than with base alone being present. This is especially important for water insoluble $C_6$ and higher aldehyde aldolization with small amounts of base, preferably alkali hydroxide.

A combined process also converts some of the i-aldehyde products in a so-called cross-aldolization reaction with the n-aldehyde:

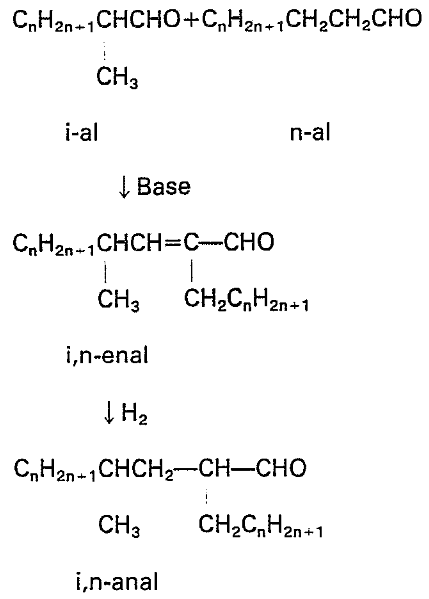


The rate of the above cross-aldolization process is slower than that of the simple aldolization. However, the relative rate of cross-aldolization increases with increasing temperature and decreasing n/i aldehyde ratios. The latter can be achieved by the addition of extra i-aldehyde to the reaction mixture.

Since high aldolization rates can be readily achieved in a combined process, the reaction parameters can be readily adjusted to provide either the unsaturated or saturated aldehydes as the major products. Short reaction times, and low olefin conversion, preferably below 50%, plus high base concentration, favor the unsaturated aldehyde. However, mostly the saturated aldol condensation product is desired. This is, of course, the favored high conversion product. It is important to note that no alcohol by-products are formed even at high olefin conversions of 80% and higher.

The preferred concentration of the strong inorganic base, i.e., alkali hydroxide, aldolization catalyst is surprisingly low, between about 0.01 and 1%, preferably between 0.05 and 0.5%.

Solvent selection is important in a preferred homogeneous, liquid phase, combined process. The preferred solvent will dissolve all the widely different components of the reaction system. Solvency for the nonpolar olefin reactant and polar caustic catalyst and water by-product require a compromise. Alcohols, particularly hydrocarbyloxyethyl alcohols are preferred. The latter are preferably of the formula

$$J(OCH_2CH_2)_jOH$$

wherein $J=C_1$ to $C_4$ alkyl, preferably primary alkyl, most preferably methyl; $C_6$ to $C_{10}$ substituted or unsubstituted phenyl, preferably phenyl; and j is an integer of from 1 to 8, preferably from 3 to 8. Such preferred solvents include methoxytriglycol, $CH_3(OCH_2CH_2)_3OH$, and phenoxyethanol, $PhOCH_2CH_2OH$. In general, the weight proportion of the relatively nonpolar hydrocarbyl segment J to that of the highly polar oligo(-oxyethyl) alcohol segment determines the relative solvent power for the nonpolar versus polar components of the reaction mixture. As such, this type of a solvent can be readily optimized for any special application of the present process. The relatively high overall polarity of this solvent ensures both homogeneous reaction and a high n/i ratio of the primary products of the combined process.

With exception of the use of the base and the use of a polar, non-hydrocarbon solvent, the conditions of the present combined process are generally the same as those of a simple hydroformylation.

General method of hydroformylation

The hydroformylation of butene-1 to provide linear pentanal and branched 2-methyl butanal products was selected for comparative studies of the catalytic properties of certain of the alkyl diaryl phosphine complexes of the invention. The complexes studied were either isolated before use or generated *in situ*. In some cases, the desired complex was generated from the known tris-(triphenyl phosphine) rhodium carbonyl hydride by the addition of the appropriate ligand in varying amounts. According to another standard method, dicarbonyl acetylacetonato rhodium and the appropriate alkyl diaryl phosphine were used as catalyst precursors. In that case, the desired rhodium carbonyl hydride complex was generated by hydrogenation during the hydroformylation experiment. Tris-(triphenyl phosphine) rhodium carbonyl hydride in the presence of varying excesses of triphenyl phosphine was used as a known catalyst standard for comparison.

The experiments were carried out in a 300 ml stainless steel (S) and a 300 ml Hastelloy (H) autoclave, respectively. Both autoclaves were equipped with identical, highly effective, impeller type stirrers, operating at 750 rpm during the experimental runs. The other standard autoclave instrumentation was

identical for both units. However, a slightly lower normal to iso aldehyde product ratio (n/i) was observed in unit H. In those cases where the type of autoclave was not specified, a stainless steel unit was used.

The standard batch hydroformylation procedure was the following: the appropriate amounts of rhodium complex were dissolved in 100 g of the proper mixture of a free phosphine and solvent. 2-propylheptyl valerate or 2-ethylhexyl acetate were used as standard solvents. They were indistinguishable. Most often the amount of complex employed provided 100 ppm rhodium concentration. This meant 100 mg, i.e., about 0.1 mmole rhodium per 100 g. Accordingly, 100 mg per Kg, about 1 mmole per kg rhodium would be present in 1 kg starting mixture. The excess ligand added to the solvent was usually calculated to provide a ligand to rhodium ratio (L/Rh) of about 140.

The 100 g rhodium complex-ligand solution was placed into the autoclave which was then deaerated by repeated pressurization with nitrogen. The solution under atmospheric nitrogen pressure was then sealed and heated to the reaction temperature, usually 100°C.

When the solution reached 100°C, 20 g of liquid butene was pressured into the autoclave with a 1 to 4 carbon monoxide-hydrogen initial gas mixture. The butene was followed by the $CO/H_2$ mixture until a pressure of 350 psig was reached. At that point, the supply of 1:4 $CO/H_2$ was shut off and the autoclave was connected to a cylinder of about 1 liter volume containing a 1:1 $CO/H_2$ feed gas mixture at 1000 psig. The connection was made through a pressure regulating valve set to provide the 1:1 $CO/H_2$ gas to the autoclave to maintain a 350 psig pressure during the reaction. The exact $H_2/CO$ ratio of the feed gas was often varied to maintain the initial $H_2/CO$ ratio in the autoclave.

In the standard tests, the autoclaves used were equipped with synthesis gas feed lines adjoining the autoclave above the Magnedrive stirrer assembly unit (Figure 4). It is to be noted that this manner of introducing synthesis gas feed far from the upper level of the liquid reaction mixture resulted in an incomplete equilibration of the synthesis gas mixture between the gas and liquid phase. Particularly in those cases where the initial synthesis gas mixture (used to pressure up the reaction mixture) had an $H_2$ to CO ratio of 10 or higher, the CO component of the subsequent one to one feed gas was not effectively delivered from the top into the liquid reaction mixture due to mass transfer limitations. Therefore, the reaction mixture was often "starved" of CO during the early fast phase of the reaction. As a consequence, the $H_2/CO$ ratio in the liquid temporarily rose to very high values. This resulted in particularly high n- to i-aldehyde product ratios. Also, olefin hydrogenation and isomerization became important side reactions. This, of course, reduced the absolute accuracy of the data on catalyst selectivities. Nevertheless, in a relative sense, the data are correct in all cases. For comparison, the widely studied Tris-TPP rhodium carbonyl hydride catalyst system was used as a standard throughout the work.

In those instances, where the effect of $H_2$ to CO ratios and the effect of CO partial pressure were specifically studied, the synthesis gas feed was introduced at the side of the autoclave, just above the liquid level. This method of operation largely avoided any temporary rise $H_2/CO$ ratios and drastically reduced hydrogenation and isomerization in cases where the initial $H_2/CO$ ratio was high. Special studies were also made in a continuous feed introduction and product flashoff operation. This allowed a continuous control of partial pressures and such provided the most accurate results (Figure 12).

The progress of the hydroformylation was followed on the basis of the amount of 1:1 $CO/H_2$ consumed. The latter was calculated on the basis of the pressure drop in the 1 liter $CO/H_2$ cylinder. Reactant conversion calculated on the basis of CO consumption was plotted against the reaction time to determine the reaction rate. The reaction rate was expressed as the fraction of the theoretical $CO/H_2$ requirement consumed per minute ($k\ min^{-1}$). The reaction was discontinued when the reaction rate drastically dropped. Dependent on the side reaction, such as butene-1 hydrogenation and butene-1 to butene-2 isomerization, the stability of the catalyst complex in the mixture, such a rate drop occurred generally between 80—98% conversion. Accordingly, the reactions were usually discontinued in that conversion range. Most often the reactions were run up to 80% conversion.

When the reaction was to be discontinued, the $CO/H_2$ feed valve was shut and the autoclave was immediately cooled with cool water. In case of low conversions, ice bath was used. When cooling was complete, the synthesis gas was released slowly. The residual liquid was visually observed for catalyst decomposition. A dark orange to brown color of the originally yellow mixture indicated increasing degrees of catalyst decomposition. Severe catalyst decomposition usually resulted in the precipitation of dark solids.

Analysis of the residual liquid mixture were carried out using gas chromatography. The ligands were analysed in a gc instrument using flame ionization detector. By this instrument, the $C_4$ hydrocarbons were detected. Due to the lower response of this detector to the aldehydes, the intensity of the hydrocarbon peaks was multiplied usually by 0.7 to obtain the necessary concentration correction. The individual, gaseous $C_4$ hydrocarbons were separated by another chromatograph. At first the gases were separated from the liquids and then the individual components of the gas were chromatographed and detected by a thermal conductivity detector.

1-Butene hydroformylation experiments (Examples 1—8)

In the following description of 1-butene hydroformylation catalysis by tris-(alkyl diaryl phosphine) rhodium carbonyl hydride based catalyst systems, at first their unique catalytic behavior will be exemplified by a detailed description of the tris-(trimethylsilylethyl diphenyl phosphine) rhodium carbonyl

hydride, i.e. SEP complex, plus SEP system. For comparison, detailed data will be also provided on the know tris-(triphenyl phosphine) rhodium carbonyl hydride, i.e. TPP complex, plus TPP system. This will be followed by short descriptions of the catalytic behavior of various substituted and unsubstituted alkyl diphenyl phosphines. Finally, an example of a continuous hydroformylation process based on the SEP system will be described.

The invention is illustrated with reference to the following Examples.

Example 1

Tris-(trimethylsilylethyl diphenyl phosphine) rhodium carbonyl hydride as a catalyst in the presence of 140-fold ligand excess at different temperatures

Tris-(trimethylsilylethyl diphenyl phosphine) was studied at the 107 ppm rhodium level in the presence of 140-fold trimethylsilylethyl diphenyl phosphine (SEP) ligand as a butene hydroformylation catalyst using the general procedure. Comparative experiments were run using 107 ppm rhodium as a tris-(triphenyl phosphine) carbonyl hydride complex with 140-fold triphenyl phosphine (TPP). Reaction rates, n/i product ratios, conversions and by-products were determined at various temperatures. The results are shown by Table 1.

The hydride was prepared as follows:

$$3(Ph_2PCH_2CH_2Si(CH_3)_3+RhCl_3\cdot 3H_2O\rightarrow[Ph_2PCH_2CH_2Si(CH_3)_3]_3Rh(CO)H$$

To a vigorously stirred, refluxing, nitrogenated solution of 11.44 g (49 mmole) of tris-(trimethylsilylethyl) diphenyl phosphine in 400 ml of ethanol, a hot solution of 1.04 g (0.4 mmole) of rhodium trichloride trihydrate in 80 ml ethanol was added at once. After a delay of 15 seconds, 40 ml warm aqueous (37%) formaldehyde solution and, immediately thereafter, 80 ml hot ethanolic solution of 3.2 g of potassium hydroxide were added. The resulting clear orange liquid reaction mixture was refluxed for 10 minutes. During the heating, the colour changed to deep orange.

The mixture was cooled to −25°C to crystallise the complex product. Crystallisation started at −10°C and was completed on standing for about 2 hours at −25°C. The crystalline complex was separated by filtration through a precooled Buechner funnel with suction and washing successively with 20 ml cold portions of ethanol, water, ethanol and n-hexane. The complex was then dried in the presence of anhydrous calcium chloride at 9.1 mm over the weekend. As a result, 2.2 g (2.2 mmole, 55%) of dry tris-(trimethylsilylethyl diphenyl phosphine) rhodium carbonyl hydride complex was obtained as a fine crystalline orange-yellow powder. In a sealed capillary tube, the complex melted between 126—129°C to a clear dark red liquid. In an open capillary, complete melting occurred at 121°C. There was no sign of decomposition on heating up to 140° in either case.

The infrared spectrum of the complex in Nujol showed a strong carbonyl band of 1985 $cm^{-1}$ and a band of medium intensity at 1900 $cm^{-1}$.

Analyses Calcd. for $C_{52}H_{70}OP_3RhS$: C, 63.01; H, 7.12; P, 9.38; Found: C, 62.89; H, 7.06; P, 9.59.

The data of the table show that both the SEP and the TPP based catalyst systems are highly active and produce a high ratio of n/i products at most temperatures. However, the temperature dependence of the two systems is very different.

TABLE I
Hydroformylation at different temperatures

Feed: Butene-1 and 1:4 $CO/H_2$ at 350 psi
Catalyst: $L_3Rh(CO)H$, Rh 107 ppm, Rh/L=140
SEP Ligand: $(CH_3)_3SiCH_2CH_2P\Phi_2$ TPP Ligand: $Ø_3P$

| Seq. No. | Variable conditions of catalysis: Catalyst ligand | Variable conditions of catalysis: Reaction temp., °C | Reaction rates and selectivities: Fraction of $CO/H_2$ reacted k, $min^{-1}$ | Product n/i ratio | Reaction time min. | Butene conversion % | By-Product mole % in product mixture: Butane | By-Product mole % in product mixture: Butene-2 | Details: Exact Rh conc. ppm | Details: Exp. run No. |
|---|---|---|---|---|---|---|---|---|---|---|
| 1 | SEP | 100 | 0.03 | 6.1 | 35 | 86.9 | 2.0 | 3.8 | 106 | 104 |
| 2 | | 120 | 0.10 | 6.2 | 35 | 96.5 | 9.4 | 6.2 | 106 | 110 |
| 3 | | 140 | 0.21 | 5.7 | 15 | 97.5 | 14.5 | 12.4 | 108 | 109 |
| 4 | | 145 | 0.27 | 5.0 | 15 | 95.2 | 11.9 | 12.1 | 109 | 115 |
| 5 | TTP | 100 | 0.21 | 7.5 | 35 | 98.9 | 10.7 | 12.1 | 107 | 100 |
| 6 | | 120 | 0.34 | 5.9 | 10 | 97.3 | 9.3 | 12.2 | 104 | 112 |
| 7 | | 140 | 0.38 | 3.4 | 10 | 98.2 | 11.2 | 21.9 | 105 | 113 |
| 8 | | 145 | 0.27 | 2.4 | 15 | 97.7 | 11.9 | 26.0 | 103 | 114 |

The novel SEP catalyst system exhibits an increasing activity with elevated temperatures. At 100°C. and 120°C. the n/i ratio of products is about the same and there is only a small n/i drop at 145°C. High butene conversion is observed at all temperatures. The only adverse effect of temperature increase is the increased hydrogenation and isomerization of the butene-1 reactant. The SEP system remains clear, bright yellow in appearance, even at 145°C.

The known TPP catalyst system exhibits the same increased activity at 120°C. and 140°C. However, the n/i ratios in this case are dramatically reduced with increasing temperatures. At 145°C., the n/i ratio products is significantly lower in the TPP than in the SEP system. At 145°C., the reaction rate of the TPP system also drops. Decomposition of this system at this temperature is indicated by darkening of the reaction mixture. The behavior of the SEP and TPP systems is compared by Figures 5A and 5B.

The results of similar but more extensive studies are shown by Figures 6 to 9. Figure 6 correlates the hydroformylation rate with the temperature. It shows that in the presence of the SEP complex catalyst, the rates of 1-butene hydroformylation were increasing with elevated temperatures up to 155°C. In the case of the TPP catalyst, increased rates were observed only to about 135°C. Beyond these temperatures, reduced hydroformylation rates were observed apparently due to catalyst decomposition.

Figure 7 correlates the hydroformylation temperature with the selectivity for producing the linear (n-) versus branched (i-) aldehyde. It is shown that the n/i ratios depend on the temperature in the case of both catalysts.

When hydroformylations are carried out above the stable temperature range of the catalysts, a drastic drop in the n/i ratios is observed. This drop occurs above 165°C for the SEP catalyst and above 135°C for the TPP catalyst. At the same hydroformylation temperature, the use of the SEP catalyst leads to somewhat higher n/i ratios. It appears that the SEP catalyst could be used at an about 20°C higher hydroformylation temperature than the TPP catalyst and would still exhibit a selectivity equal to that of TPP at the lower temperature.

With regard to the undesired hydrogenation of the 1-butene feed to produce n-butane (Figure 8), it is noted that, in the case of the SEP catalyst, the percentage of n-butane formed more than triples to about 11% when the reaction temperature is increased from 100°C to 135°C. However, there is very little increase between 135°C and 165°C. In contrast, limited data indicate that, in the case of the TPP catalyst, the level of hydrogenation stays around the 10% level between 100°C and 140°C.

The behavior of the SEP and TPP catalysts appears to be also different with respect to the isomerization of the 1-butene feed to cis- and trans-butene-2 by-products (Figure 9). In general, less isomerization occurs when the SEP catalyst is used. However, the percentage of isomerized olefin is increased with temperature in the presence of both catalysts, up to 165°C. When the SEP catalyst becomes unstable at 170°C, less 2-butenes by-products are obtained apparently due to secondary reactions, i.e., hydrogenation and hydroformylation. In contrast, the thermal destabilization of the TTP catalyst in the 140—145° range results in a large increase of the percentage of 2-butenes in the reaction mixture.

Example 2

Tris-(Trimethylsilylethyl diphenyl phosphine) rhodium carbonyl hydride as a catalyst at different levels of excess ligand concentrations

The complex catalyst used in Example 1 was studied mainly at the 105 ppm rhodium level and at 100° reaction temperature to determine the effect of the excess trimethylsilylethyl diphenyl phosphine ligand (SEP). The SEP concentration used ranged from 5 to 149 mmole per liter. Some comparative experiments were also carried out using tris-(triphenyl phosphine) rhodium carbonyl hydride and varying excess concentrations of the corresponding triphenyl phosphine ligand (TTP). The results of these studies are shown in Table 2.

The data of Table 2 show that, in general, increasing concentrations of excess ligand result in decreased reaction rates but sharply increased selectivities, i.e., n/i ratios, in both the novel and the known catalyst systems. There is an apparent inhibition and stabilization of both systems at high ligand concentrations. However, the behavior of the two catalysts is significantly different at relatively low excess ligand concentrations.

TABLE 2

Hydroformylation at different levels of excess ligand concentrations

Feed: Butene-1 and 1:4 $CO/H_2$ at 350 psi
Catalyst: $L_3Rh(CO)H$
SEP Ligand: $(CH_3)_3SiCH_2CH_2PØ_2$
TPP Ligand: $Ø_3P$

| | Variable conditions of catalysis | | | | | | Reaction rates and selectivities | | | |
|---|---|---|---|---|---|---|---|---|---|---|
| Seq. No. | Catalyst ligand | Reaction temp. °C | Auto-clave | Excess Ligand conc. mMole/lit. | Rhodium conc. ppm | Ligand to Rh ratio l/Rh | Fraction of $CO/H_2$ reacted k min-1 | Product linearity ratio, n/i | Reaction time min. | CO conversion % |
| 1 | SEP | 100 | 11 | 5 | 105 | 5.2 | 0.24 | 3.5 | 20 | 88.7 |
| 2 | | | | 24 | 105 | 24.2 | 0.09 | 4.0 | 35 | 87.1 |
| 3 | | | S | 28 | 105 | 28 | 0.12 | 4.4 | 35 | 88.0 |
| 4 | | | | 56 | 217 | 28 | 0.12 | 5.4 | 30 | 94.2 |
| 5 | | | | 143 | 105 | 143 | 0.03 | 6.1 | 35 | 83.6 |
| 6 | | 120 | S | 29 | 105 | 29 | 0.30 | 4.5 | 15 | 93.0 |
| 7 | | | | 60 | 210 | 30 | 0.25 | 5.7 | 15 | 89.6 |
| 8 | | | | | 149 | 105 | 0.10 | 6.2 | 35 | 88.1 |
| 9 | TPP | 100 | H | 5 | 105 | 5 | 0.28 | 3.0 | 15 | 89.8 |
| 10 | | | | 142 | 102 | 142 | 0.17 | 3.8 | 35 | 96.5 |
| 11 | | | S | 27 | 105 | 27 | 0.31 | 4.7 | 15 | 86.6 |
| 12 | | | | 143 | 104 | 143 | 0.03 | 6.1 | 35 | 83.6 |

The novel SEP catalyst system leads to higher n/i product ratio than the TPP system at five mmole/l excess ligand concentration (Seq. No. 1 vs. Seq. No. 9). At the intermediate SEP concentration of 56 mmole, there is a good selectivity and sufficient reaction rate (Seq. No. 4). It is interesting to observe that the positive effect of increasing catalyst complex concentration on the reaction rate can be counter-balanced by the inhibiting effect of increased SEP concentration (compare Seq. Nos. 3 vs. 4 and 6 vs. 7). Clearly, the SEP concentration is more important than the SEP/Rh ratio. At the high SEP level of 143, there is some further increase in the n/i ratio, but reaction rate is cut to about one fourth (compare Seq. Nos. 4 and 5). At this level, the rate can be increased while maintaining the high n/i ratio by increasing the reaction temperature (see Seq. No. 8 and the table of the previous example).

The effect of different ligand to rhodium ratios on the n/i ratios of butene hydroformylation at different temperatures was further examined. The results are summarized by Figure 10.

The figure shows that as the SEP/Rh ratio changes from about 140 to about 1000, the n/i ratio at 80% conversion changes from about 2 to 7. The major change in the percentage of the n-aldehyde product occurs in the 140 to 500 L/Rh range. It was shown in additional experiments that there was very little further selectivity increase when the SEP ligand was used as the solvent (i.e., in about 75% concentration).

The increased selectivity to linear aldehyde is a consequence of the increased catalyst stability in these experiments. The increased catalyst stability is also reflected in a decreasing darkening of the reaction mixture with increasing ligand concentration. Another sign of the increased stability is the better maintenance of the hydroformylation rate with increasing conversion. Finally, it was also noted that the increased ligand concentration resulted in a moderate suppression of the rate of hydrogenation. Nevertheless, hydrogenation remained significant enough to cause a decreasing $H_2/CO$ ratio during the reaction.

Similar studies of the effect of increased SEP/Rh ratio were carried out at 160, 145 and 120°C. The data obtained at 145° are also shown in Figure 10. The lower the reaction temperature, the less effect of increased L/Rh ratios was observed. At decreasing temperatures, most of the effects were observed in the range of increasingly low L/Rh ratios. Also, the main effect was on selectivity rather than on stability.

Example 3

Hydroformylation selectivity of tris-(trimethylsilylethyl diphenyl phosphine) rhodium carbonyl hydride excess ligand catalyst system at different olefin conversions, i.e.—at different carbon monoxide concentrations

Butene-1 was hydroformylated in the Hastalloy unit according to the general procedure. The catalyst and ligand concentrations were higher than usual and the reaction conditions milder as shown in Table 3. The reaction mixture was frequently sampled during the process and the samples were analyzed by gc to determine the relative selectivities to n- and i- aldehyde products and hydrocarbon by-products as a function of butene-1 conversion. The detailed data are given in Table 3.

The data of Table 3 indicate that the n- to i- ratio of aldehydes in the reaction is decreasing as the conversion increases. Up to about 60% butene conversion, the n/i ratio stays above 18.5, although it is steadily dropping (see Sample Nos. 1—2). In the 72—78% conversion range, the n/i ratio is about 14. Once butene-1 conversion reaches 90%, the n/i ratio of the product mixture is down to about 11.5.

TABLE 3

Hydroformylation selectivity at different olefin conversion levels

Feed: Butene-1 and 1:4 $CO/H_2$ at 130 psi at 110°C. under 130 psi
Catalyst: $L_3RH(CO)H$, Rh 212 ppm, L Excess 300 mMole, L/Rh 140
L: $ØPCH_2CH_2Si(CH_3)_3$

| Sample No. | Conversion related data | | | Aldehyde product linearity ratio, n/i | Mole % selectivity to various compounds | | | | |
|---|---|---|---|---|---|---|---|---|---|
| | Butene-1 conversion % | Conversion % based on $CO/H_2$ consumed | Reaction time, min. | | Aldehyde products | | Butane hydrogenation product | 2-Butene by-products | |
| | | | | | n | i | | cis | trans |
| 1 | 26.4 | 21.9 | 10 | 26 | 68.4 | 2.7 | 11.9 | 9.7 | 7.3 |
| 2 | 46.9 | 36.0 | 15 | 25 | 77.9 | 3.1 | 7.4 | 6.7 | 4.9 |
| 3 | 61.6 | 50.0 | 20 | 18.5 | 79.7 | 4.3 | 6.1 | 5.8 | 4.2 |
| 4 | 72.0 | 61.4 | 25 | 13.9 | 80.2 | 5.8 | 5.2 | 5.2 | 3.7 |
| 5 | 78.0 | 69.4 | 30 | 14.0 | 79.6 | 5.7 | 5.4 | 5.4 | 3.9 |
| 6 | 90.0 | 79.9 | 40 | 11.6 | 82.1 | 7.1 | 3.9 | 4.0 | 2.9 |
| 7 | 90.5 | 87.9 | 60 | 11.3 | 81.9 | 7.2 | 4.0 | 4.0 | 3.0 |

It was also observed that during the conversion of about 25% of the butene, the total aldehydes to hydrocarbon by-products ratio was lower than at higher conversions (about 70/30 versus 90/10). It is believed that this is due to uncontrolled nonequilibrium conditions early during the reaction. Almost all the hydrogenation occurred during the first 10 minutes of the reaction. During the early, very fast part of the reaction, the liquid reaction medium became starved of CO. Due to the resulting low CO partial pressure, the n/i product ratio became very high. However, the amount of CO during some of this period was so insufficient that much hydrogenation and isomerization occurred. In a continuous process, where the low optimum concentration of CO could be more accurately maintained, high selectivity to aldehydes could be better achieved without producing significant amounts of by-products.

Example 4

Hydroformylation with the tris-(trimethylsilylethyl diphenyl phosphine) rhodium complex system

In a series of experiments, tris-(triphenyl phosphine) rhodium carbonyl hydride was reacted with a varying excess concentration of the novel substituted diaryl alkyl phosphines. This resulted in the formation of the novel catalysts of the present invention which were studied for their catalytic properties in the usual manner in the Hastalloy unit (H).

Tris-(triphenyl phosphine) rhodium carbonyl hydride, 0.1 g (0.1 mmole), was mixed with 80 g of a mixture of 4 g (14 mole) of trimethylsilylethyl diphenyl phosphine and 76 g 2-propylheptyl valerate to provide an SEP catalyst system. For comparison, the same complex was also mixed with 80 g of a mixture of 3.7 g (14 mole) of triphenyl phosphine to provide a TPP catalyst system. This provided two systems having 105 ppm rhodium and a 140 fold ligand excess.

Butene hydroformylations were then carried out with both catalyst systems at 100°C. in the usual manner. The results indicated that the main catalytic species of the SEP system is a SEP complex. The reaction rate of the SEP system was about 1/6 of the TPP system (k $min^{-1}$ values of 0.02 and 0.12, respectively). The n/i product ratios were about the same (4.2).

Other SEP catalyst systems were made up the same way except for the different L/Rh ratios: 25 and 5. They were also employed successfully for butene hydroformylation.

Example 5

Hydroformylation with the tris-(trimethylsilylethyl diphenyl phosphine system at different $H_2/CO$ ratios

For a further study of the effect of the $H_2/CO$ ratios on hydroformylation selectivity, the feed gas was provided through the side arm of the autoclave to provide conditions during the reaction which are closer to equilibrium. This type of operation was specific to this example.

The SEP complex catalyst was formed in situ during hydroformylation from acetylacetonato dicarbonyl rhodium. The $H_2/CO$ ratios of both the initial $H_2/CO$ gas and the final unreacted synthesis gas, in the head space of the autoclave, were analyzed. The $H_2/CO$ ratio of the feed gas was adjusted to keep the initial and final $H_2/CO$ ratios the same as much as possible.

The results are shown by Table 4. The data show that as the $H_2/CO$ ratio was increased from 1 to 20 the ratio of n- to i- aldehydes was increased. It is also interesting to note that having the side arm feed resulted in much less 1-butene isomerization and hydrogenation than obtained previously with top feeding.

TABLE 4

1-Butene hydroformylation with synthesis gas of varying $H_2/CO$ ratio in the presence of SEP complex and TPP complex catalysts

Total pressure 350 psi (26 atm.); catalyst; $L_3Rh(CO)H$; L/RH=140, Rh=110 ppm
Solvent: 2-Ethylhexyl acetate

| Seq. No. | Ligand | Reaction temp. °C | $H_2/CO$ Ratio | | | CO Partial pressure pCO, psi | | Fraction of $H_2/CO$ reacted | | Reaction time, min. | Aldehyde product linearity | | Selectivities to various compounds, % aldehydes | | | |
|---|---|---|---|---|---|---|---|---|---|---|---|---|---|---|---|---|
| | | | Initial | Feed | Final | Initial | Final | Rate constant k, $min^{-1}$ | Conversion % | | Ratio n/i | 100n, % n+i | n | i | Butane | 2-Butene |
| 1 | SEP | 120 | 1.18 | 1.08 | 1.36 | 160 | 149 | 0.115 | 81 | 13 | 3.09 | 7.56 | 73.7 | 23.8 | 0.6 | 1.8 |
| 2 | SEP | 120 | 5.0 | 1.08 | 4.8 | 59 | 60 | 0.082 | 81 | 22 | 4.56 | 8.20 | 78.2 | 17.1 | 1.7 | 2.9 |
| 3 | SEP | 120 | 10.0 | 1.08 | 7.8 | 31 | 39 | 0.090 | 82 | 30 | 6.60 | 86.8 | 80.8 | 12.2 | 2.8 | 3.2 |
| 4 | SEP | 120 | 15.0 | 1.17 | 10.4 | 22 | 30 | 0.116 | 81 | 15 | 8.22 | 89.2 | 80.4 | 9.8 | 4.4 | 5.3 |
| 5 | TPP | 90 | 1.08 | 1.08 | 1.8 | 168 | 125 | 0.060 | 81 | 29 | 3.76 | 79.0 | 77.0 | 20.5 | 0.8 | 7.7 |
| 6 | TPP | 90 | 5.0 | 1.08 | 9.0 | 59 | 35 | 0.059 | 81 | 28 | 5.50 | 84.6 | 80.5 | 14.7 | 1.2 | 3.6 |
| 7 | TPP | 90 | 10.0 | 1.08 | 10.5 | 31 | 30 | 0.062 | 80 | 26 | 6.70 | 87.0 | 81.0 | 12.1 | 2.0 | 4.8 |
| 8 | TPP | 90 | 15 | 1.17 | 18 | 22 | 18 | 0.062 | 80 | 26 | 8.70 | 89.7 | 80.2 | 9.2 | 4.6 | 6.0 |

Comparative side arm feed experiments were also carried out using the known TPP catalyst system at the same concentration. At 120°C, significant side reaction continued to occur. Apparently, equilibrium conditions were not sufficiently approached. Consequently, further experiments were carried out at 90°C where the reaction rate is sufficiently slow to avoid side reactions. The results are also shown by Table 4. They show that TPP at 90°C exhibits a similar behavior to that of SEP at 120°C. The n/i ratios are slightly higher to for TPP, apparently due to a higher average of $H_2/CO$ ratios.

Example 6
Hydroformylation with the tris-(trimethylsilylethyl diphenyl phosphine rhodium complex system at different CO partial pressures

The results of the type of experiments described in Example 5 were plotted in Figure 10 to show the dependence of n/i aldehyde product ratios on the CO partial pressures. In additional experiments the $H_2/CO$ ratios were kept constant with changing CO partial pressures by maintaining an appropriate fraction of the total 350 psi (26 Atm.) gas pressure by $N_2$gas. There was relatively little change in reaction rates.

Figure 10 shows that decreasing CO partial pressures result in higher n/i product ratios even though the $H_2/CO$ ratio is kept constant. The dependence of the n/i ratios is particularly strong in the low CO partial pressure range.

Example 7
Comparative hydroformylation with tris-(trihydrocarbylsilylalkyl diphenyl phosphine) carbonyl hydride based catalyst systems

In a series of experiments, the results of which are shown in Table 5, various silyl substituted alkyl diphenyl phosphine complexes were tested as 1-butene hydroformylation catalysts under standard test conditions, using top synthesis gas feed. The data indicate, that with the exception of the last catalyst, the complexes tested show the same type of catalyst behavior as the previously discussed SEP complex. The last complex tested, i.e., the one based on the trimethylsilylmethyl ligand, was unstable. It showed less selectivity than the others even at the relatively low hydroformylation temperature used in this case.

TABLE 5

I-Butene hydroformylation in the presence of various tris(silyl substituted alkyl diphenyl phosphine) rhodium carbonyl hydride complex catalysts

Catalyst: $L_3Rh(CO)H$, Rh = 107 ppm, Rh/L = 140
Pressure: 350 psi (26 Atm)
$L=Ph_2PR$; $R_1=CH_2CH_2SiC_3H_7$; $R_2=CH_2CH_2SiPh_3$; $R_3=[Ph_2PCH_2CH_2]_2Si(CH_3)_2$;
$R_4=CH_2CH_2CH_2Si(CH_3)_3$; $R_5=CH_2Si(CH_3)_3$

| Seq. No.* | Ligand LR | Complex | Reaction temp. °C | $H_2CO$ Ratios Initial | Feed | Final | Rate constant k, $min^{-1}$ | Conversion % | Reaction time min. | Ratio n/i | $\frac{100n}{n+1}$ % | Selectivity to aldehyde products, % n | i | Butane | 2-Butene |
|---|---|---|---|---|---|---|---|---|---|---|---|---|---|---|---|
| 1a | $LR_1$ | A' | 120 | 5 | 1.08 | 3.6 | 0.072 | 80 | 30 | 8.90 | 89.9 | 63.4 | 7.1 | 20.2 | 9.3 |
| 1b | $LR_1$ | A' | 145 | 5 | 1.27 | 3.8 | 0.274 | 80 | 8 | 9.62 | 90.6 | 61.1 | 6.4 | 18.4 | 14.3 |
| 2 | $LR_2$ | B' | 145 | 5 | 1.27 | 7.1 | 0.132 | 80 | 30 | 7.59 | 88.4 | 73.6 | 9.7 | 8.3 | 8.3 |
| 3 | $LR_3$ | C' | 145 | ~4 | ~1 | — | 0.157 | 80 | 12 | 7.6 | 88.4 | — | — | — | — |
| a | $LR_4$ | D' | 120 | 5 | 1.03 | 3.7 | 0.069 | 81 | 34 | 8.34 | 89.3 | 69.9 | 8.4 | 13.2 | 8.5 |
| 4b | $LR_4$ | D' | 145 | 4 | ~1 | 2.3 | 0.260 | 82 | 9 | 5.80 | 85.3 | 67.6 | 11.7 | 10.9 | 9.8 |
| 5 | $LR_5$ | E' | 100 | 5 | 1.05 | 5 | 0.056 | 78 | 60 | 6.24 | 86.2 | 54.3 | 8.1 | 31.8 | 5.9 |

* Experiments of Seq. No. 1a and 1b were carried out in 2-ethylhexyl acetate as a solvent. The rest were in 2-propylheptyl valerate.

' A $(Ph_2PCH_2CH_2SiPh_3)Rh(CO)H$
B $[(Ph_2PCH_2CH_2)_2Si(CH_3)_2]_3Rh(CO)H$
C $[Ph_2PCH_2CH_2CH_2Si(CH_3)_3]_3Rh(CO)H$
D $[PH_2PCH_2Si(CH_3)_3]_3Rh(CO)H$
E $(PhPCH_2CH_2CH_2CH_3)_3Rh(CO)H$

Example 8

Hydroformylation with various tris(alkyl diphenyl phosphine) rhodium carbonyl hydride catalysts

In a series of standard experiments, the results of which are shown in Table 6, various tris-(alkyl diphenyl phosphine) rhodium complexes were tested as 1-butene hydroformylation catalysts. It is emphasized that a 4 to 5 $H_2$/CO ratio and a 140 L/Rh ratio was used in these tests, in contrast to published work with similar systems.

Overall, all of the n-alkyl diphenyl phosphine complexes exhibited similar catalytic behavior (Seq. Nos. 1—11). At sufficiently elevated temperatures, where they were active and stable, highly linear aldehyde products were selectively produced at a high rate. This is in contrast to the published low temperature results by Sanger and others which were referred to earlier.

It is noted that the ligand volatility in the ethyl-, propyl- and butyl- diphenyl phosphine complex systems was found to be too high for their application in a continuous product flashoff process. In contrast, the ligand of the novel tris-(hexyl diphenyl phosphine) rhodium complex showed no objectionable volatility (see Example 17). Other $C_6$ or higher alkyl substituents of appropriate structure can also provide the desired reduced volatility.

TABLE 6

1-Butene hydroformylation in the presence of various tris (alkyl diphenyl phosphine) rhodium carbonyl hydride catalysts

Catalyst: $L_3Rh(CO)H$, L/Rh=140 ppm; Percursor dicarbonyl acetylacetonate rhodium,
Total pressure 350 psi (~26 Atm)
L: $Ph_2PR$; $R_1=CH_2CH_3$; $R_2=(CH_2)_2CH_3$; $R_3=(CH_2)_3CH_3$; $R_4=CH(CH_3)C_2H_5$;
$R_5=CH_2CH_2C(CH_3)_3$; $R_6=CH_2C(CH_3)_3$

| Seq.* No. | Ligand LR' | Reaction temp. °C | $H_2$/CO Ratio | | | Fraction of $H_2$/CO reacted | | Reaction time min. | Aldehyde product linearity | | Selectivity to various compounds | | | |
|---|---|---|---|---|---|---|---|---|---|---|---|---|---|---|
| | | | Initial | Feed | Final | Rate constant k, $min^{-1}$ | Conversion % | | Ratio n/i | 100n, % n+i | Aldehyde products n | i | Butane | 2-Butenes |
| 1 | $LR_1$ | 120 | 4.9 | 1.041 | 4.6 | 0.045 | 80 | 41 | 8.39 | 89.4 | 75.8 | 9.0 | 9.7 | 5.5 |
| 2 | | 145 | 4.9 | 1.041 | 2.8 | 0.113 | 80 | 19 | 6.38 | 86.5 | 66.0 | 10.4 | 12.3 | 11.3 |
| 3 | $LR_2$ | 120 | 4.9 | 1.041 | 3.6 | 0.251 | 81 | 10 | 11.57 | 92.1 | 68.4 | 5.9 | 16.9 | 8.8 |
| 4 | | 145 | 4.9 | 1.041 | 2.6 | 0.326 | 81 | 6.5 | 5.92 | 85.6 | 63.9 | 10.8 | 12.9 | 12.4 |
| 5 | | 170 | 4.9 | 1.041 | 2.0 | 0.210 | 80 | 55 | 1.92 | 65.8 | 70.0 | 26.0 | 15.6 | 8.5 |
| 6 | $LR_3$ | 145 | 4.9 | 1.174 | 3.8 | 0.337 | 80 | 6.0 | 7.15 | 87.7 | 62.4 | 8.7 | 15.5 | 13.4 |
| 7 | $LR_4$ | 120 | | | | 0.244 | 89 | 15 | 3.14 | 75.86 | | | | |
| 8 | $LR_5$ | 120 | 4 | | 2.7 | 0.114 | 80 | 15 | 7.57 | 88.3 | 74.3 | 9.8 | 8.6 | 7.4 |
| 9 | | 145 | 4 | | | 0.285 | 82 | 8 | 6.21 | 86.1 | 70.3 | 11.3 | 8.8 | 9.5 |
| 10 | $LR_6$ | 120 | 4.9 | 1.083 | 3.0 | 0.224 | 81 | 14 | 4.82 | 82.8 | 59.2 | 12.3 | 16.7 | 11.8 |
| 11 | | 145 | 4.9 | 1.083 | 2.8 | 0.361 | 80 | 6.5 | 3.15 | 75.9 | 53.8 | 17.1 | 7.4 | 21.7 |

* The generally used solvent was 2-propylheptyl valerate. In Seq. No. 6 2-ethylhexyl acetate was used.

In the second group of test results shown in Table 6, the effect of alkyl substituents of different branching was investigated (Seq. Nos. 7—11). Compared to the n-butyl derivative, the secondary butyl derivative was found to be a much less selective catalyst for linear aldehyde production (Seq. No. 7). This is an apparent result of the steric inhibition of the desired tris-phosphine complex formation. It is also noted that the t-butyl diphenyl phosphine system showed no catalytic activity whatsoever under these condition. It is recalled that in that case no tris-phosphine was formed at all.

The last pair of ligands tested shows that minor structural differences can result in major differences in the selectivity of the catalyst system. The use of 3,3-dimethylbutyl diphenyl phosphine ligand, the carbon analog of SEP, resulted in the desired high n/i ratio of aldehydes (Seq. Nos. 8 and 9). This ligand does form tris-phosphine complex. In contrast, employing a neopentyl group having one less methylene group between the phosphorus and the sterically demanding t-butyl group led to much poorer catalyst selectivity (Seq. Nos. 10 and 11). This is again a consequence of the steric inhibition of tris-phosphine formation.

Example 9

Hydroformylation with chelating and non-chelating alkylene bis-(diphenyl phosphine) rhodium complex catalysts

In a series of experiments, the results of which are summarised in Table 7, alkylene bis-(diphenyl phosphines) were tested as ligands for rhodium complex catalyzed hydroformylation under standard conditions. The ligand to rhodium ratio was either 1.5 or about 70. Since these are bis-phosphine ligands, the above values correspond to a P/Rh ratio of 3 and 140, respectively.

The bis-phosphine ligands tested had an increasing number (n) of methylene, i.e., $CH_2$ groups, separating the two phosphorus atoms. This increase led to unexpected changes in catalysis.

In the case of the sterically crowded monomethylene (n=1) bis-phosphine, the catalyst system showed little activity and n/i selectivity at the L/Rh ratio of 1.5 and no activity at L/Rh ratio of 71 (Seq. Nos. 1 and 2). The chelate forming dimethylene (n=2) bis-phosphine showed a similar behavior (Seq. Nos. 3 and 4).

The trimethylene (n=3) bis-phosphine ligand, which forms a different chelate, is more active that the previous ligands. It has significant activity when the L/Rh ratio is 1.5 (Seq. No. 5). However, as compared to the previous ligands, it has less activity and less selectivity when the L/Rh ratio is 73 (Seq. No. 6).

In contrast to the chelating bis-phosphines, the non-chelating polymethylene (n=4, 6, 14) bis-phosphines showed higher n/i product ratios at the higher L/Rh ratio (seq. Nos. 7 to 13). The catalytic behavior of these bis-phosphine ligands, which form tris-phosphine complexes at high L/Rh ratios, is shown in detail in the case of the hexamethylene (n=6) compound. As can be seen at a 1.5 L/Rh ratio the hexamethylene bridged bis-phosphine ligand leads to an n/i-aldehyde product ratio of 4.1. At a L/Rh ratio of 70, the n/i ratio is increased to 9.7 under otherwise identical conditions.

TABLE 7
1-Butene hydroformylation in the presence of chelating and non-chelating alkylene bis-(diphenyl phosphine) rhodium complex catalysts
Rh=140 ppm; Catalyst precursor:
Dicarbonyl acetyl acetonato rhodium, total pressure 350 psi (~26 Atm)

| Seq* No. | $Ph_2P(CH_2)_nPPh_2$ Ligand: Ligand n | Complex' | Ratio L/Rh | Reaction temp. °C | $H_2CO$ ratio: Initial | Feed | Final | Fraction of $H_2/CO$ reacted: Rate constant k, $min^{-1}$ | Conversion, % | Reaction time min. | Aldehyde product linearity: Ratio n/i | 100n, n+i % | Selectivity to various compounds, % aldehydes: n | i | Butane | 2-Butene |
|---|---|---|---|---|---|---|---|---|---|---|---|---|---|---|---|---|
| 1 | 1 | F | 1.5 | 120 | 5 | 1.08 | 5.8 | 0.025 | 78 | 135 | 2.1 | 67.7 | 47.1 | 22.4 | 2.9 | 27.6 |
| 2 | 1 | | 71 | 120 | 4 | | | Nil | Nil | | | | | | | |
| 3 | 2 | G | 1.5 | 120 | 5 | 1.08 | 4.1 | 0.067 | 80 | 45 | 1.5 | 60.6 | 42.9 | 22.8 | 7.6 | 21.8 |
| 4 | 2 | | 69 | 145 | 4 | | | Nil | Nil | | | | | | | |
| 5 | 3 | H | 1.5 | 120 | 5 | 1.08 | 3.6 | 0.120 | 80 | 30 | 2.0 | 66.2 | 45.6 | 23.3 | 9.5 | 21.6 |
| 6 | 3 | | 73 | 120 | 5 | 1.08 | 5.9 | 0.030 | 80 | 65 | 1.3 | 56.5 | 52.1 | 40.2 | 4.5 | 3.2 |
| 7 | 4 | I | 69 | 145 | 4 | | | 0.190 | 81 | 11 | 3.3 | 76.5 | | | | |
| 8 | 6 | J | 1.5 | 120 | 5 | 1.08 | 3.5 | 0.139 | 81 | 15 | 4.1 | 80.4 | 63.3 | 15.5 | 9.7 | 11.5 |
| 9 | 6 | | 70 | 120 | 5 | 1.08 | 3.7 | 0.108 | 80 | 17 | 9.7 | 90.6 | 81.7 | 8.4 | 5.8 | 4.1 |
| 10 | 6 | | 70 | 145 | 4 | 1.17 | 3.3 | 0.332 | 81 | 6 | 8.0 | 88.9 | 64.4 | 8.1 | 13.9 | 13.6 |
| 11 | 6 | | 70 | 170 | 4 | 1.17 | 2.7 | 0.284 | 79 | 20 | 3.1 | 75.6 | 52.2 | 16.8 | 14.4 | 16.6 |
| 13 | 14 | K | 144 | 100 | 4 | | | 0.060 | 54 | | 3.8 | 79.2 | | | | |

* Experiments of Seq. No. 53-8 and 4 were carried out in 2-propylheptyl valerate, the rest in 2-ethylhexyl acetate.

' F $Ph_2PCH_2PPh_2$

G 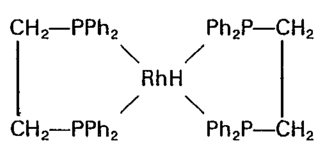

H 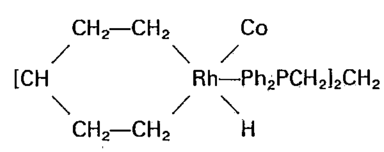

I $[Ph_2P(CH_2)_4PPh_2]_3Rh(CO)H$

' J $[Ph_2P(CH_2)_6PPh_2]_3Rh(CO)H$

K $[Ph_2P(CH_2)_{14}PPH_2]Rh(CO)H$.

Example 10

Hydroformylation with aryl, amide and amine substituted alkyl diphenyl rhodium complex catalysts

In a series of standard 1-butene hydroformylation experiments, three substituted alkyl diphenyl phosphines representing aryl, amide and amine functionalities were studied as tris-phosphine rhodium complex forming ligands. The results are shown in Table 8. As shown therein, all three complexes were highly selective catalysts for linear aldehydes when stable. As the reaction temperature was increased beyond the stable range of the desired catalyst complexes, the selectivities, i.e. n/i aldehyde ratios, decreased.

As described, the tests show that the novel 2-phenylethyl-, 2-pyrrolidinonylethyl- and 2-diethylaminoethyl-diphenyl phosphine rhodium complexes are selective catalysts in the hydroformylation of the present invention.

Example 10a

Hydroformylation with sulfone, phosphine oxide, keto, carboxylate, hydroxy and ether substituted alkyl diphenyl rhodium complex catalysts

The series of standard butene-1 hydroformylation tests summarised in Table 8a, describe the catalytic behaviour of further, variously substituted alkyl diphenyl phosphine rhodium complexes. These complexes are all of the tris-phosphine type. The data of Table 8a show that they are all selective catalysts for forming highly linear aldehydes.

With regard to the sulfone substituted ligand, 2-ethylsulfonylethyl diphenyl phosphine, it is noted that it gave a particularly selective catalyst complex. The activity of this complex rapidly increased in the 100 to 145°C range. This behaviour correlates with the formation of a highly stable complex having minimum ligand exchange at 35°. The attractive catalytic properties of such a complex apparently depend on the specific manner of thermal activation plus stabilisation in the present process.

Hydroformylation of various olefinic compounds
(Examples 11—13)

In the following, the hydroformylation of various olefinic compounds is described, mostly under standard conditions. As catalyst, tris-(trimethylsilylethyl diphenyl phosphine) rhodium carbonyl hydride was used throughout these experiments. At first, the hydroformylation of propylene will be described. Then a series of experiments on a variety of olefins including a non-hydrocarbon derivative will be discussed. Finally, it will be shown with isomeric pentenes, how the present process could be employed when starting with a mixture of olefins.

Example 11

Hydroformylation of propylene

The complex used in Examples 1 was studied at the 458 ppm rhodium level, in the presence of a one hundred fold excess of trimethylsilylethyl diphenyl phosphine ligand, as a propylene hydroformylation catalyst. The reaction temperature was 100°C., the 1:4 $CO/H_2$ pressure was 400 psi. The general procedure previously employed for butene hydroformylation was used to carry out the reaction.

TABLE 8

Butene hydroformylation in the presence of aryl, amide and amine substituted alkyl diphenyl phosphine rhodium complex catalysts

Catalyst: $L_3RH(CO)H$; L/Rh=140 Rh=110 ppm; Precursor: Dicarbonyl acetylacetonato rhodium; Total pressure, 350 psi (26 Atm); solvent 2-propylheptyl valerate

$L_1=Ph_2PCH_2CH_2Ph$; $L_2=Ph_2PCH_2CH_2$ 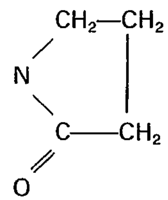 $L_3=Ph_2PCH_2CH_2N(C_2H_5)_2$

| Seq. No. | Ligand L | Example No. of complex | Reaction temp. °C | $H_2$/CO Ratio | | | Rate constant k, $min^{-1}$ | Conversion, % | Reaction time min. | Aldehyde product linearity | | Selectivity to various compounds, % aldehydes | | | |
|---|---|---|---|---|---|---|---|---|---|---|---|---|---|---|---|
| | | | | Initial | Feed | Final | | | | Ratio n/i | 100n, n+i % | n | i | Butane | 2-Butenes |
| 1 | $L_1$ | 68 | 120 | 4.9 | 1.04 | 2.7 | 0.087 | 81 | 24 | 11.3 | 91.9 | 73.8 | 6.5 | 12.1 | 7.6 |
| 2 | | | 145 | 4.9 | 1.04 | 1.9 | 0.243 | 78 | 12 | 6.4 | 86.5 | 62.2 | 9.7 | 14.3 | 13.9 |
| 3 | | | 170 | 4.9 | 1.04 | 1.8 | 0.274 | 80 | 55 | 1.9 | 65.0 | 48.9 | 26.3 | 15.4 | 9.4 |
| 4 | $L_2$ | 69 | 100 | 4.9 | 1.04 | 10.2 | 0.011 | 80 | 205 | 12.9 | 92.8 | 81.1 | 6.3 | 6.4 | 6.2 |
| 5 | | | 110 | 4.9 | 1.04 | 4.2 | 0.045 | 81 | 41 | 12.6 | 92.6 | 77.0 | 6.1 | 9.5 | 7.4 |
| 6 | | | 120 | 4.9 | 1.04 | 3.7 | 0.094 | 81 | 21 | 12.5 | 92.5 | 74.6 | 6.0 | 11.0 | 8.4 |
| 7 | | | 130 | 4.0 | 1.04 | 3.1 | 0.109 | 80 | 19 | 10.4 | 91.2 | 70.1 | 6.8 | 12.4 | 10.7 |
| 8 | | | 140 | 4.9 | 1.04 | 3.0 | 0.125 | 80 | 20 | 7.6 | 88.4 | 65.3 | 8.6 | 13.6 | 12.5 |
| 9 | | | 145 | 4.9 | 1.04 | 2.5 | 0.185 | 80 | 18 | 6.2 | 86.1 | 63.0 | 10.1 | 12.8 | 14.1 |
| 10 | | | 155 | 4.9 | 1.04 | 2.4 | 0.201 | 80 | 40 | 3.5 | 77.8 | 58.2 | 16.6 | 13.2 | 12.0 |
| 11 | | | 170 | 4.9 | 1.04 | 2.0 | 0.156 | 77 | 75 | 2.0 | 66.7 | 49.1 | 24.5 | 16.4 | 6.2 |
| 12* | | 25 | 120 | 5.0 | 1.08 | 3.4 | 0.122 | 81 | 15 | 7.3 | 88.0 | 74.3 | 10.2 | 9.8 | 5.7 |

* Solvent 2-ethylhexyl acetate

TABLE 8a
1-Butene hydroformylation in the presence of sulfone, phosphine oxide, keto, ester, hydroxy and ether substituted alkyl diphenyl phosphine catalysts

Catalyst: $L_3Rh(CO)h$; L/Rh = 140; Rh = 110 ppm, Precursor: Dicarbonyl acetylacetonato rhodium; Total pressure 350 psi (~26 Atm)

Li = $Ph_2PR$; $R_1 = CH_2CH_2SO_2C_2H_5$; $R_2 = CH_2CH_2P(=O)Ph_2$; $R_3 = CH_2CH_2COCH_2$; $R_4 = CH_2CH_2CO_2CH_3$; $R_5 = CH_2CH_2CH_2OH$; $R_6 = CH_2CH_2CH_2$

| Seq.* No. | Ligand LR | Example No. of complex | Reaction temp. C | $H_2$/CO Ratio | | | Rate constant k, $min^{-1}$ | Conversion, % | Reaction time min. | Aldehyde product linearity | | Selectivities to various compounds, % aldehydes | | | |
|---|---|---|---|---|---|---|---|---|---|---|---|---|---|---|---|
| | | | | Initial | Feed | Final | | | | Ratio n/i | 100n, n+i % | n | i | Butane | 2-Butenes |
| 1 | $LR_1$ | 71 | 100 | 5.0 | 1.08 | 4.6 | 0.016 | 80 | 115 | 12.5 | 92.6 | 85.2 | 6.8 | 2.5 | 5.5 |
| 2 | | | 120 | 5.0 | 1.08 | 4.2 | 0.048 | 80 | 37 | 14.6 | 93.6 | 80.6 | 5.5 | 6.0 | 7.8 |
| 3 | | | 145 | 5.0 | 1.27 | 6.9 | 0.160 | 77 | 10 | 11.8 | 92.2 | 77.0 | 6.5 | 6.2 | 10.2 |
| 4 | | | 170 | 5.0 | 1.27 | 3.0 | 0.119 | 80 | 50 | 2.4 | 70.1 | 47.0 | 20.0 | 18.2 | 14.8 |
| 5 | $LR_2$ | 72 | 145 | 4.0 | 1.04 | | 0.107 | 80 | 17 | 7.3 | 88.0 | — | — | — | — |
| 6 | $LR_3$ | 73 | 120 | 4.9 | 1.08 | 4.2 | 0.060 | 81 | 34 | 12.6 | 92.6 | 72.8 | 5.8 | 13.1 | 8.3 |
| 7 | | | 145 | 4.9 | 1.08 | 2.5 | 0.267 | 81 | 7.5 | 7.2 | 87.8 | 74.5 | 10.4 | 6.8 | 8.4 |
| 8 | | | 170 | 4.9 | 1.08 | 2.2 | 0.330 | 79 | 6.0 | 4.0 | 80.0 | 60.8 | 15.2 | 7.4 | 16.6 |
| 9 | $LR_4$ | 74 | 120 | 4.9 | 1.04 | 4.3 | 0.062 | 80 | 30 | 11.8 | 92.2 | 78.0 | 6.6 | 8.5 | 6.9 |
| 10 | | | 145 | 4.9 | 1.04 | 3.0 | 0.105 | 80 | 24 | 6.8 | 87.2 | 67.0 | 9.9 | 12.2 | 11.0 |
| 11 | $LR_5$ | 75 | 120 | 5.0 | 1.08 | 4.5 | 0.040 | 80 | 28 | 6.1 | 85.8 | 74.2 | 12.3 | 5.8 | 5.7 |
| 12 | $(LR_6)_2O$ | 76 | 120 | 5 0 | 1.08 | 3.7 | 0.085 | 80 | 20 | 9.2 | 90.2 | 73.3 | 8.0 | 11.4 | 7.3 |

* Experiment of Seq. No. 1—4, 11 and 12 were carried out in 2-ethylhexyl acetate, the rest in 2-propylheptyl valerate.

The reaction rate was found to be k=0.04 $min^{-1}$, expressed as the fraction reacted. In 60 minutes, 82% conversion was reached based on the $CO/H_2$ consumed. The ratio of n-butyraldehyde to methyl-propanol products was 5.0. The selectivity to these aldehydes was 87.5%. The selectivity to the by-product propane was only 2.5%.

Example 12
Hydroformylation of miscellaneous olefinic compounds

In a series of experiments, the results of which are summarized in Table 9, a number of olefins were hydroformylated using the tris-SEP complex based catalyst system (Seq. Nos. 1—7).

Using a high L/Rh ratio, 1-pentene was selectively hydroformylated at 170°C (Seq. No. 1). A lower L/Rh ratio was successfully used at 145°C for the selective hydroformylation of 1-octene (Seq. No. 2).

A comparison of the n/i selectivities indicated that, in the absence of isomerization, 1-n-olefins of increasing carbon number react with increasing selectivity. Branching of terminal olefins further increased n/i selectivity. This is shown by the example of 3-methylbutene (Seq. No. 4). Internal olefins could also be hydroformylated as shown in the case of cis butene-2-hydroformylation (Seq. No. 5). It is important to note that isomerization to 1-butene also occurred as indicated by the formation of n-valeraldehyde.

A terminal olefin having a substituent on a vinylic carbon, such as 2-ethylhexene, showed an essentially specific terminal reaction to produce only the linear aldehyde derivative (Seq. No. 6).

TABLE 9

Hydroformylation of various olefinic compounds in the presence of SEP rhodium complex based catalyst systems

Catalyst: $L_3Rh(CO)H$; L=SEP=$Ph_2PCH_2CH_2Si(CH_3)_3$;
Precursor: Dicarbonyl acetylacetonato rhodium;
Total pressure: 350 psi (26 Atm)
Solvent: 2-ethylhexyl acetate

| Seq. No. | Olefinic reactant | Rh Conc., ppm | L/Rh | Reaction temp °C | $H_2$/CO Ratio |  |  | Fraction of $H_2$/CO reacted |  | Reaction time min. | Aldehyde product linearity |  | Selectivities to various compounds aldehydes |  |  |  |
|---|---|---|---|---|---|---|---|---|---|---|---|---|---|---|---|---|
|  |  |  |  |  | Initial | Feed | Final | Constant k, $min^{-1}$ | Conversion % |  | Ratio n/i | 100n, %n+i | n | i | Butane | 2-Butenes |
| 1 | 1-Pentene | 105 | 510 | 170 | 5.0 | 117 | 3.0 | 0.193 | 80 | 12 | 7.5 | 88.2 | 53.3 | 7.1 | 19.3 | 20.2 |
| 2 | 1-Octene | 113 | 98 | 145 | 4.0 | 1.04 |  | 0.257 | 80 | 8 | 6.8 | 87.2 |  |  |  |  |
| 3 |  |  | 141 | 165 | 4.0 | 1.04 |  | 0.521 | 80 | 4 | 5.9 | 85.5 |  |  |  |  |
| 4 | 3-Methylbutene | 110 | 140 | 145 | 5.0 | 1.27 | 6.6 | 0.274 | 81 | 10 | 23.9 | 96.0 | 70.8 | 3.0 | 25.2 | 0 |
| 5* | cis-2-Butene | 447 | 256 | 170 | 5.0 | 1.27 | 3.6 | 0.018 | 80 | 150 | 1.0 | 49.5 | 38.7 | 41.4 | 6.1 |  |
| 6 | 2-Ethylhexene | 553 | 28 | 120 | 1.08 | 1.08 | 1.45 | 0.007 | 40 | 135 | ∞ | 100 | 100 | Nil | Nil | Nil |
| 7** | Diallyl Ether | 112 | 140 | 120 | 5.0 | 1.08 | 31 | 0.434 | 80 | 5.5 | 3.6 | 78.3 |  |  |  |  |

* There was a 2.6% selectivity to amyl alcohols. Both mono and bis-hydroformylated products were formed.

Finally, an oxygenated diolefinic compound, diallyl ether, was also successfully hydroformylated without any apparent, major hydrogenation side reaction (Seq. No. 7). Both the mono- and bis-hydroformylated products could be selectively produced. At low conversions, the primary unsaturated aldehyde products predominated. At high conversions, a high yield of the dialdehyde products was obtained.

Example 13
Hydroformylation of an isomeric mixture of pentenes

The results of two exemplary hydroformylation experiments using a mixed pentenes feed are presented in Table 9 to show that all or certain components of olefin mixtures can be reacted.

In the experiments shown therein the tris-SEP rhodium complex was employed in the usual manner. However, no added solvent was employed.

The data show that the 1-n-olefin component (1-pentene) was the most reactive among the significant olefin components in both runs (Nos. 1 and 2). The minor branched olefin (3-methyl butene-1) was also highly reactive. High conversions of the internal olefin components (cis- and trans- pentene-2's) and the olefinically substituted terminal olefin (2-methylbutene) could also be realized under the more forcing conditions of run No. 2. It is noted that under the latter conditions some darkening of the reaction mixture occurred indicating some long term instability.

Example 14
Continuous hydroformylation

The tris-(trimethylsilylethyl diphenyl phosphine) rhodium, carbonyl hydride catalyst system was extensively studied in a continuous hydroformylation unit. The feed was butene-1 and the products were continuously removed together with the unreacted volatile components of the reaction mixture. The typical reaction temperature for the SEP based system was 120°C. Comparative runs were also carried out with a similar TPP based system at 100°C. Both systems could be successfully operated on the short run, although it appeared that the known degradation reactions and the stripping of the valeraldehyde trimer by-product at 100°C could become a problem with TPP. The SEP system showed excellent long term stability and activity maintenance.

TABLE 10
Hydroformylation of mixed pentenes with tris-(trimethylsilylethyl diphenyl phosphine) rhodium complex catalyst system

Catalyst: $L_3Rh(CO)H$, L=SEP, L/Rh, 139;
Precursor: Dicarbonyl acetylacetonato rhodium;
Olefin: 100 g mixed pentenes without added solvent

| | Reaction conditions | | | | |
|---|---|---|---|---|---|
| Number | O: Feed | 1 | | 2 | |
| Temperature, °C | | 120 | | 120—145 | |
| Time, Min. | | 300 | | 360 | |
| Olefin Conversion, % | | 30 | | 55 | |
| Rh Conc. ppm | | 109 | | 293 | |
| | Composition of reaction mixture | | | | |
| C-Hydrocarbons | Mole % | Mole % | Conv. % | Mole % | Conv. % |
| 3-Methylbutene | 0.31 | 0 | 100 | 0 | 100 |
| i-Pentane | 0.45 | 0.53 | — | 1.02 | — |
| 1-Pentane | 8.04 | 0.89 | 89 | 0.71 | 91 |
| 2-Methylbutene | 24.61 | 19.13 | 22 | 7.14 | 71 |
| n-Pentane | 4.14 | 5.10 | — | 6.10 | — |
| t-2-Pentene | 28.97 | 19.95 | 31 | 5.63 | 81 |
| c-2-Pentene | 12.32 | 7.35 | 40 | 2.19 | 82 |
| 2-Methylbutene-2 | 21.04 | 22.22 | 0 | 22.46 | 0 |
| Aldehydes | None | Mole % | | Mole % | |
| 2-methylpentanal | — | 13.86 | | 27.33 | |
| 3-methylpentanal | — | 5.69 | | 16.97 | |
| 4-methylpentanal | — | — | | — | |
| n-hexanal | — | 5.27 | | 10.46 | |

A representative 30 day continuous operation of the SEP catalyst system is illustrated in Figure 12. With regard to the continuous operating conditions, it is noted that the total synthesis gas pressure was lower (125 psi, ~8.5 Atm) and the $H_2/CO$ ratio higher (10/1) than in most of the batch studies. Also a higher concentration of rhodium (270 ppm) and a higher L/Rh ratio (210) were employed. Under these conditions a batch experiment produced results similar to those found in the continuous operation.

In the continuous hydroformylation the catalyst was generated from dicarbonyl acetylacetonato rhodium in situ. The precursor was a more active but much less selective hydroformylation catalyst than the desired final catalyst.

In a typical operation 1-butene was introduced into the reactor at a rate of 4.4 mole per hour. The rate of CO was typically 2 standard cubic feet per hour (SCFH). The hydrogen was introduced in the 15 to 25 SCFH range. By changing the hydrogen ratio the aldehyde production rate and other parameters could be appropriately and reversibly controlled.

During the reaction isomeric valeraldehyde trimers and some tetramers were formed. At an equilibrium concentration they were in the concentration range of from about 50 to 80% by wt.

After the reaction system came to equilibrium, the rate of hydrogen gas feed introduction was

decreased from 19.2 to 17.6 SCFH (1 SCFH=28.3 $dm^3/hr$) during the seventh day of the run. This resulted in an increased production rate. As expected, this process was fully reversible. Also, a decrease of the synthesis gas feed rate above the initial level, to 24.5 SCFH on the 15th day, resulted in the expected decreased production rate.

On the nineteenth day, the reaction temperature was raised to 125°. This resulted in an about 39% reaction rate increase as expected on the basis of an activation energy of 15.5 kcal. Subsequent changes of the space velocity of the synthesis gas feed at this higher temperature resulted in the expected reaction rate changes.

On the basis of the kinetic changes observed during the approximately 3 weeks of operation shown by the figure and on the basis of other continuous hydroformylations with the same catalyst system, a rate equation was developed. This rate equation did fit all the data. The rate constant remained unchanged after the startup equilibrium period for the 25 days shown. It is noted that the lack of change of the rate constant means that there is no loss of catalyst activity during this period. The only long term change in the catalyst system was some oxidation, probably by oxygen, of the phosphine ligand to the corresponding phosphine oxide. In the presence of excess phosphine, this oxidation had no adverse effect on the reaction rate. Combined gas chromatography and mass spectroscopy studies could not show any evidence of a ligand degradation similar to that reported to occur via o-phenylation in the TPP system.

Combined hydroformylation-aldolization Examples (15—18)

Example 15

Combined hydroformylation-aldolization of 1-butene at 120°C in the presence of tris-(trimethylsilylethyl diphenyl phosphine) [SEP] rhodium carbonyl hydride complex

$$2C_2H_5CH{=}CH_2 \xrightarrow{CO/H_2} 2C_4H_9CHO$$

$$\downarrow -H_2O$$

$$\underset{\substack{|\\ C_3H_7}}{C_4H_9CH{=}CCHO} \xrightarrow{H_2} \underset{\substack{|\\ C_3H_7}}{C_5H_nCHCHO}$$

n,n-enal    n,n-anal

The combined hydroformylation, aldolization and hydrogenation of butene-1 was studied under typical conditions of the present hydroformylation process. The DTS rhodium complex was utilized as a typical substituted alkyl diaryl phosphine rhodium complex catalyst for hydroformylation and hydrogenation. Potassium hydroxide in methoxytriglycol was employed as an aldolization catalyst. The methoxytriglycol was also used as the solvent for the other components of the mixture. The catalyst system was employed at the 110 ppm rhodium concentration level. The ligand to rhodium ratio was 140. The 1-butene reactant was employed in a standard manner. The initial $H_2/CO$ mixture used to pressure the mixture to 350 psi (26 atm.) had a 5/1 mole ratio. The feed gas to maintain this pressure was a 1.5 to 1 mixture. The latter ratio was employed because it is theoretically needed to produce the n,n- and i/n-anals.

The reaction and product parameters of a group of experiments designed to observe the effect of varying concentrations of KOH are summarized in Table 11. The product parameters, i.e., selectivities to the various products were obtained by glc analyses. For the analyses of the $C_5$ and $C_{10}$ aldehydes, a special 2m Carbowax column 10% CW on Chromosorb P diatomaceous earth was used. This was provided by Supelco, Inc., Supelco Park, PA. It provided good separation of the n,n-enal from the n,n-anal.

TABLE 11

Combined hydroformylation-aldolization of 1-butene at 120°C and 350 psi (26 atm) in the presence of SEP rhodium complex and varying amounts of KOH

SEP=L=$PH_2CH_2CH_2Si(CH_3)_3$: L/Rh=140; Rh=110 ppm

| Seq. No. | KOH % | $H_2/CO$ Ratio Initial | $H_2/CO$ Ratio Feed | $H_2/CO$ Ratio Final | Fraction of $H_2/CO$ reacted: Rate constant k, $min^{-1}$ | Fraction of $H_2/CO$ reacted: Conversion % | Reaction time min. | Approx, selectivities to aldehydes, Mole %: C5's i | Approx, selectivities to aldehydes, Mole %: C5's n | Approx, selectivities to aldehydes, Mole %: n,n(i)-anal | Approx, selectivities to aldehydes, Mole %: n,n-enal | n/i Ratio | % n, 100n/(n+I) | Selectivity to n-Butane mole % |
|---|---|---|---|---|---|---|---|---|---|---|---|---|---|---|
| 1 | Nil | 5 | 1.08 | 2.7 | 0.056 | 80 | 50 | 9.2 | 90.8 | | | 9.9 | | 14.0 |
| 2 | Nil | 5 | 1.5 | 24.1 | 0.051 | 80 | 42 | 3.1 | 96.9 | | | 32.1 | 96.9 | 18.8 |
| 3 | Nil | 5 | 1.5 | 26.8 | 0.05 | 15 | 4 | 3.3 | 96.7 | | | 29.1 | 96.7 | |
| 2 | Nil | 5 | 1.50 | 21.3 | 0.042 | 15 | 6 | 6.7 | 93.3 | | | 13.8 | | |
| | | | | | | 45 | 15 | 5.6 | 94.2 | | | 16.3 | | |
| | | | | | | 60 | 24 | 5.5 | 94.5 | | | 17.2 | | |
| | | | | | | 80 | 80 | 6.2 | 93.8 | | | 15.0 | 93.8 | 17.7 |
| 3 | 0.05 | 5 | 1.50 | 47 | 0.039 | 80 | 34 | 4.1 | 47.5 | 8.4 | 40.0 | 34.9 | 97.2 | 28.9 |
| 4 | 0.05 | 5 | 1.50 | 17.7 | 0.061 | 15 | 4 | 6.1 | 81.6 | 0.8 | 11.7 | 17.6 | 94.6 | |
| | | | | | | 30 | 18 | 5.5 | 76.8 | 1.0 | 16.7 | 20.5 | 95.4 | |
| | | | | | | 45 | 11 | 5.4 | 72.9 | 1.5 | 20.2 | 21.6 | 95.6 | |
| | | | | | | 60 | 16 | 5.2 | 67.3 | 2.7 | 24.7 | 23.5 | 95.9 | |
| | | | | | | 80 | 30 | 5.9 | 51.5 | 7.8 | 34.8 | 23.2 | 95.9 | 17.7 |

TABLE 11 (contd.)
Combined hydroformylation-aldolization of 1-butene at 120°C and 350 psi (26 atm) in the presence of SEP rhodium complex and varying amounts of KOH

SEP=L=$PH_2CH_2CH_2Si(CH_3)_3$: L/Rh=140; Rh=110 ppm

| Seq. No. | KOH % | $H_2/CO$ Ratio | | | Fraction of $H_2/CO$ reacted | | Reaction time min. | Approx, selectivities to aldehydes Mole % | | | | n/i Ratio | % n, 100n n+I | Selectivity to n-Butane mole % |
|---|---|---|---|---|---|---|---|---|---|---|---|---|---|---|
| | | Initial | Feed | Final | Rate constant k, $min^{-1}$ | Conversion % | | C5's i | C5's n | n,n(i)-anal | n,n-enal | | | |
| 5 | 0.10 | 5 | 1.17 | 6.52 | 0.062 | 15 | 5 | 2.9 | 17.8 | 3.0 | 50.2 | 42.9 | 94.3 | |
| | | | | | | 80 | 32 | 9.3 | 34.1 | 13.0 | 43.6 | 15.8 | 77.1 | 15.0 |
| 6 | 0.10 | 5 | 1.50 | 5.0 | 0.048 | 80 | 42 | 3.4 | 27.1 | 18.3 | 51.2 | 49.2 | 98.0 | 31.3 |
| 7 | 0.20 | 5 | 1.50 | 5.0 | 0.043 | 80 | 40 | 2.9 | 16.0 | 16.0 | 65.2 | 60.5 | 98.4 | 28.8 |
| 8 | 0.20 | 5 | 1.50 | 13.6 | 0.049 | 15 | 3 | — | — | 10.8 | 89.2 | | — | |
| | | | | | 0.028 | 30 | 5 | 3.7 | — | 11.5 | 84.9 | 50.6 | 98.0 | |
| | | | | | | 45 | 12 | 3.0 | — | 12.0 | 85.0 | 64.4 | 98.5 | |
| | | | | | | 60 | 10 | 3.3 | 7.5 | 13.6 | 75.8 | 57.1 | 98.3 | |
| | | | | | | 80 | 32 | 3.6 | 17.7 | 18.6 | 60.1 | 48.0 | 98.0 | |
| | | | | | | 92 | 62 | 4.9 | 14.1 | 34.5 | 46.5 | 35.9 | 97.3 | 16.0 |

However, the separation of the n,n-enal from the i,n-enal was not good. The small quantities of the i,n-enal formed could not be determined. Therefore, the overall n,i-ratios in the reaction mixtures with KOH could not be exactly determined. The aldehyde selectivity to the main final $C_{10}$ aldehyde product, the n,n-enal, also includes minor quantities of the i,n-anal. However, this inclusion causes less than 10% change in the composition, since the minor i-$C_5$ aldehyde is crossaldolized at a very slow rate. The glc percentages are indicated on the basis of the peak intensities. No corrections were made for the possibly different glc response to $C_5$ and $C_{10}$ compounds.

In the first four experiments, the hydroformylation of butene was studied in methoxytriglycol but in the absence of KOH aldolization catalyst (Seq. Nos. 1 to 4) for comparison. All three experiments started with 5/1 $H_2$/CO gas. In the first experiment, the $H_2$/CO ratio of the feed gas was close to one as usual. This experiment gave the usual high n/i ratio of $C_5$ aldehydes. This indicated that the solvent is an advantageous one, comparable to other polar oxygenated solvents (Seq. No. 1).

The rest of the experiments used the same initial $H_2$/CO ratio of 5 but a different $H_2$/CO feed, of 1.5. Also, the contents of the third and fourth reaction mixture were sampled for comparison with the experiments using added KOH. This and the other sampled runs provided less reliable absolute values than the uninterrupted experiments. However, sequences gave comparative relative numbers which showed the change of selectivity with increasing conversion.

The second experiment (Seq. No. 2) showed a much increased n/i ratio compared to the first. This was the consequence of the increasing $H_2$/CO ratio, i.e., decreasing CO partial pressure during the reaction. Due to decreased availability of CO, this run also resulted in more hydrogenation of the 1-butene starting material and isomerized 2-butenes to n-butane.

The results of the first sampled experiment are somewhat similar. This experiment shows that as a consequence of increasing $H_2$/CO ratio, the selectivity is much higher at 80% conversion. (Seq. No. 3).

The fourth experiment (Seq. No. 4) was sampled four times during the run. It showed that up to 60% conversion, the n/i ratio was moderately increasing as an apparent consequence of the increasing $H_2$/CO ratio in the reaction mixture.

The second group of experiments (Seq. Nos. 5—10) was run using varying amounts of KOH, in the 0.05 to 0.2% range, under the same conditions. The data indicated that 0.2% KOH was sufficient for the rapid conversion of the primary n-$C_5$ aldehyde product (Seq. Nos. 7 and 8). The adolization rate was much slower when 0.05% KOH was used (Seq. Nos. 5 and 6). The rate of the hydroformylation was estimated on the basis of the measured rate of synthesis gas consumption. Increasing $H_2$/CO ratios generally resulted in increased n/i ratios and increased percentages of n-butane formation. Due to apparent CO starvation, the non-sampled mixtures gave rise to significantly higher $H_2$/CO ratios than those frequently sampled during the run.

The hydrogenation of the unsaturated aldehyde to the saturated aldehyde was relatively low. At 45% synthesis gas conversion, the percentage n,n-anal formed was less than 10% of the n,n-enal present. At that conversion, the overall selectivity to the n,n-enal was in excess of 80%.

Example 16

Sequential hydroformylation, aldolization, hydrogenation in separate steps

In a series of experiments, n-valeraldehyde was produced by the hydroformylation of 1-butene and separated from the i-isomer. A 20% methoxytriglycol solution of the n-valeraldehyde was then aldolized to provide the n,n-enal condensation product. It was observed that the aldolization was much slower in the absence of the hydroformylation catalyst system than in the presence of it in the previous example. After 30 minutes reaction time, only a 1.2% conversion was reached. After 14 hours, the aldehyde conversion was 47.2%, i.e., the concentration of the n,n-enal in mole equivalents was 47.2%.

During the above experiment, and other experiments with KOH solutions in methoxytriglycol, yellow, then amber, then brown color formation was observed, indicating potential instability. The addition of 2% KOH to methoxytriglycol resulted in an amber color even at room temperature. Therefore, the amount of KOH in the hydroformylation experiments was minimized.

To the reaction mixture from the above aldolization experiment, the hydroformylation catalyst of the previous Example was added. Then the mixture was pressured to 570 psi (~39 atm.) and heated as usual to 120°C with a 20/1 mixture of $H_2$/CO. A high $H_2$/CO ratio was used to increase the hydrogenation rate of the n,n-enal to the n,n-anal.

The hydrogenation of the n,n-enal to the n,n-anal was followed by glc. During the first 90 minutes, the percentage conversion increased as follows: 6 (5 min.); 13 (20 min.); 21 (40 min.); 28 (60 min.); and 39 (90 min.). Under these conditions, no further significant aldolization of the n-$C_5$ aldehyde occurred.

Example 17

Combined hydroformylation-aldolization of 1-butene with various tris-(alkyl diphenyl phosphine) rhodium carbonyl hydride complexes

The combined hydroformylation aldolization of 1-butene under the conditions of Example 15 was also studied with the tris-(n-butyl diphenyl phosphine) and the tris-(n-hexyl diphenyl phosphine) complexes. The SEP complex was also used in this group of experiments under similar conditions but using a 1/1 rather than a 5/1 initial $H_2$/CO reactant ratio. The results are shown in Table 12.

Overall, the data of Table 12 show that different alkyl diphenyl phosphine complexes are similar catalysts for combined hydroformylation aldolization. The results also indicate that the provision of sufficient carbon monoxide for hydroformylation is a key factor in avoiding olefin hydrogenation.

The first two experiments (Seq. Nos. 1 and 2) with the butyl diphenyl phosphine complex (A) show the effect of the KOH on the aldolization. The results are similar to those obtained in comparative experiments using the SEP complex in a previous example (See Table 11). The second pair of experiments (Seq. Nos. 3 and 4) shows the effect of starting with a synthesis gas having a low, i.e., 1.5, $H_2CO$ ratio. Lower selectivities to the n-product are obtained but the reaction rates are increased and the by-product n-butane formation is drastically reduced. The two different catalyst ligands used in these experiments, i.e., n-hexyl diphenyl phosphine (B) and trimethylsilylethyl phosphine (C), led to similar results.

TABLE 12

Combined hydroformylation aldolization of 1-butene at 120°C in the presence of various tris-(alkyl diphenyl phosphine) rhodium carbonyl hydride complexes

$L=Ar_2PR$; A: $R=C_4H_9$; B: $R=C_6H_9$; C: $R=CH_2CH_2Si(CH_3)_3$;
L/Rh=140; Rh=110 ppm; Pressure=350 psi (26 atm)

| Seq. No. | Ligand species | KOH % | $H_2/CO$ Ratio | | | Fraction $H_2/CO$ reacted | | Reaction time min. | Approximate selectivities to aldehydes, Mole % | | | | n/i Ratio | % n 100n n+I | Selectivity to n-Butane mole % |
|---|---|---|---|---|---|---|---|---|---|---|---|---|---|---|---|
| | | | | | | | | | C5's | | C10's | | | | |
| | | | Initial | Feed | Final | Rate constant k, $min^{-1}$ | Conversion % | | i- | n | n,n(i)-anal | n,n-enal | | | |
| 1 | A | Nil | 5 | 1.5 | 20.1 | 0.058 | 15 | 4 | 4.9 | 95.1 | | | 19.6 | 95.1 | |
| | | | | | | | 80 | 34 | 4.4 | 95.6 | | | 21.9 | 95.6 | 13.0 |
| 2 | A | 0.1 | 5 | 1.5 | 21.0 | 0.043 | 15 | 5 | 10.7 | 16.8 | 7.3 | 65.2 | 15.1 | 93.8 | |
| | | | | | | | 80 | 42 | 6.7 | 24.5 | 21.9 | 46.9 | 24.1 | 96.0 | 13.7 |
| 3 | B | 0.1 | 1.5 | 1.5 | 11.6 | 0.151 | 15 | 2 | 18.5 | 75.2 | | 7.3 | 4.8 | 82.8 | |
| | | | | | | | 80 | 12 | 14.1 | 63.1 | 6.7 | 16.0 | 7.7 | 88.5 | 1.9 |
| 4 | C | 0.1 | 1.5 | 1.5 | 6.8 | 0.088 | 15 | 3 | 18.0 | 75.7 | | 6.3 | 4.9 | 83.1 | |
| | | | | | | | 80 | 20 | 16.0 | 73.6 | 0.9 | 9.5 | 5.9 | 88.5 | |
| | | | | | | | 109 | 120 | 19.0 | 12.8 | 34.4 | 33.8 | 7.5 | 88.2 | 2.1 |

Example 18
Combined hydroformylation-aldolization of 1-butene at 145°C in the presence of tris-SEP and tris-TPP rhodium carbonyl hydride complexes

The combined hydroformylation-aldolization of 1-butene was also studied under similar conditions at 145°C. At this temperature, the known tris-TPP complex is unstable under the reaction conditions. In contrast, the novel tris-SEP complex is stable. The experimental conditions and results are shown in Table 13.

As it is shown in this table, in the first pair of experiments (Seq. Nos. 1 and 2), both the triphenyl phosphine (TPP) complex and the trimethylsilylethyl diphenyl phosphine (SEP) complex were employed as hydroformylation catalysts in methoxytriglycol in the absence of KOH. A comparison of the results showed that the rate of the SEP complex catalyzed reaction was higher. Even more significantly, the selectivity of the SEP complex to produce aldehydes of high n/i ratios was much higher (Seq. No. 1). At 80% conversion, the SEP catalyzed reaction had a 7.6 n/i ratio. The comparable ratio for the TPP system was 3.1 (Seq. No. 2). Most revealingly, the TPP reaction gave an n/i ratio of 12.4 at 15% conversion. Apparently, during the further course of the experiment, the TPP catalyst system decomposed and led to species of much lower catalytic activity and selectivity.

In the second pair of experiments (Seq. Nos. 3 and 4), the same two catalyst systems were employed in the presence of KOH to effect hydroformylation and aldolization. KOH was found to be an effective aldolization catalyst. Both complexes were also effective in catalyzing the hydrogenation of the aldol condensation products. However, the difference between the activity and selectivity of the two catalysts remained. The SEP complex plus KOH system produced a 6.6 n/i ratio of aldehydes at 80% conversion (Seq. No. 3). The comparative n/i ratio for the TPP complex plus base was only 4.2 (Seq. No. 4).

TABLE 13

Combined hydroformylation aldolization of 1-butene at 145°C and 350 psi (~26 atm.) in the presence of tris-SEP and tris-TPP rhodium carbonyl hydride complexes

SEP=$Ph_2PCH_2CH_2CH_2Si(CH_3)_3$;
TPP=$Rh_3P$; L/Rh=140; Rh=110 ppm

| Seq. No. | Ligand species | KOH % | $H_2/CO$ Ratio | | | Fraction $H_2/CO$ reacted | | Reaction time min. | Approximate selectivities to aldehydes, Mole % | | | | n/i Ratio | %n 100n n+I | Selectivity to n-Butane mole % |
|---|---|---|---|---|---|---|---|---|---|---|---|---|---|---|---|
| | | | Initial | Feed | Final | Rate constant k, $min^{-1}$ | Conversion % | | C5's i- | C5's n | C10's n,n(i)-anal | C10's n,n-enal | | | |
| 1 | SEP | Nil | 5 | 1.17 | 2.7 | 0.174 | 79 | 18 | 11.6 | 88.4 | | | 7.6 | 88.6 | 12.0 |
| 2 | TPP | Nil | 5 | 1.5 | 14.7 | 0.138 | 15 | 1 | 7.5 | 92.5 | | | 12.4 | 92.5 | |
| | | | | | | | 80 | 90 | 24.6 | 75.4 | | | 3.1 | 75.4 | 15.4 |
| 3 | SEP | 0.2 | 5 | 1.5 | 4.6 | 0.07 | 80 | 42 | 15.2 | 21.1 | 44.4 | 19.4 | 6.7 | 87.0 | 19.4 |
| 4 | TPP | 0.1 | 5 | 1.5 | 6.4 | 0.121 | 15 | 1.5 | 8.6 | 38.4 | 3.0 | 50.0 | 16.8 | 94.4 | |
| | | | | | | | 80 | 80 | 29.7 | 12.0 | 48.5 | 9.8 | 4.2 | 80.6 | 13.2 |

The above quantitive observations on the relative stability of the SEP and TPP based systems could be qualitatively predicted when observing the respective reaction mixtures after the reactions. The SEP systems without and with base were yellow and amber, respectively. The TPP systems with and without base become black.

## Claims

1. A continuous hydroformylation process comprising reacting an olefinic compound with hydrogen and carbon monoxide in the presence of a reaction mixture comprising (1) a rhodium-containing catalyst having no reactive halogen and having at least one ligand complexed with said rhodium, wherein said ligand comprises a complex compound containing at least one diaryl phosphino alkyl group and wherein the number of such diaryl phosphino alkyl groups in complex association with said rhodium is at least 2, and (2) a non-complexed ligand substantially all of which is a non-complexed compound containing at least one diaryl phosphino alkyl group wherein the molar ratio of non-complexed ligand to rhodium is greater than 75:1 and wherein the ratio of the partial pressures of hydrogen to carbon monoxide is at least 3:1.

2. A process according to claim 1 wherein the molar ratio of non-complexed ligand to rhodium is greater than 100:1 which results in an aldehyde product having a normal to iso isomer ratio of at least about 4:1.

3. A process according to claim 1 wherein said reaction is conducted at a temperature; olefin, CO and $H_2$ feed rates; a rhodium concentration and a non-complexed ligand to rhodium molar ratio so as to provide a rate of production of aldehyde of at least about 0.1 g mole/l-hr, and wherein said aldehyde is continuously removed from the vapor phase of said reaction mixture.

4. A process according to claim 3 wherein said reaction is conducted at a temperature; olefin, $H_2$ and CO feed rates; a rhodium concentration and a non-complexed ligand to rhodium molar ratio so as to provide an aldehyde product having a normal to iso isomer ratio of at least about 3:1, and wherein the ratio of the partial pressures of hydrogen to carbon monoxide is at least 4:1.

5. A process according to claim 3 or 4 wherein said complexed and non-complexed compounds containing at least one diaryl phosphino alkyl group are of the formula:

$$[(Ar_2PQ)_bE^yR_{y-b}]$$

wherein

Ar is an aryl group containing from 6 to 10 carbon atoms,

Q is an alkylene radical or an alkylene radical, the carbon chain of which is interrupted with at least one ether oxygen or phenylene group, wherein said alkylene radical contains from 2 to 30 carbon atoms:

E represents

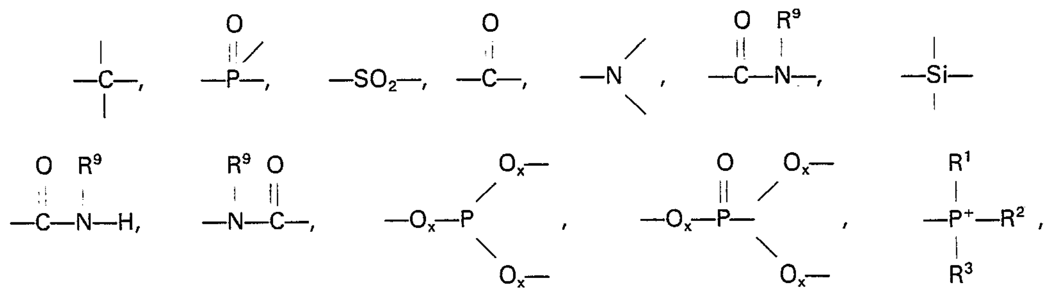

—O— or —S—, wherein

$R^1$, $R^2$ and $R^3$ are each alkyl groups containing from 1 to 30 carbon atoms, $R^9$ is H, an alkyl group containing from 1 to 30 carbon atoms or an aryl group containing from 6 to 10 carbon atoms, and wherein x is 0 or 1 with the proviso that at least one x is 1;

y represents the bonds of the group E available for bonding to said Q and R groups;

b represents an integer of from 1 to 4 provided that y−b is not less than zero;

R independently represent an alkyl group containing from 1 to 30 carbon atoms or an aryl group containing from 6 to 10 carbon atoms and with the proviso that when E is

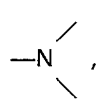

y=3, and b=1, R is $R_2$ the divalent radical

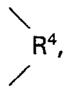, 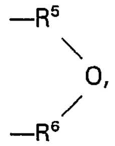, 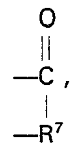, or 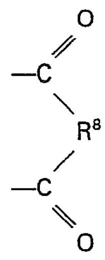

which together with the N atom form a heterocyclic ring, wherein $R^4$, $R^5$, $R^6$, $R^7$ and $R^8$ are hydrocarbyl radicals such that said heterocyclic ring contains 5 or 6 carbon atoms.

6. A process according to claim 5 wherein b=1.

7. A continuous hydroformylation process comprising:

(A) reacting an olefinic compound with hydrogen and carbon monoxide in the presence of a reaction mixture comprising:

(1) a rhodium-containing catalyst of the formula:

$$[(Ar_2PQ)_bE^yR_{y-b}]_g \cdot (RhX_n)_s$$

wherein

Ar is an aryl group containing from 6 to 10 carbon atoms,

Q is an alkylene radical or an alkylene radical, the carbon chain of which is interrupted with at least one ether oxygen or phenylene group, wherein said alkylene radical contains from 2 to 30 carbon atoms;

E represents

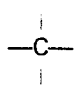 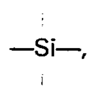, 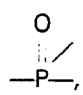, $—SO_2—$, 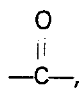, 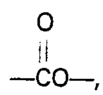, 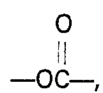, 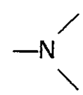, 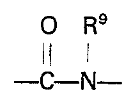

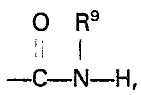, 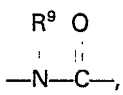, 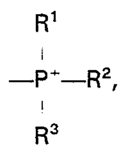, 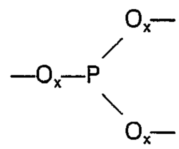 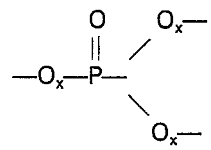 —O— or —S—,

wherein

$R^1$, $R^2$ and $R^3$ are each alkyl groups containing from 1 to 30 atoms, $R^9$ is H, an alkyl group containing from 1 to 30 carbon atoms or an aryl group containing from 6 to 10 carbon atoms, and wherein x is 0 or 1 with the proviso that at least one x is 1;

y represents the bonds of the group E available for bonding to said Q and R groups;

b represents an integer of from 1 to 4 provided that y−b is not less than zero;

R independently represents an alkyl group containing from 1 to 30 carbon atoms or an aryl group containing from 6 to 10 carbon atoms and with the proviso that when E is

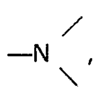,

y=3, and b=1, R is $R_2$ the divalent radical

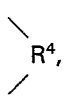, 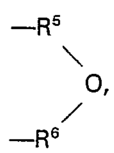, 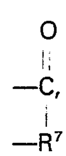, or 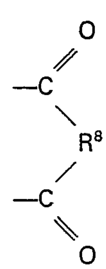

which together with the N atom form a heterocyclic ring, wherein $R^4$, $R^5$, $R^6$, $R^7$ and $R^8$ are hydrocarbyl radicals such that said heterocyclic ring contains 5 or 6 carbon atoms;

x is an anion or organic ligand, excluding halogen, satisfying the coordination sites of the rhodium metal;

g is 1 to 6 with the proviso that g multiplied by b is 1 to 6;

n is 2 to 6; and

s is 1 to 3.

(2) a non-complexed ligand substantially all of which is a non-complexed compound of the formula:

$$(Ar_2PQ)_bE^yR_{y-b}$$

wherein

Ar, Q, E, R, y and b are as defined above, said reaction being conducted at a temperature; olefin, $H_2$ and CO feed rates; a rhodium concentration, and a non-complexed ligand molar to rhodium ratio so as to provide a rate of production of aldehyde product of at least about 0.1 g mole/l-hr, and the ratio of partial pressures of said hydrogen to said carbon monoxide being at least 3:1; and

(B) continuously removing aldehyde from the vapor phase of said reaction mixture.

8. A process according to claim 7 wherein the rhodium-containing catalyst has the formula:

$$[(Ar_2PQ)E^yR_{y-1}]_3 \cdot RhH(CO)$$

the non-complexed ligand has the formula:

$$(Ar_2PQ)E^yR_{y-1}$$

and wherein said reaction is conducted at a temperature; olefin, $H_2$ and CO feed rates; a rhodium concentration, and a non-complexed ligand to rhodium molar ratio so as to provide a $C_{n+1}$ aldehyde product from a $C_n$ olefin having a normal to iso isomer ratio of at least about 4:1.

9. A hydroformylation process for converting a $C_n$ olefinic compound to a $C_{2n+2}$ aldehyde comprising reacting said olefin with $H_2$ and CO in the presence of a base aldol condensation catalyst and the rhodium-containing catalyst as defined in claim 7.

10. A continuous hydroformylation process for the production of aldehydes comprising the step of reacting a 1-n-olefin with $H_2$ and CO in the presence of a tris- or bis(alkyl diaryl phosphine) rhodium carbonyl complex catalyst free from halogen on the rhodium and excess free tertiary phosphine ligand wherein ratios of $H_2$/CO and ligand/Rh are employed to produce as the major products n- and i-aldehydes in a ratio above 4:1.

11. A process according to claim 10 wherein the partial pressure of the CO reactant is less than about 1379 kPa.

**Patentansprüche**

1. Kontinuierliches Hydroformylierungs-Verfahren, dadurch gekennzeichnet, daß eine olefinische Verbindung mit Wasserstoff und Kohlenmonoxid in Gegenwart einer Reaktionsmischung zur Reaktion gebracht wird, die (1) einen rhodiumhaltigen Katalysator mit keinem reaktiven Halogen und mindestens einem mit dem Rhodium komplexierten Liganden, wobei der Ligand eine Komplexverbindung umfaßt, die mindestens eine Diarylphosphinoalkylgruppe enthält und wobei die Zahl der sich in komplexer Verbindung mit dem Rhodium befindlichen Diarylphosphinoalkylgruppen mindestens 2 beträgt, und (2) einen nicht komplexierten Liganden enthält, der im wesentlichen vollständig eine nicht komplexierte Verbindung ist, die mindestens eine Diarylphosphinoalkylgruppe enthält, wobei das molare Verhältnis von nicht komplexierten Liganden zu Rhodium größer als 75:1 ist und das Verhältnis der Partialdrücke von Wasserstoff zu Kohlenmonoxid mindestens 3:1 beträgt.

2. Verfahren nach Anspruch 1, dadurch gekennzeichnet, daß das molare Verhältnis von nicht komplexiertem Liganden zu Rhodium größer als 100:1 ist, was zu einem Aldehydprodukt mit einem Verhältnis von Normal- zu Iso-Isomer von mindestens etwa 4:1 führt.

3. Verfahren nach Anspruch 1, dadurch gekennzeichnet, daß die Reaktion bei einer Temperatur, Olefin-, CO— und $H_2$-Einspeisungsgeschwindigkeiten, einer Rhodium-Konzentration und einem solchen molaren Verhältnis von nicht komplexiertem Liganden zu Rhodium durchgeführt wird, daß eine Produktionsgeschwindigkeit von Aldehyd von mindestens etwa 0,1 g Mol/l-h erhalten wird, und daß der Aldehyd kontinuierlich aus der Dampfphase der Reaktionsmischung, entfernt wird.

4. Verfahren nach Anspruch 3, dadurch gekennzeichnet, daß die Reaktion bei einer Temperatur, Olefin-, $H_2$— und CO-Einspeisungsgeschwindigkeiten, einer Rhodiumkonzentration und einem solchen molaren Verhältnis von nicht komplexiertem Liganden zu Rhodium durchgeführt wird, daß ein Aldehydprodukt mit einem Verhältnis von Normal- zu Iso-Isomer von mindestens etwa 3:1 erhalten wird, und daß das Verhältnis der Partialdrücke von Wasserstoff zu Kohlenmonoxid mindestens 4:1 beträgt.

5. Verfahren nach Anspruch 3 oder 4, dadurch gekennzeichnet, daß die komplexierten und nicht komplexierten Verbindungen, die mindestens eine Diarylphosphinoalkylgruppe enthalten, die Formel:

$$[(Ar_2PQ)_bE^yR_{y-b}]$$

besitzen, wobei Ar eine Arylgruppe mit 6 bis 10 Kohlenstoffatomen ist, Q ein Alkylenrest oder ein Alkylenrest mit einer Kohlenstoffkette, die durch mindestens einen Ethersauerstoff oder eine Phenylengruppe unterbrochen ist, ist, wobei der Alkylenrest 2 bis 30 Kohlenstoffatome enthält, E

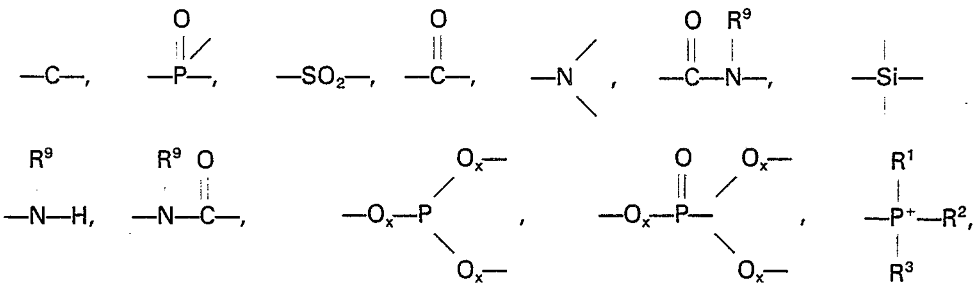

—O— oder —S— bedeutet, wobei $R^1$, $R^2$ and $R^3$ jeweils Alkylgruppen mit 1 bis 30 Kohlenstoffatomen sind, $R^9$ H, eine Alkylgruppe mit 1 bis 30 Kohlenstoffatomen oder eine Arylgruppe mit 6 bis 10 Kohlenstoffatomen ist, x 0 oder 1 ist mit der Maßgabe, daß mindestens ein x 1 ist, y die Bindungen der Gruppe E, die für die Bindung an die Gruppen Q und R zur Verfügung stehen, bedeutet, b eine ganze Zahl von 1 bis 4 bedeutet, vorausgesetzt, daß y−b nicht weniger als 0 ist, R unabhängig eine Alkylgruppe mit 1 bis 30 Kohlenstoffatomen oder eine Arylgruppe mit 6 bis 10 Kohlenstoffatomen mit der Maßgabe bedeutet, daß, wenn E

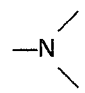

ist, y=3 ist und b=1 ist, R der zweiwertige Rest $R_2$ und zwar

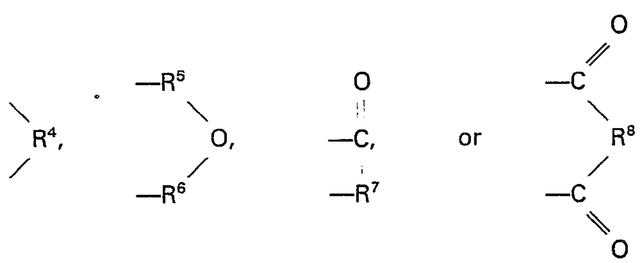

or

ist, der zusammen mit dem N-Atom einen heterocyclischen Ring bildet, wobei $R^4$, $R^5$, $R^6$, $R^7$ und $R^8$ solche Hydrocarbylreste sind, daß der heterocyclische Ring 5 oder 6 Kohlenstoffatome enthält.

6. Verfahren nach Anspruch 5, dadurch gekennzeichnet, daß b=1 ist.

7. Kontinuierliches Hydroformylierungsverfahren, dadurch gekennzeichnet, daß

(A) eine olefinische Verbindung mit Wasserstoff und Kohlenmonoxid in Gegenwart einer Reaktionsmischung zur Reaktion gebracht wird, die

(1) einen rhodiumhaltigen Katalysator mit der Formel:

wobei Ar eine Arylgruppe mit 6 is 10 Kohlenstoffatomen ist,

Q ein Alkylenrest oder ein Alkylenrest mit einer Kohlenstoffkette, die durch mindestens einen Ethersauerstoff oder eine Phenylengruppe unterbrochen ist, ist, wobei der Alkylenrest 2 bis 30 Kohlenstoffatome enthält, E

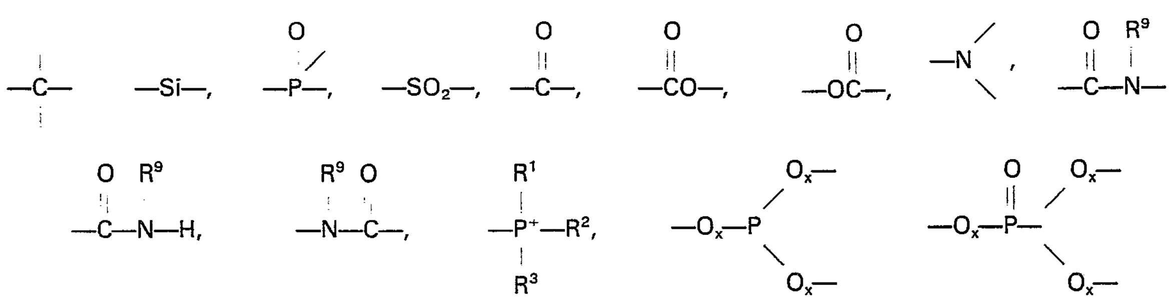

—O— oder —S— bedeutet, wobei $R^1$, $R^2$ und $R^3$ jeweils Alkylgruppen mit 1 bis 30 Kohlenstoffatomen sind, $R^9$ H, eine Alkylgruppe mit 1 bis 30 Kohlenstoffatomen oder eine Arylgruppe mit 6 bis 10 Kohlenstoffatomen ist, x 0 oder 1 ist mit der Maßgabe, daß mindestens ein x 1 ist,

y die Bindungen der Gruppe E, die für die Bindung an die Gruppen Q und R zur Verfügung stehen, bedeutet,

b eine ganze Zahl von 1 bis 4 bedeutet, vorausgesetzt, daß y−b nicht weniger als 0 beträgt,

R unabhängig eine Alkylgruppe mit 1 bis 30 Kohlenstoffatomen oder eine Arylgruppe mit 6 bis 10 Kohlenstoffatomen bedeutet mit der Maßgabe, daß, wenn E

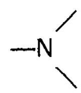

ist, y=3 ist und b=1 ist, R der zweiwertige Rest $R_2$ und zwar ist, der mit dem N-Atom einen heterocyclischen Ring bildet, wobei $R^4$, $R^5$, $R^6$, $R^7$ und $R^8$ solche Hydrocarbylreste sind, daß der heterocyclische Ring 5 oder 6 Kohlenstoffatome enthält,

X ein Anion oder ein organischer Ligand, ausgenommen Halogen, ist, das oder der die Koordinationsstellen des Rhodiummetalls absättigt,

g 1 bis 6 ist mit der Maßgabe, daß g multipliziert mit b 1 is 6 ist, n 2 bis 6 ist und s 1 bis 3 ist, und

(2) einen nicht komplexierten Liganden enthält, der im wesentlichen vollständig eine nicht komplexierte Verbindung mit der Formel

$$(Ar_2PQ)_bE^yR_{y-b}$$

ist, wobei Ar, Q, E, R, y und b die oben angegebene Bedeutung besitzen, die Reaktion bei einer Temperatur, Olefin-, $H_2$- und CO-Einspeisungsgeschwindigkeiten, einer Rhodiumkonzentration und einem solchen molaren Verhältnis von nicht komplexiertem Liganden zu Rhodium durchgeführt wird, daß eine Produktionsgeschwindigkeit von Aldehydprodukt von mindestens etwa 0,1 g Mol/l-h erhalten wird, und das Verhältnis der Partialdrücke von Wasserstoff zu dem Kohlenmonoxid mindestens 3:1 beträgt und

(B) Aldehyd kontinuierlich aus der Dampfphase der Reaktionsmischung entfernt wird.

8. Verfahren nach Anspruch 7, dadurch gekennzeichnet, daß der rhodiumhaltige Katalysator die Formel:

$$[(Ar_2PQ)E^yR_{y-1}]_3 \cdot RhH(CO)$$

besitzt, der nicht komplexierte Ligand die Formel:

$$(Ar_2PQ)E^yR_{y-1}$$

besitzt und die Reaktion bei einer Temperatur, Olefin-$H_2$-und CO-Einspeisungsgeschwindigkeiten, einer Rhodiumkonzentration und einem solchen molaren Verhältnis von nicht komplexiertem Liganden zu Rhodium durchgeführt wird, daß ein $C_{n+1}$-Aldehydprodukt von einem $C_n$-Olefin mit einem Verhältnis von Normal- zu Iso-Isomer von mindestens etwa 4:1 erhalten wird.

9. Hydroformylierungsverfahren zur Umwandlung einer $C_n$-Olefinverbindung in einen $C_{2n+2}$-Aldehyd, dadurch gekennzeichnet, daß ein Olefin mit $H_2$+CO in Gegenwart eines Basealdolkondensationskatalysators und des rhodiumhaltigen Katalysators gemäß Anspruch 7 zur Reaktion gebracht wird.

10. Kontinuierliches Hydroformylierungsverfahren für die Herstellung von Aldehyden, dadurch gekennzeichnet, daß ein 1-n-Olefin mit $H_2$ und CO in Gegenwart eines tris- oder bis-(Alkyldiarylphosphin)-rhodiumcarbonylkomplex-Katalysators, der am Rhodium frei von Halogen ist, und überschüssigem freiem tertiärem Phosphinliganden umgesetzt wird, wobei solche Verhältnisse von $H_2$/CO und Ligand/Rh verwendet werden, daß als Hauptprodukte n- und i-Aldehyde in einem Verhältnis von mehr als 4:1 hergestellt werden.

11. Verfahren nach Anspruch 10, dadurch gekennzeichnet, daß der Partialdruck des CO-Reaktanten weniger als etwa 1379 kPa beträgt.

**Revendications**

1. Procédé d'hydroformylation continu comprenant la réaction d'un composé oléfinique avec de l'hydrogène et de l'oxyde de carbone en présence d'un mélange réactionnel comprenant (1) un catalyseur contenant du rhodium ne comportant pas d'halogène réactif et ayant au moins un coordinat complexé avec le dit rhodium, dans lequel le dit coordinat comprend un composé complexe contenant au moins un groupe diarylphosphinoalkyle et dans lequel le nombre de ces groupes diarylphosphinoalkyle en association complexe avec le dit rhodium est d'au moins 2, et (2) un coordinat non complexé dont la quasi-totalité est un composé non complexé contenant au moins un groupe diarylphosphinoalkyle dans lequel le rapport molaire du coordinat non complexé au rhodium est supérieur à 75:1 et dans lequel le rapport des pressions partielles de l'hydrogène à l'oxyde de carbone est d'au moins 3:1.

2. Procédé selon la revendication 1, dans lequel le rapport molaire du coordinat non complexé au

rhodium est supérieur à 100:1, ce qui conduit à un produit aldéhyde ayant un rapport isomérique normal:iso d'au moins environ 4:1.

3. Procédé selon la revendication 1, dans lequel ladite réaction est réalisée à une température, avec des débits d'alimentation en oléfine, en CO et en $H_2$, une concentration en rhodium et un rapport molaire coordinat non complexé: rhodium tels qu'ils donnent une vitesse de production d'aldéhyde d'au moins environ 0,1 g.mole/l.h et dans lequel le dit aldéhyde est éliminé en continu de la phase vapeur du dit mélange réactionnel.

4. Procédé selon la revendication 3, dans lequel ladite réaction est réalisée à une température, avec des débits d'alimentation en oléfine, en $H_2$ et en CO, une concentration en rhodium et un rapport molaire coordinat non complexé: rhodium tels qu'ils donnent un produit aldéhyde ayant un rapport isomérique normal:iso d'au moins environ 3:1 et dans lequel le rapport des pressions partielles de l'hydrogène à l'oxyde de carbone est d'au moins 4:1.

5. Procédé selon la revendication 3 ou 4, dans lequel les dits composés complexé et non complexé contenant au moins un groupe diarylphosphinoalkyle ont pour formule

$$[(Ar_2PQ)_bE^yR_{y-b}]$$

dans laquelle

Ar est un groupe aryle contenant de 6 à 10 atomes de carbone,

Q est un radical alkylène ou un radical alkylène dont la chaîne carbonée est interrompue par au moins un groupe oxygène ou phénylène, dans lequel le dit radical alkylène contient de 2 à 30 atomes de carbone;

E représente

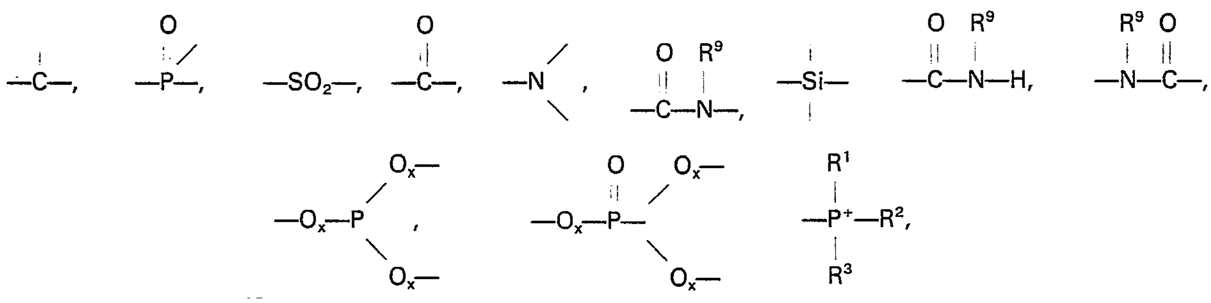

—O— ou —S—, où $R^1$, $R^2$ et $R^3$ sont chacun des groupes alkyle contenant de 1 à 30 atomes de carbone, $R^9$ est H, un groupe alkyle contenant de 1 à 30 atomes de carbone ou un groupe aryle contenant de 6 à 10 atomes de carbone et où x est 0 ou 1, à condition qu'au moins un x soit égal à 1;

y représente les liaisons du groupe E disponibles pour la liaison aux dits groupes Q et R;

b représente un nombre entier de 1 à 4, à condition que y−b ne soit pas inférieur à zéro;

R représente indépendamment un groupe alkyle contenant de 1 à 30 atomes de carbone ou un groupe aryle contenant de 6 à 10 atomes de carbone et à condition que, lorsque E est

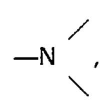

y=3 et b=1, R soit $R_2$, le radical bivalent

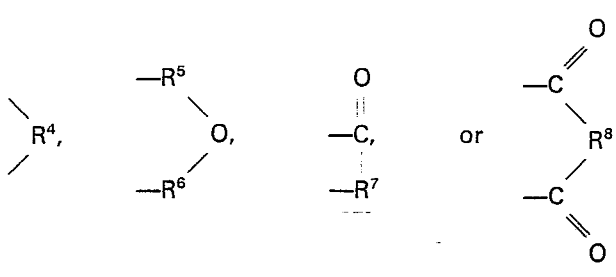

qui forment, avec l'atome de N, un hétérocycle, dans lequel $R^4$, $R^5$, $R^6$, $R^7$ et $R^8$ sont des radicaux hydrocarbyle tels que le dit hétérocycle contienne 5 ou 6 atomes de carbone.

6. Procédé selon la revendication 5, dans lequel b=1.

7. Procédé d'hydroformylation continu comprenant:

(A) la réaction d'un composé oléfinique avec de l'hydrogène et de l'oxyde de carbone en présence d'un mélange réactionnel comprenant

(1) un catalyseur contenant du rhodium de formule:

$$[(Ar_2PQ)_bE^yR_{y-b}]_g \cdot (RhX_n)_s$$

dans laquelle

Ar est un groupe aryle contenant de 6 à 10 atomes de carbone,

Q est un radical alkylène ou un radical alkylène dont la chaîne carbonée est interrompue par au moins un groupe oxygène ou phénylène, dans lequel le dit radical alkylène contient de 2 à 30 atomes de carbone;

E représente

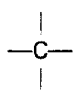, 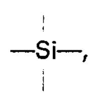, 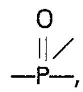, $-SO_2-$, 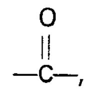, 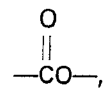, 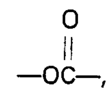, 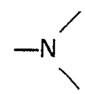, 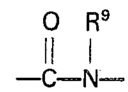

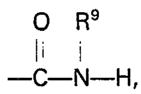, 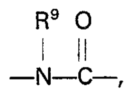, 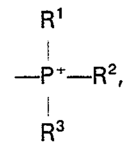, 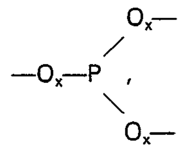, 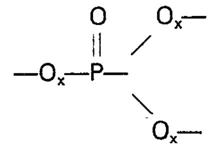

—O— ou —S—, où $R^1$, $R^2$ et $R^3$ sont chacun des groupes alkyle contenant de 1 à 30 atomes de carbone, $R^9$ est H, un groupe alkyle contenant de 1 à 30 atomes de carbone ou un groupe aryle contenant de 6 à 10 atomes de carbone et où x est 0 ou 1, à condition qu'au moins un x soit égal à 1;

y représente les liaisons du groupe E disponibles pour la liaison aux dits groupes Q et R;

b représente un nombre entier de 1 à 4, à condition que y−b ne soit pas inférieur à zéro;

R représente indépendamment un groupe alkyle contenant de 1 à 30 atomes de carbone ou un groupe aryle contenant de 6 à 10 atomes de carbone et à condition que, lorsque E est

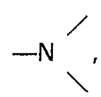,

y=3 et b=1, R soit $R_2$, le radical bivalent

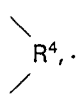, 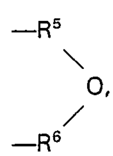, 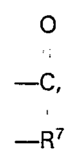, or 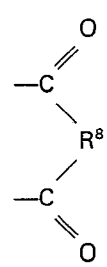

qui forment, avec l'atome de N, un hétérocycle, dans lequel $R^4$, $R^5$, $R^6$, $R^7$ et $R^8$ sont des radicaux hydrocarbyle tels que le dit hétérocycle contienne 5 ou 6 atomes de carbone;

x est un anion ou un coordinat organique, à l'exclusion d'un halogène, satisfaisant aux sites de coordination du rhodium métallique;

g est égal à 1 à 6, à condition que g multiplié par b soit égal à 1 à 6;

n est égal à 2 à 6; et

s est égal à 1 à 3;

(2) un coordinat non complexé dont la quasi-totalité est un composé non complexé de formule

$$(Ar_2PQ)_bE^yR_{y-b}$$

dans laquelle Ar, Q, E, R, y et b sont tels que définis ci-dessus, la dite réaction étant réalisée à une température, avec des débits d'alimentation en oléfine, en $H_2$ et en CO, une concentration en rhodium et un rapport molaire coordinat non complexé: rhodium tels qu'ils donnent une vitesse de production du produit aldéhyde d'au moins environ 0,1 g.mole/l.h, les rapports des pressions partielles du dit hydrogène au dit oxyde de carbone étant d'au moins 3:1; et

(B) l'élimination en continu de l'aldéhyde de la phase vapeur du dit mélange réactionnel.

8. Procédé selon la revendication 7, dans lequel le catalyseur contenant du rhodium a pour formule:

$$[(Ar_2PQ)E^yR_{y-1}]_3 \cdot RhH(CO)$$

le coordinat non complexé a pour formule:

$(Ar_2PQ)E^yR_{y-1}$

et dans lequel la dite réaction est réalisée à une température, avec des débits d'alimentation en oléfine, en $H_2$ et en CO, une concentration en rhodium et un rapport molaire ligand non complexé: rhodium, tels qu'ils donnent un produit aldéhyde en $C_{n+1}$ à partir d'une oléfine en $C_n$ ayant un rapport isomérique normal:iso d'au moins environ 4:1.

9. Procédé d'hydroformylation pour transformer un composé oléfinique en $C_n$ en aldéhyde en $C_{2n+2}$ comprenant la réaction de la dite oléfine avec $H_2$ et CO en présence d'un catalyseur de condensation aldol de base et du catalyseur contenant du rhodium tel que défini dans la revendication 7.

10. Procédé d'hydroformylation continu pour la production d'aldéhydes, comprenant les étapes de réaction d'une 1-n-oléfine avec $H_2$ et CO en présence d'un catalyseur à base d'un complexe tris- ou bis-(alkyldiarylphosphine)-rhodium-carbonyle sans halogène sur le rhodium et d'un excès de coordinat phosphine tertiaire libre dans lequel les rapports $H_2/CO$ et coordinat/Rh sont employés pour produire comme produits majeurs des n-aldéhydes et des iso-aldéhydes dans un rapport supérieur à 4:1.

11. Procédé selon la revendication 10, dans lequel la pression partielle du réactif CO est inférieure à environ 1379 kPa.

KEY STEPS IN THE MECHANISM OF PHOSPHINE-RHODIUM COMPLEX CATALYZED HYDROFORMYLATION OF OLEFINS

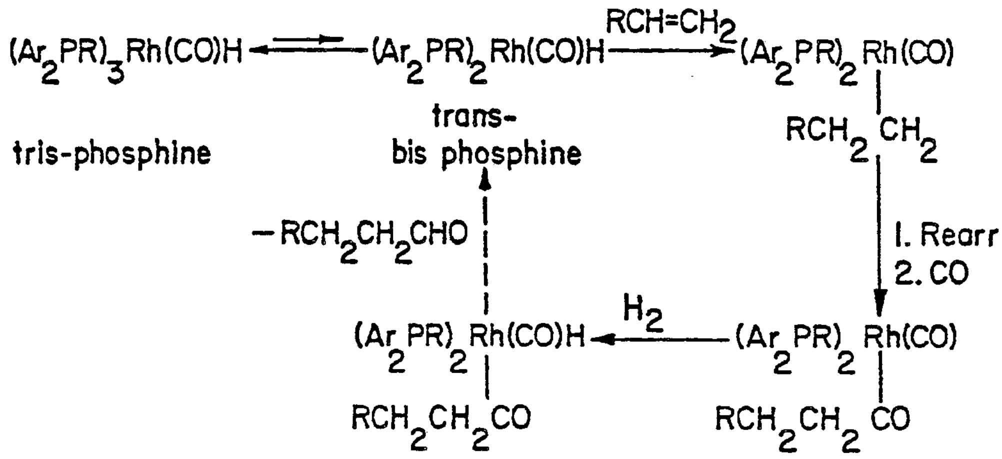


FIG.1

COMPARATIVE $^{31}P$ NMR SPECTRA OF RHODIUM COMPLEXES OF VARYING ALIPHATIC CHARACTER

Derived Via Ligand Exchange

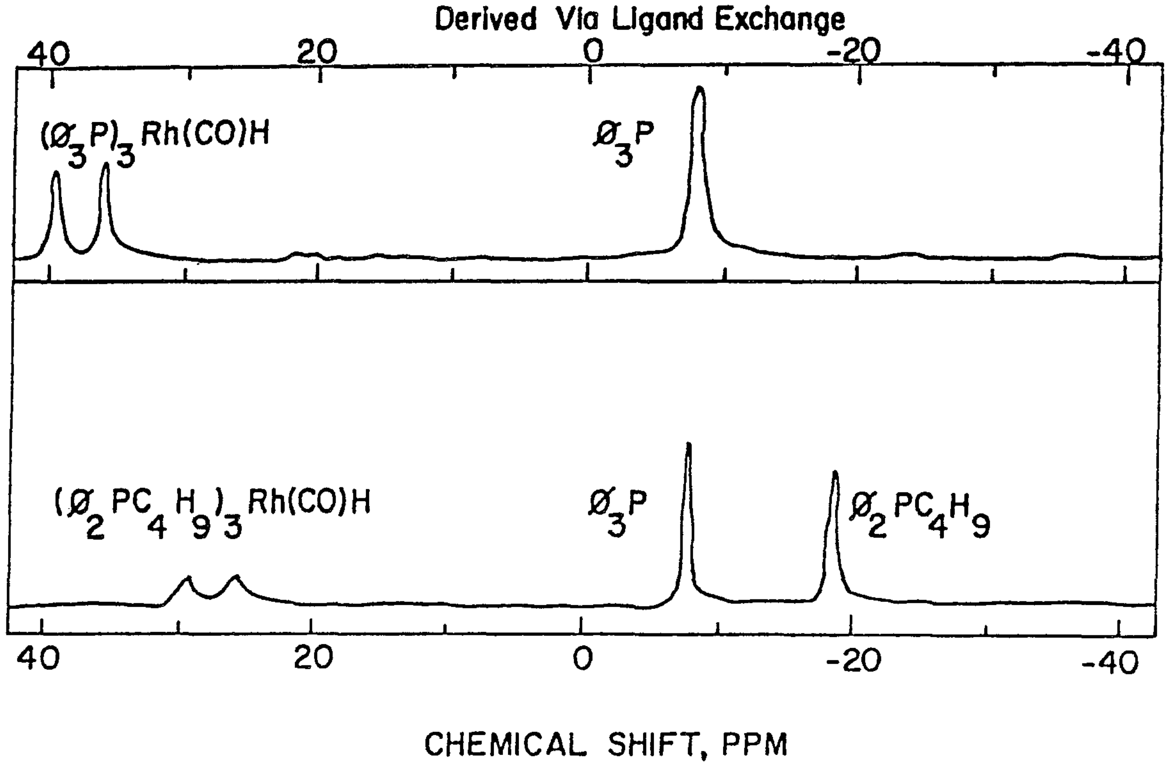


FIG. 2

$^{31}P$ NMR STUDY OF LIGAND EXCHANGE RATES AS A FUNCTION OF TEMPERATURE

$(\varnothing_3P)_3Rh(CO)H + 3\varnothing_3P^* \rightleftharpoons (\varnothing_3P^*)_3Rh(CO)H + 3\varnothing_3P$

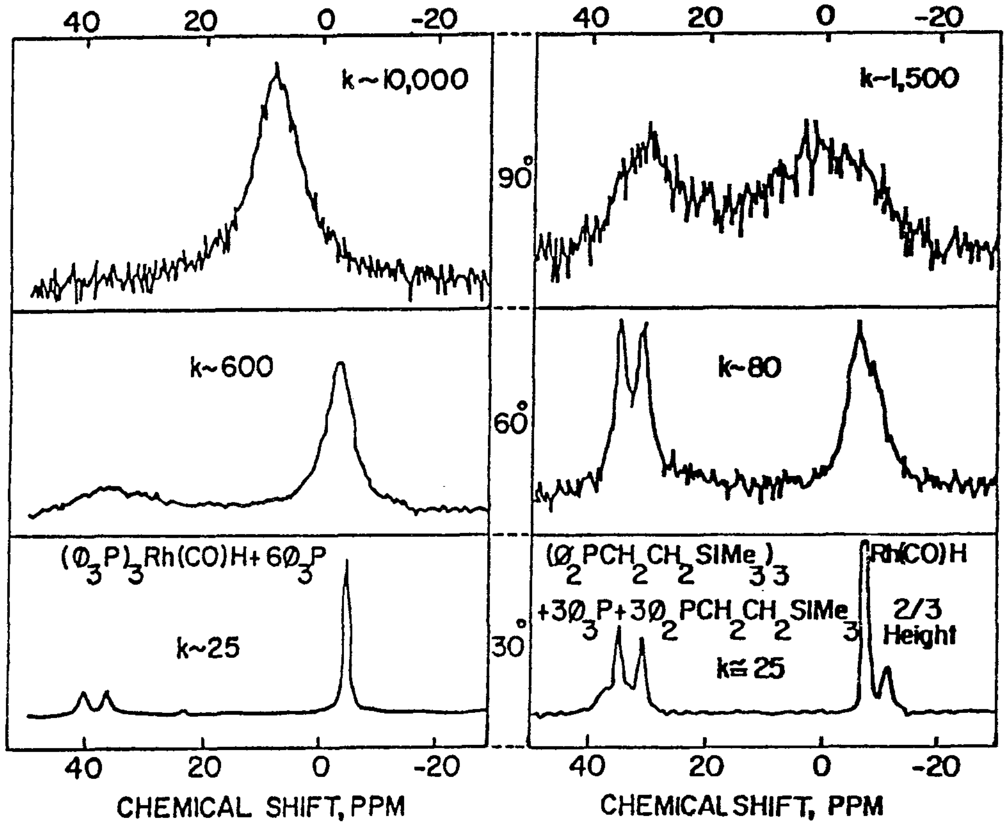


FIG.3

SCHEME OF AUTOCLAVE FOR HYDROFORMYLATION

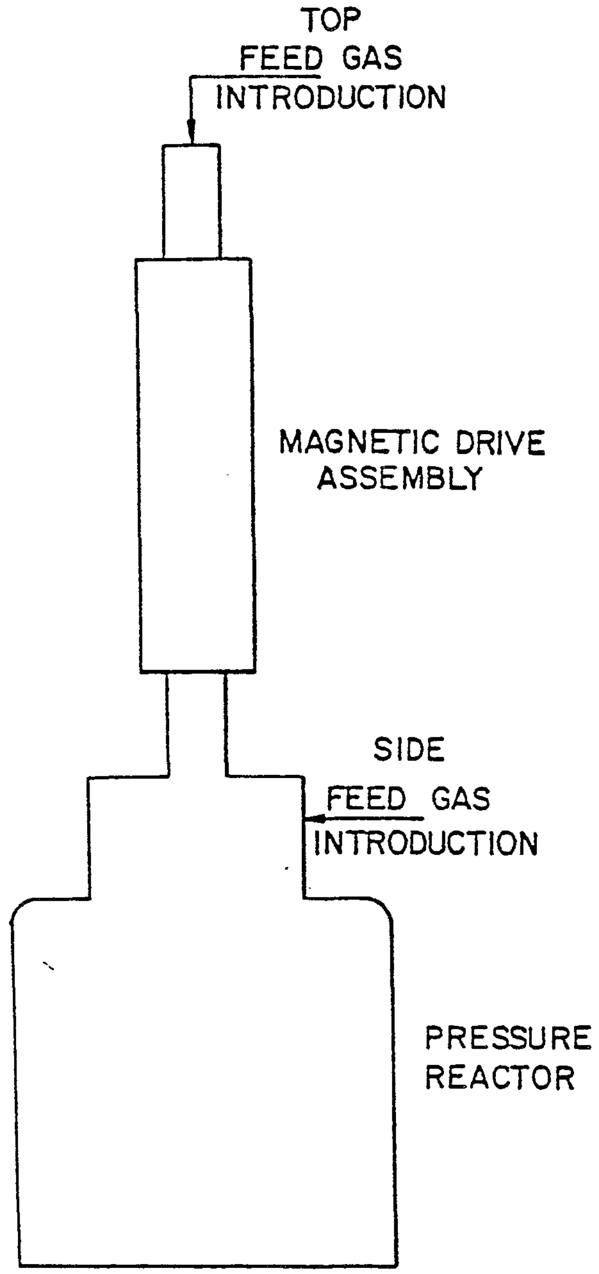


FIG. 4

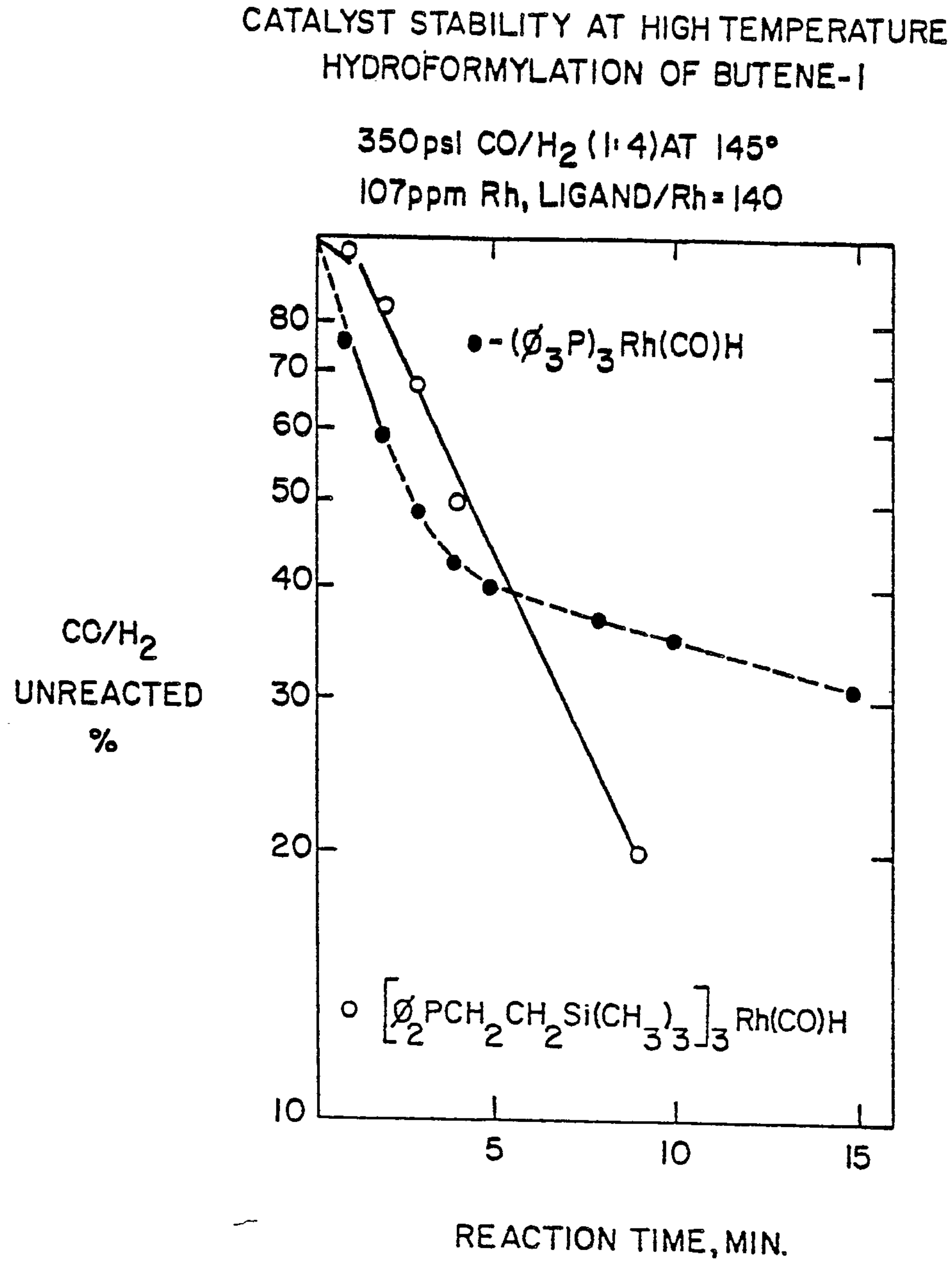
CATALYST STABILITY AT HIGH TEMPERATURE
HYDROFORMYLATION OF BUTENE-1
350psi $CO/H_2$ (1:4) AT 145°
107ppm Rh, LIGAND/Rh = 140
CO/$H_2$ UNREACTED %
80
70
60
50
40
30
20
10
● - $(Ø_3P)_3Rh(CO)H$
○ $[Ø_2PCH_2CH_2Si(CH_3)_3]_3Rh(CO)H$
5
10
15
REACTION TIME, MIN.

FIG. 5A

CATALYST STABILITY AT LOW LIGAND/Rh RATIO: HYDROFORMYLATION OF BUTENE-1

350 psi $CO/H_2$ (1:4) AT 100°

105 ppm Rh, LIGAND/Rh ≅ 27

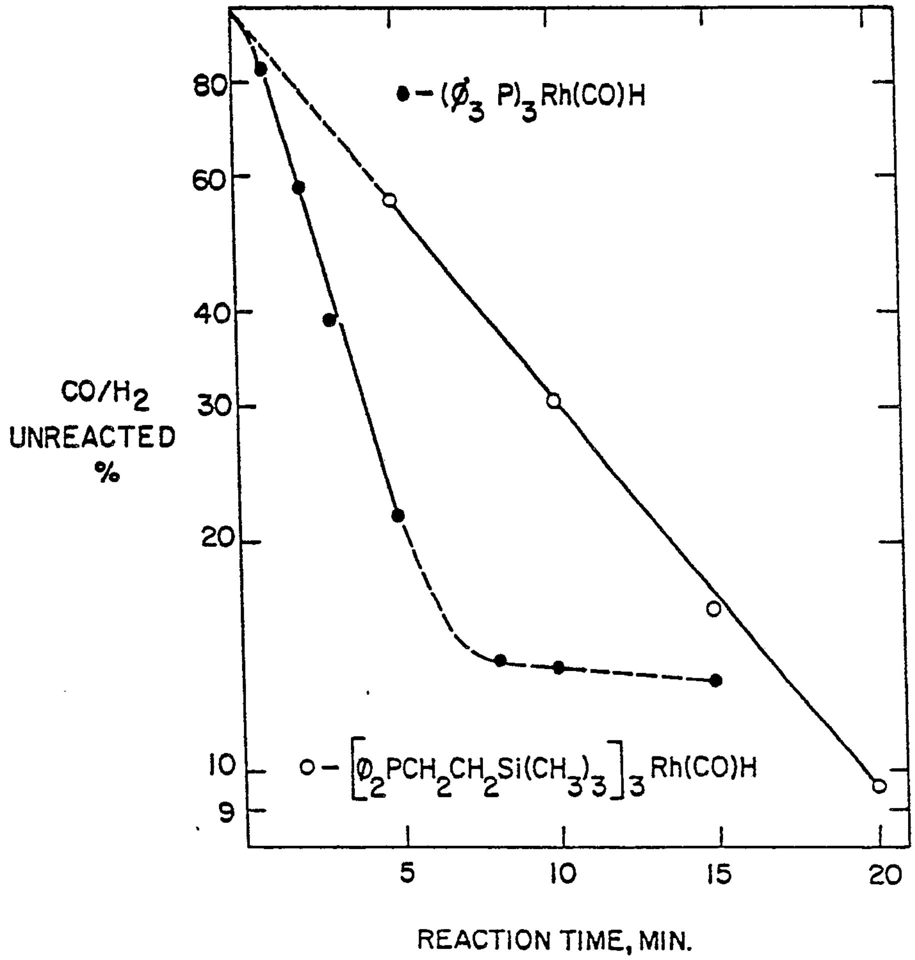


FIG. 5B

BUTENE HYDROFORMYLATION RATE VERSUS TEMPERATURE CORRELATIONS IN THE PRESENCE OF EXXON AND UNION CARBIDE RHODIUM-PHOSPHINE COMPLEX CATALYSTS

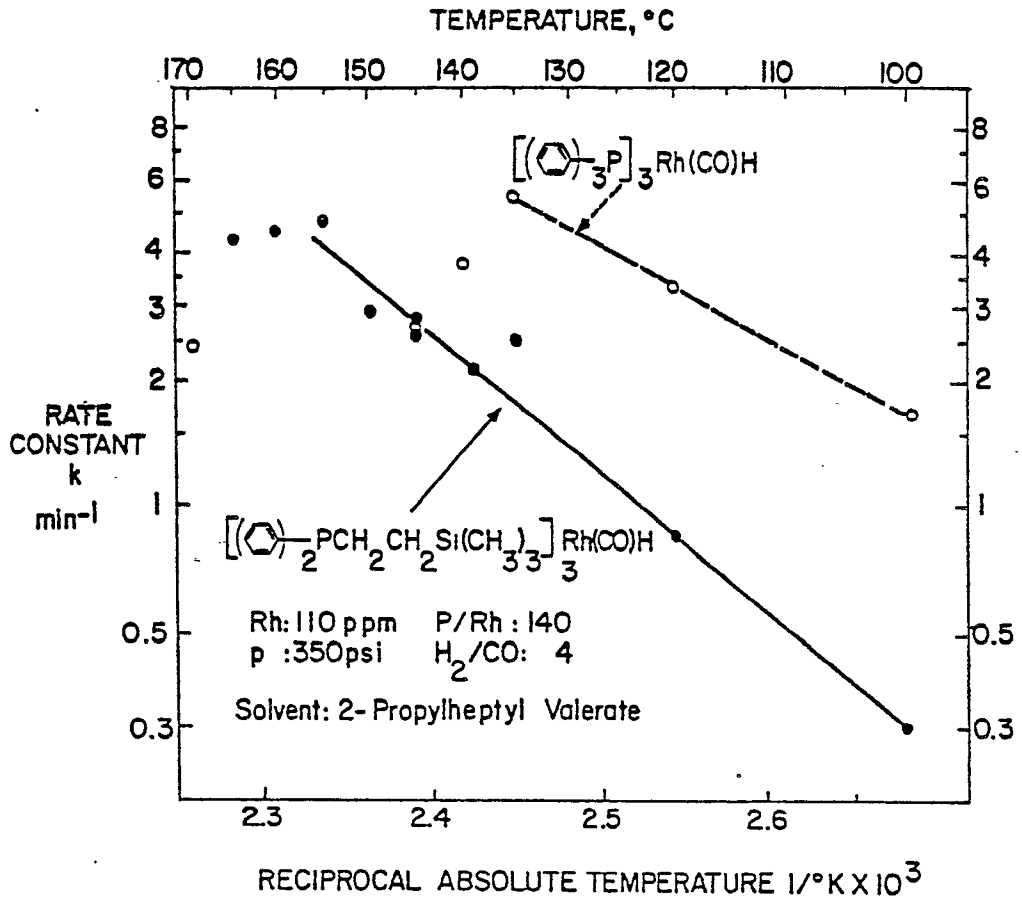


FIG. 6

1-BUTENE HYDROFORMYLATION TEMPERATURE VERSUS n-VALERALDEHYDE (n) TO TOTAL VALERALDEHYDE (n+I) RATIO CORRELATIONS IN THE PRESENCE OF SEP AND TPP BASED RHODIUM-PHOSPHINE COMPLEX CATALYSTS

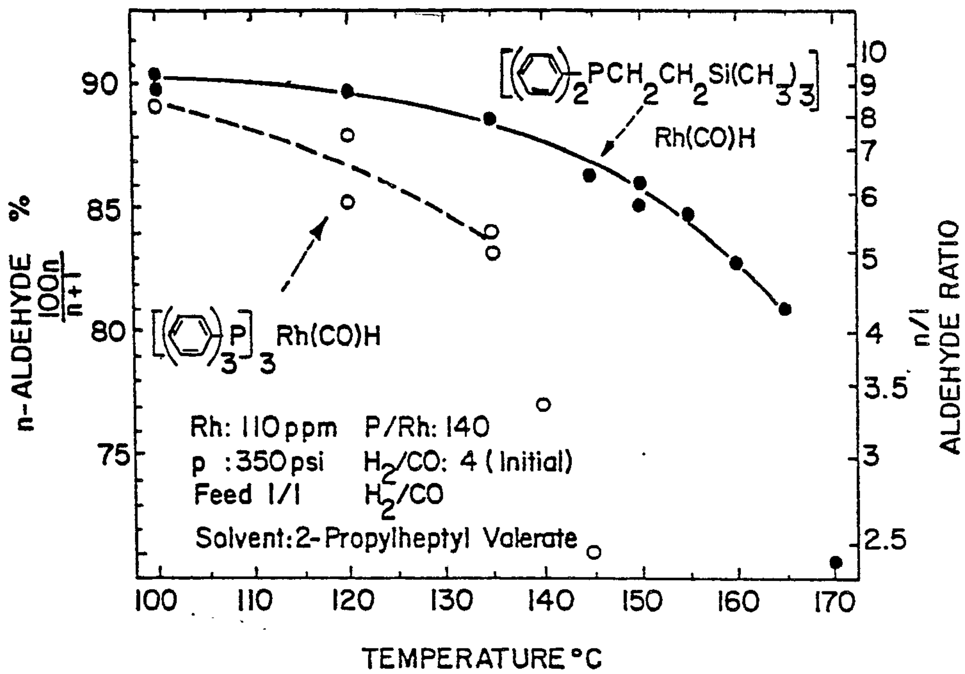


FIG. 7

I-BUTENE HYDROFORMYLATION TEMPERATURE VERSUS
BUTANE BY-PRODUCT FORMATION CORRELATIONS
IN THE PRESENCE OF SEP AND TPP BASED COMPLEX CATALYSTS

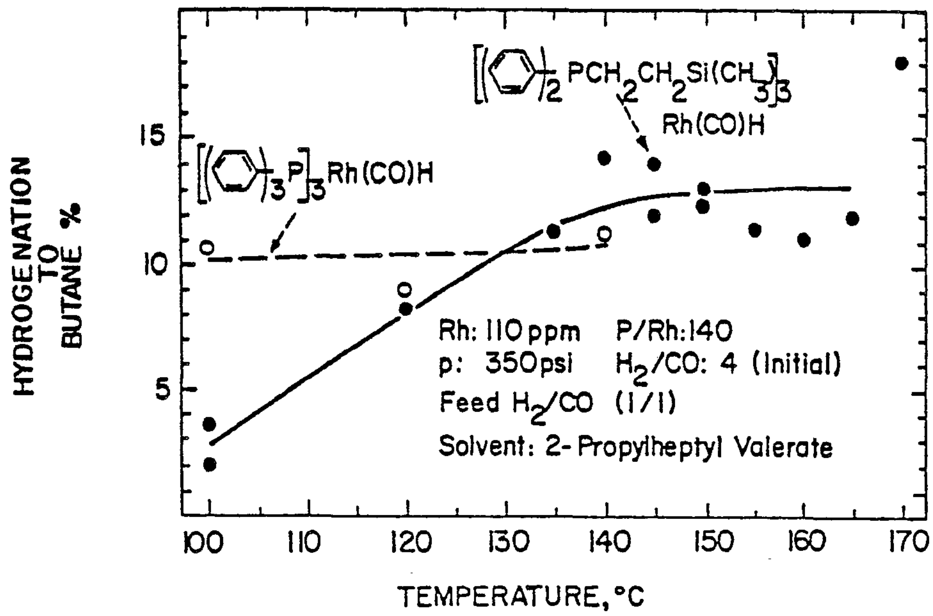


FIG. 8

I-BUTENE HYDROFORMYLATION TEMPERATURE VERSUS
2-BUTENES BY-PRODUCT FORMATION
CORRELATIONS IN THE PRESENCE OF SEP AND TPP COMPLEX
CATALYSTS

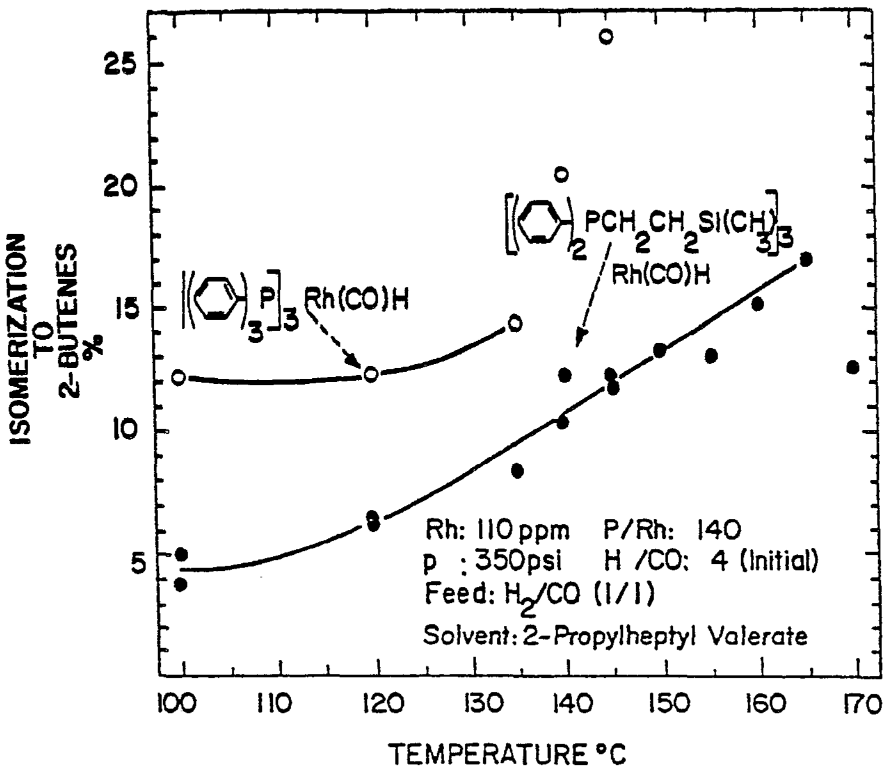


FIG.9

EFFECT OF DTS LIGAND TO RHODIUM RATIO
ON SELECTIVITY OF BUTENE HYDROFORMULATION AT 145 AND 170°C
at 110 ppm Rh conc. and 350 psi pressure
$L_3Rh(CO)H$ COMPLEX; L = DTS = $O_2PCH_2CH_2SiMe_3$

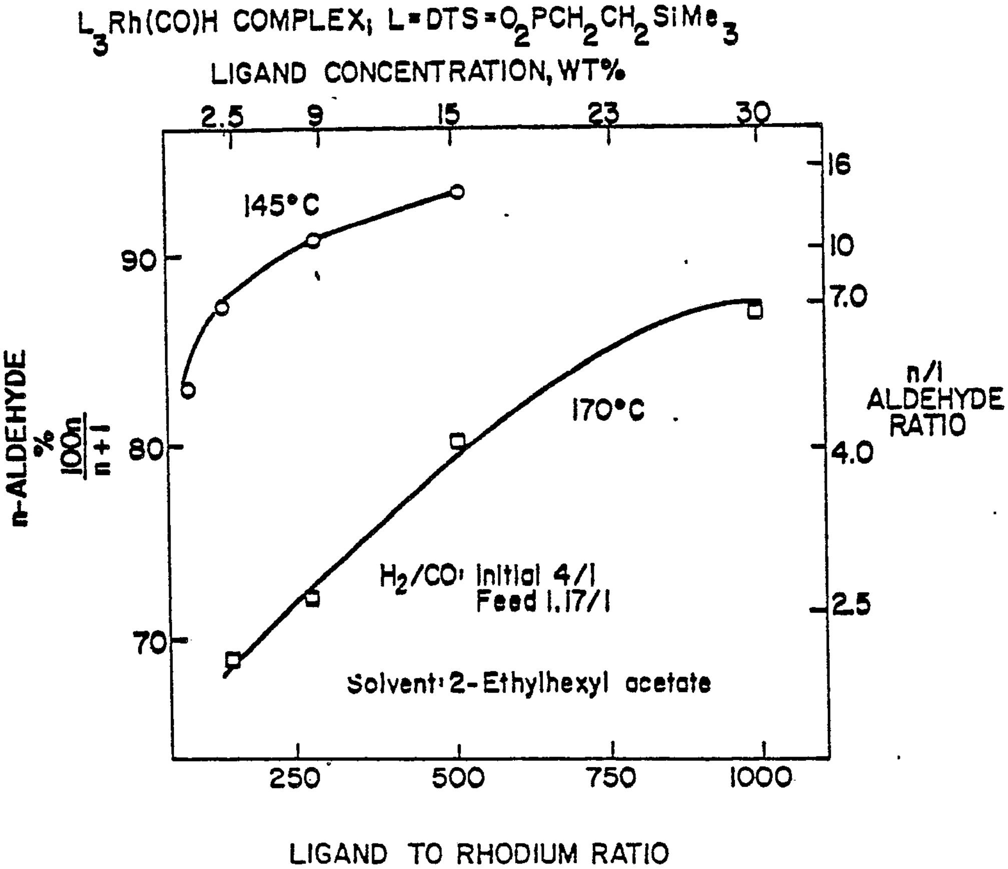


FIG. 10

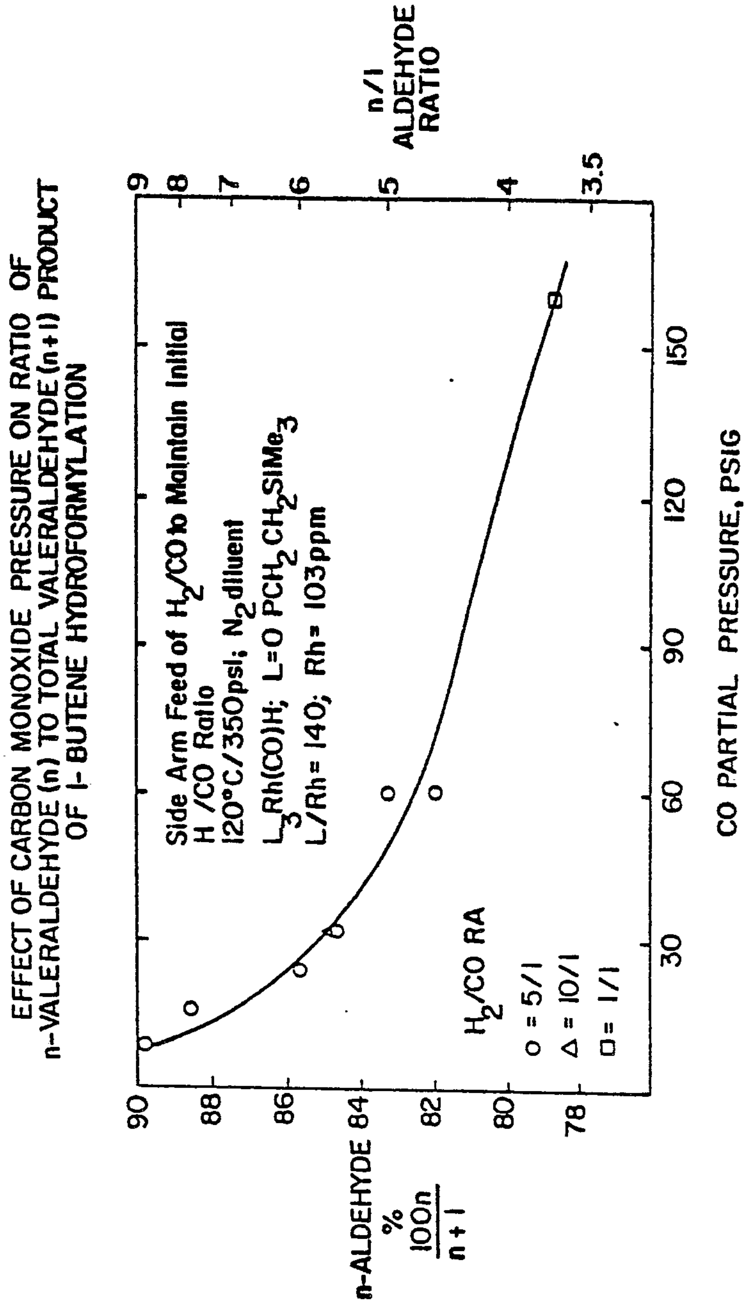
EFFECT OF CARBON MONOXIDE PRESSURE ON RATIO OF
n-VALERALDEHYDE (n) TO TOTAL VALERALDEHYDE (n+I) PRODUCT
OF I- BUTENE HYDROFORMYLATION
Side Arm Feed of $H_2$/CO to Maintain Initial
$H_2$/CO Ratio
120°C/350psi; $N_2$ diluent
$L_3$Rh(CO)H; L=O $PCH_2CH_2SiMe_3$
L/Rh= 140; Rh= 103ppm
$H_2$/CO RA
o = 5/1
Δ = 10/1
□ = 1/1
n-ALDEHYDE % $\frac{100n}{n+1}$
90
88
86
84
82
80
78
n/I ALDEHYDE RATIO
9
8
7
6
5
4
3.5
30
60
90
120
150
CO PARTIAL PRESSURE, PSIG

FIG.11

ALDEHYDE PRODUCTION RATE DURING CONTINUOUS BUTENE HYDROFORMYLATION
As shown with 140 psi of 10/1 $H_2$/CO at 120° for 448 hours and then at 125°
In the presence of 270 ppm Rh and Rh/P mole ratio of 210

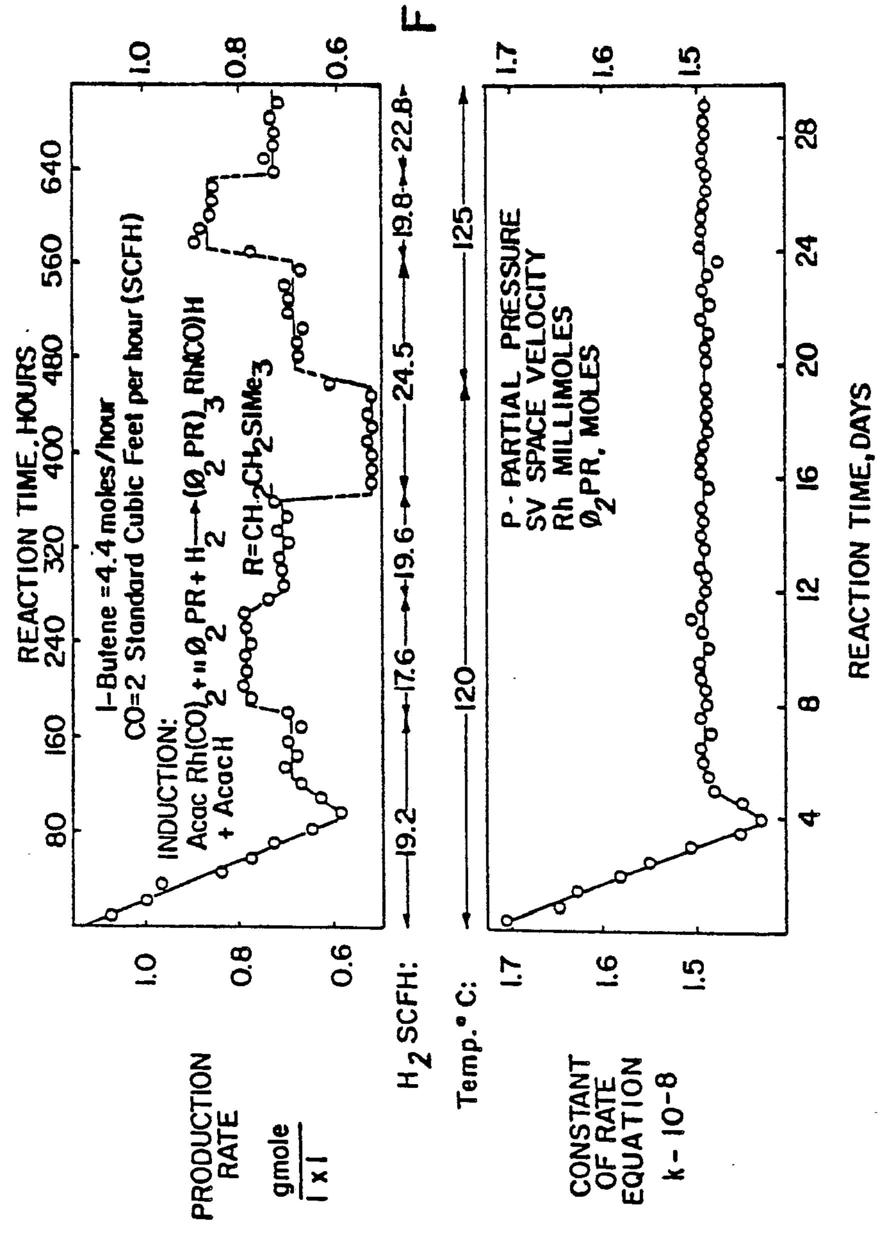


FIG.12